(19) Europäisches Patentamt
European Patent Office
Office européen des brevets

(11) EP 2 348 030 B1

(12) **EUROPEAN PATENT SPECIFICATION**

(45) Date of publication and mention of the grant of the patent:
**06.05.2015 Bulletin 2015/19**

(21) Application number: **10010647.5**

(22) Date of filing: **16.03.2001**

(51) Int Cl.:
***C07J 31/00*** *(2006.01)*
***C07J 33/00*** *(2006.01)*
***C07J 3/00*** *(2006.01)*
***C07J 43/00*** *(2006.01)*
***A61K 31/58*** *(2006.01)*
***C07J 71/00*** *(2006.01)*
***A61P 5/44*** *(2006.01)*

(54) **17-Alpha-substituted-11-beta-substituted-4-aryl and 21-substituted 19-norpregnadienedione as antiprogestational agents**

17-Alpha-substituiertes-11-Beta-substituiertes-4-aryl und 21-substituiertes 19-Norpregnadiendion als Antiprogestationswirkstoffe

Modification structurale de 17-alpha-substitué-11-bêta-substitué-4-aryle et 21-substitué 19-norpregnadienedione en tant que nouveaux agents antiprogestatifs

(84) Designated Contracting States:
**AT BE CH CY DE DK ES FI FR GB GR IE IT LI LU MC NL PT SE TR**

(30) Priority: **17.03.2000 US 526855**

(43) Date of publication of application:
**27.07.2011 Bulletin 2011/30**

(62) Document number(s) of the earlier application(s) in accordance with Art. 76 EPC:
**08009259.6 / 2 033 965**
**01918812.7 / 1 265 911**

(73) Proprietor: **The Government of the United States of America**
**as represented by the Secretary of the Department**
**of Health and Human Services**
**Rockville, MD 20852-3804 (US)**

(72) Inventors:
- **Kim, Hyun K.**
  **Bethesda, MD 20817 (US)**
- **Blye, Richard P.**
  **Highland, MD 20777 (US)**
- **Rao, Pemmaraju N.**
  **San Antonio, TX 78228 (US)**
- **Cessas, James W.**
  **Floresville, TX 78114 (US)**
- **Acosta, Carmie K.**
  **San Antonio, TX 78213 (US)**

(74) Representative: **Campbell, Patrick John Henry**
**J A Kemp**
**14 South Square**
**Gray's Inn**
**London WC1R 5JJ (GB)**

(56) References cited:
**WO-A1-97/41145** **WO-A1-99/45022**
**WO-A2-01/18025**

- **RAO P N ET AL: "11beta-Substituted 13beta-ethyl gonane derivatives exhibit reversal of antiprogestational activity", STEROIDS, ELSEVIER SCIENCE PUBLISHERS, NEW YORK, NY, US, vol. 63, no. 1, 1 January 1998 (1998-01-01), pages 50-57, XP004103060, ISSN: 0039-128X, DOI: 10.1016/S0039-128X(97)00119-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates generally to the field of steroids and, in particular, to novel 17-α-substituted, 11-β-substituted-4-aryl and 21-substituted 19-norpregnadienedione analogs which possess potent antiprogestational activity with minimal antiglucocorticoid activity.

### BACKGROUND OF THE INVENTION

There have been numerous attempts over the past few decades to prepare steroids with antihormonal activity. These have been reasonably successful where antiestrogens and antiandrogens are concerned. However, the discovery of effective antiprogestational and antiglucocorticoid steroids has proved to be a formidable task for the steroid chemist. It has been generally recognized for some years, however, that antiprogestational steroids would find wide applicability in population control, while antiglucocorticoids would be extremely valuable in the treatment of, for example, Cushing's syndrome and other conditions characterized by excessive endogenous production of cortisone. In the last decade, largely through the efforts of Teutsch, *et al.* of the Roussel-Uclaf group in France, a new series of 19-nortestosterone derivatives has been synthesized with strong affinity for the progesterone and glucocorticoid receptors and with marked antiprogestational and antiglucocorticoid activity *in vivo.* This important discovery revealed the existence of a pocket in the progesterone/glucocorticoid receptors that is able to accommodate a large 11β-substituent on selected 19-nortestosterone derivatives. By suitable selection of such a substituent, steroids with antihormonal properties were obtained.

The pioneering studies of Teutsch, *et al.* on the synthesis of antiprogestational and antiglucocorticoid steroids is summarized in a recent review article (G. Teutsch in Adrenal Steroid Antagonism. Ed. M. K. Agarwal, Water de Gruyter and Co., Berlin, 1984. pp. 43-75) describing the work leading to the discovery of RU-38,486, the first steroid of this type selected for clinical development RU-38,486 or mifepristone was found to be an effective antiprogestational/contragestative agent when administered during the early stages of pregnancy (IPPF Medical Bulletin 20; No. 5, 1986). In addition to these antiprogestational properties, mifepristone has very significant antiglucocorticoid activity and was successfully used by Nieman, et al., J. Clin. Endocrinology Metab., 61:536, (1985)) in the treatment of Cushing's syndrome. In common with the vast majority of steroidal hormone analogs, mifepristone additionally exhibits a range of biological properties. Thus, for example, it exhibits growth-inhibitory properties towards estrogen-insensitive T47Dco human breast cancer cells (Horwitz, Endocrinology, 116:2236, 1985). Experimental evidence suggests that the metabolic products derived from mifepristone contribute to its antiprogestational and antiglucocorticoid properties (Heikinheimo, et al., J. Steroid Biochem., 26:279 (1987)). WO-A-99/45022 discloses anti-progestational 11β-phenyl-Pregna-4,9-diene-3,20-diones with a piperidine substituent on the phenyl group.

Ideally, for purposes of contraception, it would be advantageous to have compounds which possess antiprogestational activity without (or with minimal) antiglucocorticoid activity. Although there have been a number of attempts to modify the mifepristone structure in order to obtain separation of the antiprogestational activity from the antiglucocorticoid activity, this goal has not yet been fully achieved. As such, there remains a need in the art for the development of new steroids which possess antiprogestational activity with minimal antiglucocorticoid activity.

### SUMMARY OF THE INVENTION

The present invention provides new steroids which possess potent antiprogestational activity with minimal antiglucocorticoid activity. More particularly, the present invention provides compounds having the general formula:

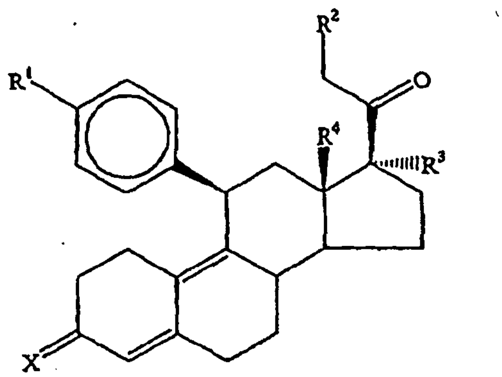

I

wherein: $R^1$-$R^4$ and X are as defined in the claims.

As explained above, the compounds of the present invention possess potent antiprogestational activity with minimal antiglucocorticoid activity and, thus, they are suitable for long term use in the treatment of human endocrinologies or other conditions in steroid-sensitive tissues. Specific conditions for treatment include, but are not limited to, endometriosis (Kettel, L.M., et al., Fertil Steril, 56:402-407; Murphy, A.A., et al., Fertil Steril, 6:3761-766; Grow, D.R, et al., J. Clin. Endocrinol. Metab., 81:1933-1939.) uterine leiomyoma (Murphy, *A.A., et al., Ibid.;* Murphy, A.A., et al., J. Clin. Endocrinol. Metab., 76:513-517), uterine fibroid (Brogden, R.N., et al., Drugs, 45:384:409), meningioma (Brogden, R.N., *et al., Ibid.;* Poisson, M., et al., J. Neurooncol., 1:179-189; Carroll, R.S., et al., Cancer Res., 53:1312-1316*;* Mahajan, D.K. and London, S.N., Fertil Steril, 68:967-976 (1997)), and metastatic breast cancer (Brogden, R.N., *et al., Id.*; Rochefort, H., Trends in Pharmacol. Sci., 8:126-128; Horwitz, K.B., Endocr. Rev., 13:146-163 (1992) Mahajan, D.K. and London. S.N., *Id.*). Other uses include, but are not limited to, contraception (Wood, A.J.J., N. engl. J. Med., 329:404-412 (1993); Ulmann, A., et al., Sci. Amer., 262:42-48 (1990)), emergency postcoital contraceptive (Reel, J.R, et al., Contraception, 58:129-136 (1998)) and inducement of cervical ripening.

As such, in addition to providing compounds of Formula I, the present invention provides methods wherein the compounds of Formula I are advantageously used, *inter alia,* to antagonize endogenous progesterone; to induce menses; to treat endometriosis; to treat dysmenorrhea; to treat endocrine hormone-dependent tumors (*e.g.,* breast cancer, uterine leiomyomas, *etc.*); to treat meningiomas; to treat uterine fibroids; to inhibit uterine endometrial proliferation; to induce cervical ripening; to induce labor, and for contraception.

Other features, objects and advantages of the invention and its preferred embodiments will become apparent from the detailed description which follows.

## BRIEF DESCRIPTION OF THE DRAWINGS

**Figures 1** through **11** illustrate the synthetic schemes used to prepare the compounds of Formula I.

## DETAILED DESCRIPTION OF THE INVENTION AND PREFERRED EMBODIMENTS

In one aspect, the present invention provides compounds as previously defined.

The compounds of the present invention can readily be synthesized in a variety of ways using modem synthetic organic chemistry techniques. Typically, the compounds of the present invention are prepared using the synthetic schemes set forth in Figures 4 and 6-9. In general, there are five strategic steps that are useful in the synthesis of the antiprogestational agents of the present invention. They are: (**1**) C21-substitution; (**2**) construction of the 17α-hydroxy-20-ketone pregnane side chain with the natural configuration via the SNAP reaction; (**3**) modification of the 17α-hydroxy moiety; (**4**) regiospecific synthesis of the epoxide and 1,4-conjugate grignard addition of a variety of 4-substituted aryl compounds; and (**5**) deketalization at C3 and 20 and concomitant dehydratration at C5. Each of these five strategic steps is described in greater detail hereinbelow. Moreover, a more detailed description of the synthetic protocols used to prepare the compounds of the present invention is set forth in the Example Section. It will be readily apparent to those of skill in the art that the particular steps, or combination of steps, used will vary depending on the compound being synthesized. Figures 1-3, 5, 10, 11 and the corresponding parts of the description below, disclose the synthesis of compounds related to those of the invention which illustrate synthetic techniques useful in the synthesis of the compounds of the invention.

### 1. 21-Substitution

In particular embodiments, a number of different functional groups, such as F, Cl, Br, Me, hydroxy, alkoxy (*e.g.,* methoxy, ethoxy, *etc.*), acyloxy (*i.e.,* formyloxy, acetoxy, propionyloxy, *etc.*), cypionyloxy, methoxyacetoxy, and acylthio, have been introduced at C-21 of lead compound 17α-acetoxy-11β-(4-*N,N*-dimethylaminophenyl)-19-norpregna-4,9-diene-3,20-dione (CDB-2914 or C-21H or **69B**) using the synthetic schemes set forth in **Figures 1, 2** and **3.** For instance, a Silicon Nucleophilic Annulation Process (SNAP) on 17β-cyanohydrin (**5**) was used to prepare all of the 21-halogenated compounds with the exception of the 21-fluoro compound. This compound, however, was readily obtained by reacting the 21-mesylate with KF in acetonitrile in the presence of a crown ether. In addition, the 17α-acetoxy-21-ol compound (**41**) was obtained selectively from the ethoxyethylidenedioxy derivative (**18**) by means of buffered hydrolysis, whereas the 17α-ol-21-acetate derivative (**8**) was prepared from reacting the 21-halo compound with KOAc. It is interesting to note that both the 21-acetate and the 17α-acetate produced the 17α,21-diol (**9**) by a base catalyzed methanolosis. Thereafter, this 17α,21-diol was readily converted to the 17α,21-diacetate (**15**) by a mixed anhydride procedure. With regard to the synthesis of 17α-acetoxy-21-cypionate (**40**), the hydroxyl group at C-21 of the 17α,21-diol (**9**) was first converted to the corresponding cypionate (**39**) and then the 17α-OH group was acetylated. The 17α-acetoxy-21-thioacetate (**17**) was obtained by reaction of the 21-iodo compound generated *in situ* from the corresponding bromo compound

(**7B**), with potassium thioacetate followed by acetylation of the 17α-alcohol as shown in the synthetic scheme set forth in **Figure 1.**

Moreover, the 21-methyl analog (**28**) was prepared following the synthetic route set forth in **Figure 2.** The key reactions in this scheme are (1) the conversion of the 17α-cyanohydrin to the 17α-trimethylsilyloxy, 17α-aldehyde, and (2) the creation of the 21-methylprogesterone skeleton (**21** → **22**).

In addition, the 21-methoxy analog (**38**) was obtained following the synthetic scheme set forth in **Figure 3.** The key step in this scheme is the reaction of the 17α,21-diol protected at C-3 and C-20 with Meerwein's trimethyloxonium tetrafluoroborate salt in the presence of the sterically more hindered, less nucleophilic base, 1,8-bis(dimethylamino)naphthalene, as the proton sponge to selectively methylate the less-hindered 21-hydroxyl group. The subsequent epoxidation of the crude 21-methoxy compound (**34**) produced a 2:1 mixture of α and β epoxides as evidenced by $^1$H NMR. The crude epoxide (**35**) was subjected directly to the copper (I) catalyzed conjugate Grignard addition, assuming 66% of the crude epoxide was the desired - epoxide, hydrolysis and acetylation gave the 21-methoxy compound (**38**) with a purity of 98%. Following similar procedures, the 21-ethoxy compound (**46**) was obtained using triethyloxonium tetrafluoroborate salt. Treatment of the 21-acetete (**15**) and 21-methoxy compound (**38**) with hydroxylamine HCl followed by adjustment of the pH to pH 7 provided the desired 3-oximes, **47** and **48**, respectively, as a mixture of syn- and anti-isomers. Under these conditions, the sterically hindered C-20 ketone was intact as evidenced by IR spectroscopy.

In addition, using methods similar to those described above, additional functional groups, such as propionyloxy- (**126a**), 2-methoxyacetoxy- (**126b**), methylcarbonate (**126c**), 2-(*N,N*-dimethylamino)acetoxy- (**133**), and thiocyanato- (**138**) were readily synthesized (*see, e.g.,* **Figure 10 and 11**). Their synthetic methodology is straightforward. All of these compounds were derived from the previously prepared 17α,21-dihydroxy-11β-[4-(*N,N*-dimethylamino)phenyl]-19-norpregna-4,9-diene-3,20-dione (**9** in **Figure 1** or **124** in **Figure 11**). The C21-(1-ethenyl)oxy analog (**129**) was obtained from the C17a-acetoxy-21-ol (**128**) by reaction with ethyl vinyl ether in the presence of mercury(II) trifluoroacetate. Compound **128** was, in turn, obtained from hydrolysis of the 17α,21-cyclic ortho ester (**18** in **Figure 1** or **127** in **Figure 11**). Reaction of the C17α,21-diol (**9** in **Figure 1** or **124** in **Figure 11**) with methyl chloroformate in pyridine gave the methyl carbonate at C21(**125c**). Subsequent acetylation at C17 led to the target compound **126c** *(see,* **Figure 11**). Treatment of the C17α,21-diol (**9** or **124**) with methoxyacetyl chloride, followed by acetylation, provided **126b** (*see,* **Figure 11**). The synthesis of the 21-thiocyanato analog (**138**), which is illustrated in **Figure 11,** involved the preparation of the 21-mesylate (**136**), followed by thiocyanation at C21 (**137**) using the modified procedure of Abramson, H.N., et al. (J. Pharm. Sci. 65:765-768 (1976)). Subsequent acetylation at C17 led to the target compound (**138**). The 21-(*N,N*-dimethylamino)aretoxy (**133**) analog was obtained by preparing the 21-chloroacetate (**130**), acetylation of the 17α-OH (**131**) and converting the latter to the 21-iodoacetate (**132**) followed by the reaction of **132** with dimethylamine (*see*, **Figure 10**). This order of sequence did not result in hydrolysis of the 21-ester group. It is pointed out that an attempt to prepare the 21-iodoacetate (**132**) directly from the diol (**124**) was not as successful.

The 17α,21-diformate (**139**), which is illustrated in **Figure 10,** was synthesized by perchloric acid catalyzed formulation of the 17α,21-diol (**124**) following the procedure of Oliveto, E.P., et al. (J. Am. Chem. Soc., 77:3564-3567 (1955)). NMR analysis of this material indicated a 55:45 mixture of the 17α,21-diformate (**139**) resonating at 8.029 (s, C17-OC<u>H</u>O) and 8.165 ppm (s, C21-OC<u>H</u>O), respectively, and the 21-monoformate (**140**) at 8.172 ppm (s, C21-OC<u>H</u>O). Therefore, chromatographic separation was essential to obtain the pure 17α,21-diformate (**139**).

Syntheses of the 17α,21-dimethoxy derivatives (**113a**, **113b**, **133c** and **133d**) were achieved via oxidation at C-21 to afford the 21-hydroxy derivative (**107**) of the 17α-methoxy compound (**94**) following a modification of the procedure reported by Moriarty, R.M. et al., J. Chem. Soc. Chem. Commun., 641-642 (1981), and Velerio, et al., Steroids, 60:268-271 (1995). Subsequent O-methylation provided the key 17α,21-dimethoxy intermediate (**108**) (*see,* **Figure 8**). Reduction of the 20-ketone (**108**) to the 20ξ-ol (**109**) followed by epoxidation at C5 and C10, copper (I) catalyzed conjugate Grignard addition to the 5α, 10α-epoxide (**110**), selective oxidation of the secondary alcohol, 20ξ-ol (**111**) using IBX to the 20-ketone (**112**), hydrolysis and acetylation, led to the target 17α,21-dimethoxy derivatives (**113**).

**2. Silicon Nucleophilic Annelation Process (SNAP)**

As described herein silylation of β-cyanohydrin ketal with halomethyldimethylsilyl chloride afforded the chloro- or bromomethyldimethylsilyl ether. The reductive SNAP reaction provided the 17α-hydroxy-20-ketopregnane side chain with the natural configuration at C17 (Livingston, D.A., et al., J. Am. Chem. Soc., 112:6449-6450 (1990); Livingston, D.A., Adv.Med. Chem., 1:137-174 (1992); U.S. Patent No. 4,092,693, which issued to Livingston, D.A., et al. (May 1, 1990); U.S. Patent No. 4,977,255, which issued to Livingston, D.A., et al. (December 11, 1990). Alternatively, the formation of the halomethyldimethylsilyl ether, followed by treatment with lithium diisopropyl amide, provided the 21-substituted -17α-hydroxy-20-ketopregnanes.

### 3. 17α-Substitution

All 17a-esters illustrated in **Figures 4-11** were prepared from their 17a-hydroxy precursors. With the exception of the 17α-formate (**69A**) and the 17α,21-diformate (**139**), all 17α-esters were also obtained via a mixed anhydride procedure (Carruthers, N.I. et al., J. Org. Chem., 57:961-965 (1992)).

17α-methoxy steroid (**93**) became available in large quantities from the 17a-hydroxydienedione (**92**) leading to a new series of antiprogestational agents, such as compounds **97** and **113**. Methylation of 17α-hydroxy group was most efficiently carried out using methyl iodide and silver oxide with acetonitrile as a cosolvent as described in the general procedure of Finch, et al. (J. Org. Chem., 40:206-215 (1975)). Other syntheses of 17α-methoxy steroids have been reported in the literature (*see, e.g.,* Numazawa, M. and Nagaoka, M, J. Chem. Soc. Commun., 127-128 (1983); Numazawa, M. and Nagaoka, M., J. Org. Chem., 50:81-84 (1985); Glazier, E.R., J. Org. Chem., 27:4397-4393 (1962).

The 17α-methoxymethyl compound (**91**) was obtained in 0.7% overall yield via the 14-step sequence illustrated in **Figure 5** starting from estrone methyl ether (**77**). No attempts were made to optimize the yield. The general strategy involved: (1) Construction of the 20-ketopregnane side chain; (2) Formation of the 17,20-enol acetate and subsequent alkylation with bromomethyl methyl ether; (3) Elaboration of the 3-ketal-5(10),9(11)-diene; (4) Epoxidation; (5) Conjugate Grignard addition; and (6) Hydrolysis.

### 4. 11β-Aryl-4-Substitution

The introduction of a variety of 4-substituted phenyl group at C11β into 19-norprogesterone requires the 5α,10α-epoxide. Epoxidation of **2**, **23, 34, 42, 50, 88, 94, 99, 109** and **119** has been known to be problematic *(see,* Wiechert, R. and Neef, G., J. Steroid Biochem., 27:851-858 (1987)). The procedure developed by Teutsch, G., et al. (Adrenal Steroid Antagonism (Agarwal, M.K., ed.), 43-75, Walter de Gruyter & Co., Berlin, N.Y. (1984)), *i.e.,* $H_2O_2$ and hexachloro or fluoroacetone, proved to be regioselective, but not highly stereoselective. A mixture of 5α,10α-epoxide and the corresponding 5β,10β-isomer was formed in approximately a 3:1 ratio. However, reduction of the C20-ketone (**108**) to the C20-ol (**109**) prior to epoxidation, resulted in a 9:1 ratio of the desired 5α,10α-epoxide.

Treatment of the 5α,10α-epoxides with 3-5 equivalents of Grignard reagents prepared from various 4-substituted aryl bromides *(see,* Yur'ev, Y.K., et al., Izvest. Akad. Nauk. S.S.S.R., Otdel Khim Nauk, 166-171 (CA 45: 10236f, (1951)); Wolfe, J.P. and Buchwald, S.L., J. Org. Chem., 62:6066-6068 (1997); Veradro, G., et al., Synthesis, 447-450 (1991); Jones, D.H., J. Chem. Soc. (C), 132-137 (1971); Detty et al., J. Am. Chem. Soc., 105:875-882 (1983), and Rao, P.N. et al., Steroids, 63:523-550 (1998)) in the presence of copper (I) chloride as a catalyst provided the desired 11β-4-substituted phenyl steroids. It is noted that 4-bromothioanisole was purchased from the Aldrich Chemical Co. (Milwaukee, Wisconsin). Evidence of the 11β-orientation of the 4-substituted phenyl substituent was shown by the upfiled shift of the C18 methyl group (8 = 0.273 - 0.484 ppm in $CDCl_3$), which is in agreement with Teutsch's observations *(see,* Teutsch, G. and Belanger, A., Tetrahedron Lett., 2051-2054 (1979)).

The presence of an unprotected 20-ketone resulted in low yields or in undesirable Grignard product mixtures. This was circumvented by reduction of the 20-ketone (analysis of this material by NMR indicated a single isomer; no further work was done for identification of this single isomer) prior to epoxidation and subsequent oxidation of the 20-alcohol by use of iodoxybenzoic acid (IBX) (Dess, D.B. and Martin, J.C., J. Org. Chem., 48:4155-4156 (1983); Frigerio, M. and Santagostino, M., Tetrahedron Letters, 35:8019-8022 (1994); and Frigerio, M. et al., J. Org. Chem., 60:7272-7276) after Grignard addition (*see,* **Figure 8**).

In case of **Figures 5** and **6,** the C3-ketone group was protected as a monoethyleneketal, and the C20-ketone was found to be intact when the Grignard reaction was followed during the multi-step procedures. For the syntheses of the 17α,21-diacetoxy derivatives (**Figure 7**), the strategy was to accomplish the conjugate addition prior to the SNAP reaction using the multi-step process described herein.

### 5. Deketalization

Deketalization with concomitant dehydration at C-5 in acidic media proceeded smoothly to provide the 4,9-diene-3,20-dione.

Quite surprisingly, the compounds of Formula I possess potent antiprogestational activity with minimal antiglucocorticoid activity. As a result of their antiprogestational activity, the compounds of Formula I can advantageously be used, *inter alia,* to antagonize endogenous progesterone; to induce menses; to treat endometriosis; to treat dysmenorrhea; to treat endocrine hormone-dependent tumors; to treat meningioma; to treat uterine leiomyonas, to treat uterine fibroids; to inhibit uterine endometrial proliferation; to induce labor; to induce cervical ripening, for hormone therapy; and for contraception.

More particularly, compounds having antiprogestational activity are characterized by antagonizing the effects of progesterone. As such, the compounds of the present invention are of particular value in the control of hormonal

irregularities in the menstrual cycle, for controlling endometriosis and dysmenorrhea, and for inducing menses. In addition, the compounds of the present invention can be used as a method of providing hormone therapy either alone or in combination with estrogenic substances in postmenopausal women, or in women whose ovarian hormone production is otherwise compromised.

Moreover, the compounds of the present invention can be used for control of fertility during the whole of the reproductive cycle. For long-term contraception, the compounds of the present invention can be administered either continuously or periodically depending on the dose. In addition, the compounds of the present invention are of particular value as postcoital contraceptives, for rendering the uterus inimical to implantation, and as "once a month" contraceptive agents.

A further important utility for the compounds of the present invention lies in their ability to slow down growth of hormone-dependent tumors and/or tumors present in hormone-responsive tissues. Such tumors include, but are not limited to, kidney, breast, endometrial, ovarian, and prostate tumors, *e.g.,* cancers, which are characterized by possessing progesterone receptors and can be expected to respond to the compounds of this invention. In addition, such tumors include meningiomas. Other utilities of the compounds of the present invention include the treatment of fibrocystic disease of the breast and uterine.

Compounds of the present invention suitable for use in the above method can readily be identified using *in vitro* and *in vivo* screening assays known to and used by those of skill in the art. For instance, a given compound can readily be screened for its antiprogestational properties using, for example, the anti-McGinty test and/or the anti-Clauberg test described in the examples. In addition, a given compound can readily be screened for its ability to bind to the progesterone and/or glucocorticoid receptors or to inhibit ovulation using the assays described in the examples. Moreover, a given compound can readily be screened for its ability to inhibit tumor cell growth (*e.g.,* malignant tumor growth, *i.e.,* cancer) or to abolish tumorigenicity of malignant cells *in vitro* or *in vivo*. For instance, tumor cell lines can be exposed to varying concentrations of a compound of interest, and the viability of the cells can be measured at set time points using, for example, the alamar Blue® assay (commercially available from BioSource, International of Camarillo, California). Other assays known to and used by those of skill in the art can be employed to identify compounds useful in such methods.

The compounds according to the present invention can be administered to any warm-blooded mammal such as humans, domestic pets, and farm animals. Domestic pets include dogs, cats, *etc.* Farm animals include cows, horses, pigs, sheep goats, *etc.*

The amount of active ingredient that can be combined with a carrier material to produce a single dosage form will vary depending upon the disease treated, the mammalian species, and the particular mode of administration. For example, a unit dose of the steroid can preferably contain between 0.1 milligram and 1 gram of the active ingredient. A more preferred unit dose is between 0.001 and 0.5 grams. It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those of skill in the area.

The compounds of the present invention can be administered by a variety of methods. Thus, those products of the invention that are active by the oral route can be administered in solutions, suspensions, emulsions, tablets, including sublingual and intrabuccal tablets, soft gelatin capsules, including solutions used in soft gelatin capsules, aqueous or oil suspensions, emulsions, pills, lozenges, troches, tablets, syrups or elixirs and the like. Products of the invention active on parenteral administration can be administered by depot injection, implants including Silastic TM and biodegradable implants, intramuscular and intravenous injections.

Compositions can be prepared according to any method known to the art for the manufacture of pharmaceutical compositions and such compositions can contain one or more agents selected from the group consisting of sweetening agents, flavoring agents, coloring agents and preserving agents. Tablets containing the active ingredient in admixture with nontoxic pharmaceutically acceptable excipients which are suitable for manufacture of tablets are acceptable. These excipients can be, for example, inert diluents, such as calcium carbonate, sodium carbonate, lactose, calcium phosphate or sodium phosphate, granulating and disintegrating agents, such as maize starch, or alginic acid; binding agents, such as starch, gelatin or acacia; and lubricating agents, such as magnesium stearate, stearic acid and talc. Tablets can be uncoated or, alternatively, they can be coated by known methods to delay disintegration and adsorption in the gastrointestinal tract and thereby provide a sustained action over a longer period. For example, a time delay such as glyceryl monostearate or glyceryl distearate alone or with a wax can be employed.

Formulations for oral use can also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil; liquid paraffin or olive oiL

Aqueous suspensions of the invention contain the active materials in admixture with excipients suitable for the manufacture of aqueous suspensions. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (*e.g.,* lecithin), a condensation product of an alkylene oxide with a fatty acid (*e.g.,* polyoxyethylene stearate), a condensation product of ethylene oxide with a long chain aliphatic alcohol (*e.g.,* heptadecaethylene oxycetanol), a condensation product of ethylene oxide with a partial ester derived from a fatty acid and a hexitol (*e.g.,* polyoxyethylene sorbitol mono-oleate), or a condensation product of ethylene oxide with a partial ester derived from fatty acid and a hexitol anhydride (*e.g.,* polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Ophthalmic formulations, as is known in the art, will be adjusted for osmolarity.

Oil suspensions can be formulated by suspending the active ingredient in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These compositions can be preserved by the addition of an antioxidant such as ascorbic acid.

Dispersible powders and granules of the invention suitable for preparation of an aqueous suspension by the addition of water can be formulated from the active ingredients in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical compositions of the invention can also be in the form of oil-in-water emulsions. The oily phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion can also contain sweetening and flavoring agents.

Syrups and elixirs can be formulated with sweetening agents, such as glycerol, sorbitol or sucrose. Such formulations can also contain a demulcent, a preservative, a flavoring or a coloring agent.

The pharmaceutical compositions of the invention can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent, such as a solution of 1,3-butanediol. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

The compounds of this invention can also be administered in the form of suppositories for rectal administration of the drug. These compositions can be prepared by mixing the drug with a suitable non-irritating excipient which is solid at ordinary temperatures but liquid at the rectal temperatures and will therefore melt in the rectum to release the drug. Such materials are cocoa butter and polyethylene glycols.

They can also be administered by in intranasal, intraocular, inntravaginal, and intrarectal routes including suppositories, immfflation, powders and aerosol formulations.

Products of the invention which are preferably administered by the topical route can be administered as applicator sticks, solutions, suspensions, emulsions, gels, creams, ointments, pastes, jellies, paints, powders, and aerosols.

The invention will be described in greater detail by way of specific examples The following examples relating to the compounds 63, 97b, 104a, 105a, 106a, 113c, 122a, 123a, 141 and 142b are embodiments of the present invention. All other examples are disclosed for illustrative or comparative purposes only.

## EXAMPLES

### EXAMPLE 1

This example illustrates the preparation and properties of 17α-Acetoxy11β-[4-(N-piperidino)phenyl]-19-norpregna-4,9-diene-3,20-dione (71) **(Figure 4).**

***Step 1. 3,20-bis-Ethylenedioxy-17α-hydroxy-19-norpregna-5(10),9(11)-diene (50):***

A mixture of 17α-hydroxy-19-norpregna-4,9-diene-3,20-dione (**92**, 10 g, 31.8 mmol), ethylene glycol (11.10 g, 178.7 mmol), freshly distilled triethyl orthoformate (14g, 94.1 mmol) and toluenesulfonic acid monohydrate (0.3 g, 1.58 mmol) in $CH_2Cl_2$ (150 mL) was stirred at room temperature under nitrogen overnight. Analysis by TLC (5% acetone in $CH_2Cl_2$) at that time indicated a complete reaction. Solid $NaHCO_3$ (~1 g) was added and the mixture was diluted with

$CH_2Cl_2$ (~100 mL) and poured into $H_2O$. The mixture was extracted with $CH_2Cl_2$ (3x). The organic fractions were washed with $H_2O$ (3x), filtered through sodium sulfate, combined and concentrated *in vacuo* to give 12 g of the crude product **50** as a yellow foam. Crystallization of this crude material from $CH_2Cl_2$/MeOH containing a trace of pyridine gave 9.8 g of the pure diketal **50** as a light yellow solid in 77% yield; m.p. 169 - 171°C. FTIR(KBr, diffuse reflectance) $\nu_{max}$ 3484 and 2912 $cm^{-1}$. NMR (300 MHz, $CDCl_3$): $\delta$ 0.792 (s, 3 H, C18-$CH_3$), 1.378 (s, 3 H, C21-$CH_3$), 3.816 and 4.047 (m, 4 H, C20-ketal), 3.983 (s, 4 H, C3-ketal) and 5.555 (m, 1H, C11-CH=), MS (EI) m/z (relative intensity): 402 ($M^+$,100.0), 366 (2.5), 340 (20.8) 270 (59.9) and 99 (50.1).

***Step 2. 3,20-bis-Ethylenedioxy-17α-hydroxy-5α,10α-epoxy-19-norpregna-9(11)-ene (51):***

Hydrogen peroxide (30%, 3.3 mL, 32.31 mmol) was added to a solution of hexafluoroacetone trihydrate (3.34 g, 16.17 mmol) in $CH_2Cl_2$ (53 mL) cooled to 0°C. Solid $Na_2HPO_4$ (1.48 g, 10.43 mmol) was added and the mixture stirred at 0°C for ½ hr. A solution of the 3,20-diketal (**50**, 6.0 g, 14.9 mmol) in $CH_2Cl_2$ (45 mL), precooled to 0°C, was added over a period of 10 min and the reaction mixture was stirred overnight at 5°C. Analysis by TLC (5% acetone in $CH_2Cl_2$) at that point indicated absence of the starting material. The reaction mixture was diluted with $CH_2Cl_2$ (~100 mL) and washed with 10% $Na_2SO_3$ solution (2x) and saturated $NaHCO_3$ solution (2x). The organic fractions were filtered through $Na_2SO_4$, combined and concentrated *in vacuo* to give 7 g of **51** of a white foam. Trituration of the epoxide mixture ($\alpha$ and $\beta$) with ether afforded 3.05 g of the pure 5α,10α-epoxide **51** as a white solid in 48.9% yield; m.p. =172-1.73°C. FTIR (KBr, diffuse reflectance) $\nu_{max}$ 3439, 2950, 1705, 1642 and 1593 $cm^{-1}$. NMR (300 MHz, $CDCl_3$) $\delta$ 0.789 (s, 3 H, C18-$CH_3$), 1.365 (s, 3 H, C21-$CH_3$), 3.810 - 4.094 (m, 8 H, C3- and C20-ketals) and 6.013 (m, 1H, C11-CH=). MS (EI) m/z (relative intensity): 418 ($M^+$, 0.5), 400 (1.4), 293 (0.9), 131 (2.5), 99 (4.3) and 87 (100.00).

***Step 3. 3,20-*bis*-Ethylenedioxy-5α,17α-dihydroxy-11β-[4-(N-piperidino)phenyl]-19-norpregn-9-ene (54):***

Magnesium (1.74 g, 71.7 mmoll) was weighed into a 250 mL round bottom two-neck flask equipped with a reflux condenser, a magnetic stirring bar and a rubber septum A small crystal of iodine was added and the system was flushed with dry nitrogen. The system plus contents were flame dried under nitrogen. The system was cooled to room temperature and freshly distilled THF (60 mL) was added via syringe. A small amount (~0.1 mL) of dry dibromoethane was added and the mixture stirred at room temperature. After evidence of reaction was observed (disappearance of $I_2$, color, bubble formation on the surface of magnesium), a solution of N-(4-bromophenyl)piperidine (Wolfe, J.P. and Buchwald, S.L., J. Org. Chem., 62:6066-6068 (1997); and Veradro, G. et al., Synthesis, 447-450 (1991)) (17.21 g, 71.7 mmol) in dry THF (40 mL) was added *via* syringe. The mixture was then stirred in a hot water bath for 3.5 hr, after which time the majority of the magnesium metal had reacted. The mixture was cooled to room temperature and copper (I) chloride (710 mg, 7.17 mmol) was added as a solid, and the mixture was then stirred in a hot water bath for 3.5 hr, after which time the majority of the magnesium metal had reacted. The mixture was cooled to room temperature and copper (I) chloride (710) mg, 7.17 mmol) was added as a solid and the mixture stirred at room temperature for ½ hr. The 5α,10α-epoxide (**51**, 6.0 g, 14.3 mmol) in dry THF (40 mL) was added *via* syringe and the mixture stirred at room temperature for ½ hr. At this time, a small aliquot of the reaction mixture was withdrawn, quenched with saturated $NH_4Cl$· solution, and extracted with a small amount. of EtOAc. A TLC (10% acetone in $CH_2Cl_2$) of the organic layer indicated the absence of the starting material. Saturated $NH_4Cl$ solution (~100 mL) was added to the reaction mixture, and the mixture was stirred at room temperature for ½ hr while air was drawn through the reaction mixture (to oxidize copper) via a 6 inch needle inserted through the rubber septum by applying a partial vacuum to the top of the condenser. The contents of the flask was diluted with $H_2O$ (~250 mL) and extracted with $CH_2Cl_2$ (3x). The organic fractions were washed with saturated $NH_4Cl$ solution (1x), $H_2O$ (1x), brine (1x) and then dried over anhydrous $Na_2SO_4$. The organic fraction was filtered and concentrated *in vacuo* to yield 26.8 g of an oil. The material was placed on a flash column and eluted and using 10% acetone in $CH_2Cl_2$ yielding 5.25 g of 54 as an off-white solid in 63.87% yield; m.p. = 211-214°C (sealed tube). FTIR (KBr, diffuse reflectance) $\nu_{max}$ 3508, 2933, 2790, 1609 1511, 1441, 1365 and 1234 $cm^{-1}$. NMR ($CDCl_3$) $\delta$ 0.45 (s, 3 H, C18-$CH_3$), 1.38 (s, 3 H, C21-$CH_3$), 3.05 - 3.2 (m, 4 H, -N-$(CH_2)_2$-), 3.8 - 4.05 (m, 8 H, 3- and 20-ketals), 4.1 (d, 1H, C11α-CH) and 6.8 - 7.1 (dd, 4 H, aromatic-CH's). Anal. Calcd. for $C_{35}H_{45}O_6N$: C, 72.51; H, 8.52; N, 2.41. Found: C, 71.84; H, 8.60; N, 2.46. MS (EI) m/z (relative intensity): 579 ($M_+$).

***Step 4. 17α-Hydroxy-11β-[4-(N-Piperidino)phenyl]-19-norpregna-4,9-diene-3,20-dione (63):***

Nitrogen was bubbled through a mixture of EtOH (120 mL) and $H_2SO_4$ (8.5%,15 mL) for ½ hr to remove oxygen. The Grignard adduct (54, 4.0 g, 6.89 mmol) was added as a solid with stirring. The mixture was put into an oil bath preheated to 95°C for ½ hr. The mixture was cooled in an ice bath and quenched with saturated $K_2CO_3$ (pH = ~10). The reaction mixture was diluted with $H_2O$ (250mL) and extracted with $CH_2Cl_2$ (3x). The organic fractions were washed with saturated $NaHCO_3$ -(1x), $H_2O$ (1x), brine (1x), combined, dried over anhydrous $Na_2SO_4$ and concentrated *in vacuo* to

give 3.35 g of a foam. This material was purified by flash column chromatography using 10% acetone in $CH_2Cl_2$ to yield 2.95 g of a crude product (**63**) which was crystallized from $CH_2Cl_2$ and ether to yield 2.45 g of an off-white crystalline product (**63**) in two crops in 61.4% yield; m.p. = 219-221°C. FTIR. (KBr, diffuse reflectance) $\nu_{max}$ 3433, 2942, 1708, 1654, 1605, 1512 and 1234 $cm^{-1}$. HMR ($CDCl_3$) $\delta$ 0.45 (s, 3 H, C18-Me), 2.25(s, 3 H, C21-Me), 3.05 - 3.2 (m, 4 H, -N-$(CH_2)_2$-), 4.35 (d, 1H, C11$\alpha$-CH), 5.75 (s, 1H, C4-CH=), 6.8 - 7.0 (dd, 4 H, aromatic-CH). MS (EI) m/z (relative intensity): 161 (100), 174 (11.43) and 473 (75.71, $M^+$). Anal. Calcd. for $C_{31}H_{39}O_3N$: C, 78.61; H, 8.30; N, 2.96. Found: C, 77.59; H, 8.29; N, 3.03.

***Step 5. Preparation of the target compound <u>71</u>:***

The diketone (**63**,1.7 g, 3.59 mmol) was dissolved in $CH_2Cl_2$ (50 mL) and cooled to 0°C in an ice bath. In a separate round bottom flask, trifluoroacetic anhydride (15.11g, 71.78 mmol) and acetic acid (4.75 g, 71.78 mmol) were added to $CH_2Cl_2$ (100 mL), flushed with dry nitrogen and stirred at room temperature for ½ hr. This mixed anhydride was then placed in an ice bath and cooled to 0°C. The cold mixed anhydride solution was then added to the steroid solution and treated with *p*-toluenesulfonic acid (628 mg, 3.3 mmol). The reaction mixture was stirred for 2 hr at 0°C. The reaction was quenched with saturated $K_2CO_3$ (pH = ~10), diluted with $H_2O$ and extracted with $CH_2Cl_2$ (3x). The organic layers were washed with $H_2O$ (2x) and brine (1x), dried over $Na_2SO_4$, filtered and concentrated to yield 3.38 g of crude material. A flash column using 10% acetone in $CH_2Cl_2$ yielded 1.66 g of **71** as an off-white solid in 54.1% yield. The crude product **71** was recrystallized from $CH_2Cl_2$ and $Et_2O$. The material retained $CH_2Cl_2$ and was dried in a heating pistol *in vacuo* over refluxing benzene for 5 days to afford 895 mg of **71** as an off-white solid in 48.4% yield; m.p. =175-183°C (sealed tube). FTIR (KBr, diffuse reflectance) $\nu_{max}$ 2936, 1733, 1717, 1654, 1609, 1512, 1450, 1372, 1259 and 1235 $cm^{-1}$. NMR (300 MHz, $CDCl_3$) $\delta$ 0.340 (s, 3 H, C18-Me), 2.091 (s, 3 H, C17-OAc), 2.131 (s, 3 H, C21-$CH_3$), 3.120 (m, 4H, -N-$(CH_2)_2$-), 4.370 (m, 1H, C11$\alpha$-CH), 5.778 (s, 1H, C4-CH=) and 6.810 - 7.000 (m, 4 H, aromatic-CH's). MS(EI) m/z (relative intensity): 161(100), 174(11.11) and 515 ($M^+$, 59.72).

Reverse-phase HPLC analysis on Waters NovaPak $C_{18}$ column eluted with MeOH:$H_2O$ in the ratio of 70:30 with 0.05% TEA at a flow rate of 1 mL/min and at 260 nm indicated it to be 99.5% pure. Anal. Calcd. for $C_{31}H_{41}O_4N$:½EtOH: C, 76.86; H, 8.01; N, 2.72. Found: C, 76.64; H, 8.06; N, 2.69.

**EXAMPLE 2**

This example illustrates the preparation and properties of 17$\alpha$-Acetoxy-1$\beta$-[4-N-Piperidino)phenyl]-19-norpregna-4,9-diene-3,20-dione (97b) (Figure 6):

***Step 1. 17$\alpha$-Hydroxy-19-norpregna-4,9-diene-3,20-dione <u>(92)</u>:***

Under nitrogen, the diketal (**50**, 20.0 g, 49.7 mmol) was dissolved in a mixture of THF (333 mL) and $H_2O$ (333 mL) followed by trifluoroacetic acid (1 L, 13.46 mmol). The reaction mixture was then stirred at room temperature for 2 hr, after which time, TLC (10% acetone in $CH_2Cl_2$, overspotted with concentrated $NH_4OH$ indicated a complete reaction. The reaction mixture was cooled in an ice bath and neutralized by the dropwise addition of concentrated (29.5%) $NH_4OH$ (862 mL, ~13.46 mol) over a period of about an hour. The reaction mixture was diluted with $H_2O$ (~500 m L) and extracted with methylene chloride (3x). The organic fractions were washed with saturated $NaHCO_3$ (1x) and $H_2O$ (1x), brine (1x), then filtered through anhydrous sodium sulfate, combined and concentrated *in vacuo.* Crystallization of the residue from acetone/hexanes gave 12 g of the pure product **92** as a white crystalline solid in 76.8% yield; m.p. = 203-205°C. FTIR (KBr, diffusion reflectance) $\nu_{max}$ 3438, 2950, 1702, 1642 and 1593 $cm^{-1}$. $^1H$ NMR (300 MHz, $CDCl1_3$) $\delta$ 0.857 (s, 3 H, C18-$CH_3$), 2.289 (s, 3 H, C21-$CH_3$) and 5.669 (s,1 H, C4-CH=). $^{13}C$ NMR ($CDCl_3$): $\delta$ 14.703, 23.901, 25.341, 25.714, 27.515, 27.615, 30.260, 30.765, 33.470, 36.971, 39.086, 47.846, 50.696. 89.565 (C17), 122.015 (C4), 125.440(C10). 145.632 (C9). 157.339 (C5), 199.824 (C3) and 211.201 (C20). MS (BI) m/z (relative intensity): 314 ($M^+$, 100), 296 (13.6), 271 (58.0), 213 (67.0) and 91 (35.9). Anal. Calcd. for $C_{20}H_{26}O_3$: C, 76.40; H, 8.34. Found: C, 76.23; H, 8.29.

***Step 2. 17$\alpha$-Methoxy-19-norpregna-4,9-diene-3,20-dione (<u>93</u>):***

A suspension of the 17$\alpha$-hydroxy dienedione (**92**, 19 g, 31.80 mmol) in $CH_3CN$ (167 mL) was stirred magnetically under nitrogen. Methyl iodide (134 mL; freshly opened) was added and a solution formed immediately. Silver oxide (8.1 g, 35.0 mmol) was added, the joints were well-greased to prevent evaporation of methyl iodide, and the flask was wrapped in foil to protect the contents from light. The mixture was brought to a gentle reflux and the reaction allowed to proceed overnight. The next morning, analysis by TLC (5% acetone in $CH_2Cl_2$) indicated virtually all the starting material had been converted to a single, less polar component. The reaction was allowed to cool to room temperature and filtered through a Celite filter cake on a sintered glass funnel. The filtrate was evaporated *in vacuo* to recover a thick syrup.

Crystallization from boiling $CH_3OH$ afforded small white crystals. The crystals were collected on a Buchner funnel, triturated with cold $CH_3OH$, and dried under vacuum to recover 5.74 g. Flash chromatography of the mother liquors (5% acetone in $CH_2Cl_2$) afforded 1.69 g of additional material. The total purified product recovered was 7.43 g of **93** as white crystals in 71.1% yield; m.p. = 154-155°C. FTIR (KBr, diffuse reflectance) $\nu_{max}$ 2952, 1704, 1660, 1614 and 1583 $cm^{-1}$. $^1H$ NMR (300 MHz, $CDCl_3$) $\delta$ 0.739 (s, 3 H, C18-$CH_3$), 2.164 (s, 3 H, C21-$CH_3$), 3.141 (s, 3 H, C17$\alpha$-$OCH_3$) and 5.672 (s, 1 H, C4-CH=). $^{13}C$ NMR ($CDCl_3$): $\delta$ 14.264, 23.156, 23.431. 23.775. 25.547. 25.753. 26.431. 27.445. 30.755, 30.793. 37.054, 39.220, 47.243, 51.348. 52.258. 96.714 (C17), 122.057 (C4),125.228 (C10), 145.588 (C9), 157.192 (C5),199.637 (C3) and 210.479 (C20). MS (EI) m/z (relative intensity): 328 ($M^+$, 5.8), 285 (66), 253 (64) and 213 (100). Anal. Calcd, for $C_{21}H_{28}O_3$: C, 76.79; H, 8.59, Found: C, 76.64; H, 8.59.

***Step 3. 3, 3-Ethylenedioxy-17α-methoxy-19-norpregna-5(10),9(11)-dien-20-one (94):***

Under nitrogen, a mixture of the 17α-methoxydione (**93**, 17.0 g, 51.76 mmol), triethylorthoformate (42.5 mL, 250 mmol), ethylene glycol (14 mL), 250 mmol) and *p*-toluenesulfonic acid monohydrate (0.5 g, 2.6 mmol) in dry $CH_2Cl_2$ (500 mL) was stirred at room temperature for 2 hr. After that time, TLC (2% acetone in $CH_2Cl_2$) indicated absence of starting material with formation of one major product. The reaction mixture was diluted with $CH_2Cl_2$ (~200 mL) and washed with saturated $NaHCO_3$ solution (1x), $H_2O$ (1x) and brine. The organic fractions were filtered through anhydrous sodium sulfate, combined and concentrated *in vacuo*. Recrystallization of the residue from hot methanol containing a trace of pyridine gave 16.2 g of the pure 3-ketal 94 as a white solid in 84.1% yield; m.p. = 123-125°C. FTIP, (KBr, diffuse reflectance) $\nu_{max}$ 2927 and 1705 $cm^{-1}$. $^1H$ NMR (300 MHz, $CDCl_3$) $\delta$ 0.553 (s, 3 H, C18-$CH_3$), 2.147 (s, 3 H, C21-$CH_3$), 3.147 (s, 3 H, C17$\alpha$-$OCH_3$), 3.983 (s, 4 H, C3-ketal) and 5.568 (br s, 1 H, C11-CH=). $^{13}C$ NMR ($CDCl_3$): $\delta$ 15.746, 23.123, 24.026, 24.570. 26.422. 27.972, 31.150, 31.298, 31.839, 38.233, 41.238, 46.079, 47.391, 52.318, 64.325, 64.448, 96.792, 108.131, 117.907, 126.081,129.914 and 135.998 (signal/noise ratio obscured C20 at ~210 ppm). Anal. Calcd. for $C_{23}H_{32}O_4$: C, 74.16; H, 8.66. Found: C, 74.16; H, 8.68.

***Step 4. 3,3-Ethylenedioxy-5α,10α-epoxy-17α-methoxy-19-norpregn-9(11)-en-20-one (95):***

Hydrogen peroxide (30% 3.0 mL, 29.3 mmol) was added to a vigorously stirred mixture of hexafluoroacetone trihydrate (4.0 mL, 28.7 mmol) in $CH_2Cl_2$ (70 mL) cooled to 0°C in an ice bath. After stirring at 0°C for ½ hr, solid $Na_2HPO_4$ (2.1 g, 14.8 mmol) was added followed by a solution of the 3-ketal (**94**, 7.0 g, 18.8 mmol) in $CH_2Cl_2$ (70 mL), precooled to 0°C. The mixture was then stirred at 5°C overnight. The reaction mixture was diluted with $CH_2Cl_2$ (~200 mL) and washed with 10% $Na_2SO_3$ solution (1x) and $H_2O$ (2x). The organic fractions were filtered through anhydrous $Na_2SO_4$, combined and concentrated *in vacuo* to give 7.29 g of **95** as a white foam in quantitative yield. Attempts to crystallize out the 5α,10α-epoxide by trituration with ether/pentane or mixtures of $CH_2Cl_2$ and pentane were unsuccessful. Analysis by NMR indicated a 4:1 mixture of the 5α,10α- and the 5β,10β-epoxides. NMR (300 MHz, $CDCl_3$): $\delta$ 0.554 (s, 3 H, C18-$CH_3$), 2.139 (s, 3 H, C21-$CH_3$); 3.8 - 4.0 (m, 4 H, C3-ketal $CH_2$'s), 5.845 (m, 0.2 H, C11-CH= of β-epoxide) and 6.034 (m, 0.8H, C11-CH= of α-epoxide).

***Step 5 3,3-Ethylenedioxy-5α-hydroxy-17α-methoxy-11β-[4-(N piperidino)phenyl]-19-norpregna-5(10),9(11)-dien-20-one (96b):***

Magnesium (845 mg, 34.7 mmol) was added to a 500 mL, 3-neck flask equipped with a reflux condenser, a magnetic stirrer and a rubber septum.. A small crystal of iodine was added and the system flushed with nitrogen and flame dried. After cooling, dry THF (20 mL) and 1,2-dibromoethane (0.2 mL) were added. The mixture was stirred under nitrogen and heated in a warm water bath until evidence of the reaction was observed. A solution of N-(4-bromophenyl)piperidine (Veradro, et al., Synthesis, 447-450 (1991)) (8.35 g, 34.7 mmol) in dry THF (30 mL) was then added *via* syringe and the mixture stirred and heated in a warm water bath for 3 ½ hr. Solid copper (I) chloride (688 mg, 6.95 mmol) was added followed ½ hr later by a solution of the epoxide mixture (95, 2.7 g, assumed 6.95 mmol) in dry THF (30 mL). The reaction mixture was stirred at room temperature for 45 min, then quenched by the addition of saturated $NH_4Cl$ solution. In order to oxidize Cu (I) to Cu (II), air was drawn through the reaction mixture for ½ hr with vigorous stirring. The mixture was then extracted with $CH_2Cl_2$ (3x). The organic fractions were washed with saturated $NH_4Cl$ solution, $H_2O$ and brine, combined, dried over $Na_2SO_4$, filtered and concentrated *in vacuo* to give 11.3 g of the residue as a dark oil. The material was purified *via* flash chromatography (5% acetone in $CH_2Cl_2$) twice to give 1.22 g of the Grignard adduct **96b** as a white foam in 32% yield; m.p. =126 - 131°C (dec). FTIR (KBr, diffuse reflectance) $v_{max}$ 3523, 2938, 1707, 1610, 1511 and 1447 $cm^{-1}$. NMR (300 MHz, $CDCl_3$) $\delta$ 0.207 (s, 3H, C18-Me), 1.682 (m, 6 H, -$(CH_2)_3$- of piperidine), 2.147 (s, 3H, C21-$CH_3$), 3.103 (s, 3 H, C17$\alpha$-$OCH_3$), 3.05 - 3.2 (m, 4 H, -N$(CH_2)_2$-), 3.8 - 4.05 (m, 4 H, C3-ketal), 4.23 (m, H, C11$\alpha$-CH) and 6.78 - 7.05 (dd, 4 H, aromatic-CH's). MS (EI) m/z (relative intensity): 549 ($M^+$, 59.7), 531 (18.1), 174 (20.8), 161 (100) and 99 (11.1). Anal. Calcd. for $C_{34}H_{47}O_5N$: C, 74.28; H, 8.62; N, 2.55. Found: C, 73.45; H, 8.51;

N, 2.53.

***Step 6. Preparation of the target compound* <u>*97b*</u>*:***

Under nitrogen, a solution of the Grignard adduct (<u>**96b**</u>, 1.0 g, 1.81 mmol) in THF (10 mL) was treated with $H_2O$ (10 mL) and glacial HOAc (30 mL). After stirring overnight at room temperature, TLC (5% acetone in $CH_2Cl_2$) indicated incomplete deketalzation and dehydration. The reaction mixture was heated to ~50°C in a warm water bath for 2 hr, after which time TLC indicated a complete reaction. The mixture reaction was cooled in an ice bath and neutralized with the addition of concentrated $NH_4OH$ (~35 mL). The mixture was then further diluted with $H_2O$ (~100 mL) and extracted with $CH_2Cl_2$ (3x). The organic fractions were washed with $H_2O$, brine, combined, dried over $Na_2SO_4$, and concentrated *in vacuo* to give 900 mg of foam. The crude material was purified by flash chromatography (5% acetone in $CH_2Cl_2$) to give 630 mg of the target compound <u>**97b**</u> as a foam. Recrystallization of the compound <u>**97b**</u> from EtOH afforded 325 mg of the target compound <u>**97b**</u> as an off-white solid in 35.7% yield. HPLC analysis of <u>**97b**</u> on a Waters NovaPak $C_{18}$ column eluted with MeOH/$H_2O$ (80:20) with 0.05% $Et_3N$ at a flow rate of 1 mL/min and $\lambda$ = 260 nm indicated this compound to be 97.7% pure. FTIR (KBr, diffuse reflectance) $\nu_{max}$ 2934,1708,1665,1610 and 1512 $cm^{-1}$. NMR (300 MHz, $CDCl_3$) $\delta$ 0.273 (s, 3 H, C18-$CH_3$), 2.174 (s, 3 H, C21-$CH_3$), 3.139 (s, 3 H, C17$\alpha$-$OCH_3$), 4.35(d, H, C11$\alpha$-CH), 5.746 (s, 1 H, C4-CH=) and 6.8 - 7.0 (dd, 4 H, aromatic-CH's). MS (EI) m/z (relative intensity): 487 (84.3), 412 (4.3), 318 (8.6), 251 (7.14), 206 (11.4), 174 (15.7) and 161 (100).' Anal. Calcd. for $C_{32}H_{41}O_3N$: C, 78.85; H, 8.42; N, 2.87. Found: C, 78.00; H, 8.37; N, 3.00.

**EXAMPLE 3**

This example illustrates the preparation and properties of 17$\alpha$,21-Diacetoxy-11$\beta$-[4-(N-piperidino)phenyl]-19-norpregna-4,9-diene-3,20-dione (<u>**106a**</u>) **(Figure 7)**:

***Step 1. 3, 3-Ethylenedioxy-17$\beta$-cyano-17$\alpha$-trimethylsilyloxyesttra-5(10),9(11)-diene* <u>*(99):*</u>**

Under nitrogen, pyridine (136.9 g, 1740 mmol) solution of the cyanohydrin ketal (<u>**98**</u>, 25 g, 73.22 mmol) was treated with chlorotrimethylsilane (44g, 394 mmol). The mixture was stirred at room temperature overnight. The reaction mixture was poured into a 50:50 mixture of ice/saturated $NaHCO_3$ solution (~1.2 L), stirred until the ice had melted, and extracted with hexane (3x). The organic extracts were washed with $H_2O$ (3x), brine (1x), combined, dried over anhydrous $Na_2SO_4$, and concentrated *in vacuo.* The remaining pyridine was azeotropically removed *in vacuo* with heptane. Crystallization of the residue from pentane gave 26.1 g of the pure silyl ether (<u>**99**</u>) as a white solid in 86.2% yield; m.p.= 99 - 101°C. FTIR (KBr, diffuse reflectance) $\nu_{max}$ 2944, 2908, 2231 and 1253 $cm^{-1}$. NMR. (300 MHz, $CDCl_3$) 8 0.229 (s, 9 H, C17$\alpha$-$OSi(CH_3)_3$), 0.894 (s, 3 H, C18-$CH_3$), 3.987 (s, 4 H, 3-$OCH_2CH_2O$) and 5.615 (t, 1 H, J = 2.55 Hz, C11-CH=). MS (EI) m/z (relative intensity): 413 ($M^+$, 100.0), 398 (5.5), 385 (24.0), 371 (6.4), 237 (33.9) and 69.3 (86.0).

***Step 2. 3; 3-Ethylenedioxy-5$\alpha$,10$\alpha$-epoxy-17$\beta$-cyano-17$\alpha$-trimethylsilyloxyestr-9(11)-ene (100):***

Hydrogen peroxide (30%, 12 mL, 117.12 mmol) was added to a vigorously stirred mixture of hexafluoroacetone trihydrate (20.20 g, 112.5 mmol) in $CH_2Cl_2$ (185 mL) cooled to 0°C in an ice bath. The reaction mixture was stirred at 0°C for ½ hr, and solid $Na_2HPO_4$ (11g, 77.5 mmol) was added followed by an ice-cold solution of the silyl ether (<u>**99**</u>,25 g, 60.44 mmol) in $CH_2Cl_2$ (185 mL). The mixture was then stirred at 0°C for 5 hr, then at 5°C overnight. Analysis by TLC (5% acetone in $CH_2Cl_2$) at that time indicated a complete reaction, The reaction mixture was diluted with $CH_2Cl_2$ (~200 mL) and washed with 10% $Na_2SO_3$ solution (1x), $H_2O$ (1x) and brine (1x). The organic fractions were filtered through anhydrous sodium sulfate, combined and concentrated *in vacuo*. Trituration of the residue with ether afforded 16.66 g of the pure 5$\alpha$,10$\alpha$-epoxide (<u>**100**</u>) as a white solid in 64.16% yield; m.p. 156-160°C. FTIR (KBr, diffuse reflectance) $\nu_{max}$ 2955, and 2228 $cm^{-1}$. NMR (300 MHz, $CDCl_3$) 8 0.219 (s, 9 H, $OSi(CH_3)_3$), 0.894 (s, 3 H, C18-$CH_3$), 3.85 - 3.97 (s, 4 H, C3-$OCH_2CH_2O$) and 6.082 (t, 1H, J = 2.6 Hz, C11-CH=). MS(BI) m/z (relative intensity): 429 ($M^+$, 18.5), 401(2.8), 343 (11.1), 238 (9.5), 99 (100.0) and 86 (36.2),

**Step *3. 3,3-Ethylenedioxy-5$\alpha$-hydroxy-11$\beta$-[4-(N-piperidino)phenyl]-17$\beta$-cyano-17$\alpha$-trimethyl- silyloxyester-9-ene (<u>101a</u>):***

Magnesium (0.95 g, 39.1 mmol) was added to a 500 mL, 3-neck flask equipped with a magnetic stirrer, rubber septum and a condenser. A crystal of iodine was added followed by dry THF (50 mL) and two drops of 1,2-dibromoethane. A solution of N-(4-bromophenyl)piperidine (*see*, EXAMPLE 2, Step 5) (10.24 g, 42.64 mmol) in dry THF (50 mL) was then added, and the mixture was stirred under nitrogen and heated to reflux for 1 hr. At the end of that time, all of the

magnesium metal had reacted. The reaction was allowed to cool to room temperature, and solid copper (I) chloride (0.7 g, 7.07 mmol) was added followed ½ hr later by a solution of the 5α,10α-epoxide (**100**, 5.55 g, 12.92 mmol) in dry THF (50 mL). The mixture was stirred at room temperature for 1.5 hr. Analysis by TLC (5% acetone in $CH_2Cl_2$) of a small aliquot quenched with $NH_4Cl$ solution and extracted with EtOAc indicated a complete reaction. The reaction mixture was cooled in an ice bath and quenched by the addition of saturated $NH_4Cl$ solution (15 mL). The reaction mixture was allowed to warm to room temperature, and air was drawn through the reaction mixture for ½ hr to oxidize Cu(I) to Cu(II). The mixture was extracted wtih $CH_2Cl_2$ (3x) and the organic fractions washed with $H_2O$ (3x). The organic fractions were combined, dried over anhydrous $Na_2SO_4$, filtered and concentrated *in vacuo.* Trituration of the residue with pentane gave 7.37 g of **101a** as an off-white solid in 97% yield.; m.p.=127 - 130°C. FTIR (KBr, diffuse reflectance) $\nu_{max}$ 3510, 2945, 2228, 1611 and 1510 $cm^{-1}$. NMR (300 MHz, $CDCl_3$) δ 0.241 (s, 9 H, 17α-OSi(CH3)$_3$, 0.533 (s, 3 H, C18-CH3), 3.107 (t, 4 H, J = 5.6 Hz, piperidine α-$CH_2$'s), 3.884 - 4.043 (s, 4 H, C3-$OCH_2CH_2O$) 4.284 (d, 1H, J = 6.9 Hz, C11α-CH), 6.831 (d, 2H, J=8.7 Hz, 3', 5' aromatic-CH's) and 7.060 (d, 2H, J= 8.7 Hz, 2', 6' aromatic-CH's). MS(EI) m/z (relative intensity): 590 ($M^+$, 38.1), 572 (10.3), 320 (4.0), 174 (12.1), 161 (100.0),100 (1.7), 99 (7.8) and 71 (7.0) Anal. Calcd. for $C_{35}H_{50}N_2O_4Si \cdot 1/3C_5H_{11}$: C, 71.61; H, 8.85; N, 4.56. Found: C, 71.79; H, 8.89; N, 4.49.

***Step 4. 17β-cyano-11β-[4-(N-piperidino)phenyl]-17α-hydroxyestra-4,9-dien-3-one (102a):***

A solution of the Grignard adduct (**101a,** 7.27 g, 12.3 mmol) was dissolved in THF (25 mL) and the system was flushed with nitrogen. Glacial acetic acid (75 mL) and $H_2O$ (25 mL) were added and the mixture was heated to 65°C for 3 hr. Analysis by TLC (5% acetone in $CH_2Cl_2$) at that time indicated a complete reaction. The mixture was cooled to 0°C in an ice bath and the acetic acid was neutralized by slow addition of concentrated $NH_4OH$ solution (28%, ~90 mL) to a final pH of ~8 by pH paper. The mixture was diluted with $H_2O$ and extracted with $CH_2Cl_2$ (3x). The organic fractions were washed with $H_2O$ (3x), filtered through anhydrous $Na_2SO_4$, combined and concentrated *in vacuo.* Trituration of the residue with ether gave 3.8 g of the cyanohydrin (**102a**) as a white crystalline solid. The mother liquors were concentrated and purified by flash column chromatography (5% acetone in $CH_2Cl_2$) to afford an additional 0.65 g of **102a** after trituration with pentane. Total yield of the cyanohydrin (**102a**) was 4.45 g in 79.2% yield; m.p. = 205 - 208°C. FTIR (KBr, diffuse reflectance): $v_{max}$ 3436, 3211, 2939, 2855, 2234, 1658, 1634, 1609 and 1512 $cm^{-1}$. NMR (300 MHz, $CDCl_3$): δ 0.641 (s, 3 H, C18-$CH_3$), 3.125 (t, 4 H, J = 5.7 Hz, piperidine α-$CH_2$'s), 4.427 (d, 1 H, J = 5.1 Hz, Cl 1α-CH), 5.782 (s, 1 H, C4-CH=), 6.862 (d, 2 H, J = 9 Hz, 3', 5' aromatic-CH's) and 7.031 (d, 2H, J = 9 Hz, 2', 6' aromatic-CH's). MS (EI) m/z (relative intensity): 456 ($M^+$, 0.3), 429 (61.1), 401 (1.5), 174 (6.9), and 161 (100.0). Anal. Calcd. for $C_{30}H_{36}N_2O_2 \cdot 1/10H_2O$: C, 78.60; H, 7.96; N,6.11. Found: C, 78.64; H, 7.94; N, 6.11.

***Step 5. 17β-cyano-11β-[4-(N-piperidino)phenyl]-17α-chloromethyldimethylsilyloxyestra-4,9-dien-3-one (103a):***

Under nitrogen, a solution of the cyanohydrin (**102a**, 4.39 g, 9.61 mmol) and dimethylaminopyridine (0.4 g, 3.27 mmol) in dry THF (50 mL) and triethylamine (1.8 g, 17.79 mmol) was treated with chloromethyldimethylsilyl chloride (2.0 mL = 2.17 g, 15.18 mmol). After stirring overnight at room temperature, TLC (2% acetone in $CH_2Cl_2$) indicated a complete reaction. The reaction was diluted with ether (50 mL) and stirred for an additional ½ hr. The resulting suspension was filtered through Celite and the filtrate concentrated *in vacuo*. The residue was taken up in ether/$CH_2Cl_2$ (9: 1) and the solution/suspension was passed through a silica gel flash chromatography column using ether as eluent. Fractions containing the product were combined and concentrated in *vacuo* to give 5.4 g of the chloromethyl silyl ether (**103a**) as a white foam in quantitative yield. Attempts to crystallize or soldify the crude product using a variety of solvents were unsuccessful. This material was used in the subsequent reaction without further purification. NMR (300 MHz, $CDCl_3$): δ 0.403 and 0.410 (both s, 6 H; $OSi(CH_3)_2$), 0.607 (s, 3 H, C18-$CH_3$), 2.904 (s, 2 H, $(CH_3)_2Si\underline{CH_2}Cl$), 3.123 (t, 4 H, J = 5.6 Hz, piperidine α-$CH_2$'s), 4.399 (d, 1 H, J = 6 Hz, C11α-CH), 5.775 (s, 1 H, C4-CH=), 6.863 (d, 2 H, J = 8.6 Hz, 3', 5' aromatic-CH's) and 7.027 (d, 2 H, J = 8.6 Hz, 2', 6' aromatic-CH's).

***Step 6. 17α-Hydroxy-11β-[4-(N-piperidino)phenyl]-21-chloro-19-norpregna-4,9-diene-3,20-dione (104a):***

Under nitrogen and anhydrous conditions, a solution of the chloromethyl silyl ether (**103a**, 5.1 g, 9.05 mmol) in dry THF (150 mL) was cooled to -78°C, and treated dropwise with a 2.0 M solution of lithium diisopropylamide (LDA) in THF/heptane (19 mL, 38 mmol). The reaction was stirred at -78°C for 2 hr and then quenched at -78°C by the slow addition of 4 N HCl (100 mL, 400 mmol). The mixture was allowed to warm and stirred at room temperature for 1 hr. The reaction was cooled to 0°C and the excess acid was neutralized by slow addition of concentrated $NH_4OH$ solution (~25 mL). The reaction mixture was diluted with $H_2O$ (~100 mL) and extracted with $CH_2Cl_2$ (3x). The organic fractions were washed with $H_2O$ (2x), filtered through anhydrous $Na_2SO_4$, combined and concentrated *in vacuo* to give 5.6 g of a residue as a yellow foam.

This material was triturated with EtOAc to give 2.64 g of the pure 21-chloro product (**104a**) as a yellow solid.

Concentration of the mother liquors followed by flash column chromatography (7.5% acetone in $CH_2Cl_2$) and trituration with EtOAc gave an additional 0.54 g of the product. Total yield of the 21-chloro intermediate (**104a**) was 3.18 g in 69.17% yield; m.p. = 231- 234°C. FTIR (KBr, diffuse reflectance): $v_{max}$ 3395, 2939,1730, 1649,1602 and 1512 $cm^{-1}$. NMR (300 MHz, $CDCl_3$): δ 0.382 (s, 3 H, C18-$CH_3$), 3.104 (t, 4 H, J = 5.4 Hz, piperidine α-$CH_2$'s), 4.343 and 4.614 (dd, 25 H, J = 16.5 Hz, C21-$CH_2$), 4.380 (d, 1 H, J = 6.0 Hz, C11α-CH), 5.762 (s, 1 H, C4-CH=), 6.826 (d, 2 H, J = 8.9 Hz, 3', 5' aromatic-CH's) and 6.981 (d, 2 H, J = 8.9 Hz, 2', 6' aromatic-CH's). MS(EI) m/z (relative intensity): 507 ($M^+$, 23.7), 471 (18.0), 318 (6.5), and 161 (100.0). Anal. Calcd. for $C_{31}H_{38}ClNO_3 \cdot 1/6CH_2Cl_2$: C, 71.06; H, 743; N, 2.66; Cl, 8.98. Found: C, 71.06; H, 7.55; N, 2.73; Cl, 8.78.

***Step 7. 17α-Hydroxy-11β-[4-(N-piperidino)phenyl]-21-acetoxy-19-norpregna-4,9-diene-3,20-dione (105a):***

The 21-chloro intermediate (**104a**, 3.0 g, 5.9 mmol) and anhydrous potassium acetate (6.0 g, 61.14 mmol) in dry $CH_3CN$ (75 ml) was heated to reflux under nitrogen and monitored by TLC (10% acetone in $CH_2Cl_2$) which indicated a complete reaction after 3 hr. The reaction mixture was cooled to room temperature, diluted with $CH_2Cl_2$ (~50 mL), filtered and concentrated *in vacuo* to give 4.1 g of the residue as a yellow solid. This material was crystallized from $CH_2Cl_2$ / acetone to give 2.63 g of the pure 17a-ol-21-acetate (**105a**) as an off-white solid in 83.8% yield; m.p. = 277 - 281°C. FTIR (KBr, diffuse reflectance): $v_{max}$ 3440, 2937, 1742, 1727, 1648, 1601 and 1513 $cm^{-1}$. NMR (300 MHz, $CDCl_3$): δ 0.379 (s, 3 H, C18-$CH_3$), 2.174 (s, 3 H, C21-OAc), 3.101 (t, 4 H, J = 5.4 Hz, piperidine α-$CH_2$'s), 4.376 (d, 1 H, J = 6.6 Hz, C11α-CH), 4.864 and 5.106 (dd, 2 H, J = 17.3 Hz, C21-$CH_2$), 5.762 (s, 1 H, C4-CH=), 6.836 (d, 2 H, J = 9 Hz, 3', 5' aromatic-CH's) and 7.016 (d, 2 H, J = 9 Hz, 2', 6' aromatic-CH's). MS(EI) m/z (relative intensity): 531 ($M^+$, 28.3), 513 (2.9), 501 (3.2), 471 (7.4), 174 (11.6) and 161 (100.0). Anal. Calcd. for $C_{33}H_{41}NO_5 \cdot 1/5CH_2Cl_2$: C, 72.68; H, 7.61; N, 2.55. Found: C, 72.73; H, 7.53; N, 2.70.

**Step 8.** ***Preparation of the target compound <u>106a</u>:***

A mixture of trifluoroacetic anhydride (7.9 g, 37.6 mmol) and glacial acetic acid (2.21 g, 36.7 mmol) in dry $CH_2Cl_2$ (25 mL) was stirred at room temperature under nitrogen for 1 hr. *p*-Toluenesulfonic acid monohydrate (0.79 g, 4.15 mmol) was added, and the mixture was cooled to 0°C in an ice bath. A solution of the 17α-ol-21-acetate (**105a**, 2.0 g, 3.76 mmol) in dry $CH_2Cl_2$ (35 mL) was added and the reaction mixture stirred at 0°C for 2.5 hr. Assays by TLC (5% acetone in $CH_2Cl_2$) at that time indicated >90% of the starting material had been consumed. $H_2O$ (~10 mL) was added and the reaction stirred at 0°C for 10 min. Additional $H_2O$ (~50 mL) was added and the reaction allowed to warm to room temperature. The pH of the reaction mixture was carefully adjusted to 9.0 with concentrated $NH_4OH$ and the mixture was extracted with $CH_2Cl_2$ (3x). The organic fractions were washed with $H_2O$ (2x), brine (1x), filtered through anhydrous $Na_2SO_4$, combined and concentrated *in vacuo* to give 2.3 g of a yellow foam. Purification of this crude **106a** by flash chromatographies (7.5% acetone in $CH_2Cl_2$) followed by crystallization from ether gave the 17α,21-diacetate **106a** in two crops, both as white crystalline solids. *Crop 1* (0.68 g), m.p. = 188 - 189°C. Crop 2 (0.672 g), m.p. = 186 - 188°C. Total was 1.352 gin 62.6% yield. Analysis of **106a** by HPLC on a Water Associates NovaPak $C_{18}$ eluted with $CH_3CN$ / 0.05 <u>M</u> $KH_2PO_4$ [pH = 3.0] at a flow rate of 1 mL per minute and λ = 302 nm) indicated the first crop to be 99.1% pure and the second crop to be 98.1% pure. FTIR (KBr, diffuse reflectance): $v_{max}$ 2939, 2858, 2793, 1748, 1729, 1669, 1600 and 1509 $cm^{-1}$. NMR (300 MHz, $CDCl_3$): δ 0.417 (s, 3 H, C18-$CH_3$), 2.125 (s, 3 H, C17α-OAc), 2.168 (s, 3H, C21-OAc), 3.104 (t, 4H, J = 5.35 Hz, piperidino α-$CH_2$'s), 4.386 (d, 1 H, J = 6.6 Hz, C11α-CH), 4.403 and 4.946 (dd, 2H, J = 16.8 Hz, C21-<u>$CH_2$</u>OAc), 5.781 (s, 1 H, C4-CH=), 6.832 (d, 2 H, J = 9 Hz, 3', 5' aromatic-CH's) and 7.011 (d, 2 H, J = 9 Hz, 2', 6' aromatic-CH's). MS (EI) m/z (relative intensity): 573 ($M^+$, 46.3), 513 (11.5), 174 (10.4) and 161 (100.0). Anal. Calcd. for $C_{35}H_{43}NO_6$: C, 73.27; H, 7.55; N, 2.44, Found: C, 73.18; H, 7.60; N, 2.50.

**EXAMPLE 4**

This example illustrates the preparation and properties of 17α,21-Dimethoxy-11β-[4-N-piperidino)phenyl]-19-norpregna-4,9-diene-3,20-dione (<u>113c</u>) (Figure 8):

***Step 1. 3,3-Ethylenedioxy-17α-methoxy-21-hydroxy-19-norpregna-5(10),9(11)-dien-20-one (107):***

To a solution of the 17α-methoxy-3-ketal (**94**, 10.0 g, 27.1 mmol) in dry THF (150 mL) was added iodobenzene diacetate (Moriarty, et al., J. Chem. Soc., Chem. Commun., 641-642 (1981); Velerio, et al., Steroids, 60:268-271 (1995)) (34.59 g, 4x) as a solid. The suspension was stirred under nitrogen and cooled to 0°C. $H_2O$ (7.73 mL, 429.6 mmol, 16x) was added, followed by 0.5 <u>M</u> KO-tBu solution (1400 mL, 700 mmol, 26x) via transfer needle. (A 50:50 (v/v) mixture of freshly opened methanol (700 mL) and 1.0 M potassium *t*-butoxide in THF (700 mL; Aldrich) was prepared and cooled to 0°C to give a 0.5 M base solution). Upon completion of addition the reaction mixture was removed from the ice bath

and the solution alowed to warm to room temperature. The reaction was monitored every hour by TLC (5% acetone in $CH_2Cl_2$) and after 4 hr, virtually all of the starting material had been converted to approximately a 80:20 mixture of two more polar components. The reaction mixture was diluted with $H_2O$ (500 mL) and brine (500 mL) and extracted into ether (3x). Organic fractions were washed again with $H_2O$ and brine. Combined organic extracts were dried by filtration through $Na_2SO_4$, evaporated *in vacuo,* and further dried under high vacuum to recover 13.84 g of an orange oil. Purification by flash chromatography (5% acetone in $CH_2Cl_2$) gave 6.0 g of a pale yellow-white foam (**107**) in 57.5% yield. Trituration with pentane produced **107** which was dried under vacuum to recover 5.36 g of a white powder in 51.0% yield; m.p. = 147 - 152°C. FTIR (KBr, diffuse reflectance): $\nu_{max}$ 3478, 2900, 2825, 1712, 1437, 1384 and 1372 $cm^{-1}$. NMR (300 MHz, $CDCl_3$): δ 0.550 (s, 3 H, C18-$CH_3$), 3.159 (s, 3 H, C17α-$OCH_3$), 3.981 (s, 4 H, C3-$O\overline{CH_2CH_2}O$), 4.251 and 4.471 (AB, 2 H, $J_{AB}$ =19.81 Hz, C21-$CH_2$) and 5.544 (br s, 1 H, C11-CH=). MS (EI) m/z (relative intensity): 388 ($M^+$, 54.8), 356 (13.8), 297 (100.0), 211 (65.0),169 (51.1) and 99 (56.3). Anal. Calcd. for $C_{23}H_{32}O_5 \cdot 1/4H_2O$: C, 70.29; H, 8.34. Found; C, 70.21; H, 8.12.

***Step 2. 3,3-Ethylenedioxy-17α,21-dimethoxy-19-norpregna-5(10),9(11)-dien-20-one (108):***

To a solution of the 3-ketal-21-hydroxy compound (**107**, 5.0 g, 12.87 mmol) in 500 mL of 1,2-dimethoxyethane (DME) was added Proton-Sponge® [1,8-bis(dimethylamino)naphthalene] (13.79 g, 64.35 mmol, 5x) as a solid. The solution was cooled to 0°C in an ice water bath and trimethyloxonium tetrafluoroborate (9.52 g, 64.35 mmol, 5x) was added as a solid. The suspension was kept at 0°C under nitrogen, for 3 hr. At that time, TLC (5% acetone in $CH_2Cl_2$) indicated all of the starting material had been cleanly converted to the slightly less polar 3-ketal-17α,21-dimethoxy compound (**108**). $H_2O$ and EtOAc were added, the mixture was transferred to a separatory funnel, and the layers allowed to separate. The organic fraction was washed with ice-cold 1 N HCl (2x), $H_2O$ (1x), saturated $NaHCO_3$ (1x), $H_2O$ (1x), and brine (1x). Combind EtOAc extracts (3x) were dried by filtration through $Na_2SO_4$ and evaporated *in vacuo*. The resulting colorless oil was dried overnight under high vacuum to recover a white foam (**108**, 5.28 g) in quantitative yield. Analysis by TLC and NMR indicated the crude material was sufficiently pure to carry directly on to the next reaction. A small amount was triturated with pentane and dried overnight under high vacuum to give 120 mg of **108** as a white solid; m.p = 104 - 110°C. FTIR (KBr, diffuse reflectance): $\nu_{max}$ 2926, 2884, 2828, 1722, 1447, 1380, 1322 and 1252 $cm^{-1}$. NMR (300 MHz, $CDCl_3$): δ 0.585 (s, 3 H, C18-$CH_3$), 3.175 (s, 3 H, C17α-$OCH_3$), 3.442 (s, 3 H, C21-$OCH_3$), 3.983 (s, 4 H, C3-$O\overline{CH_2CH_2}O$), 4.182 and 4.367 (AB, 2 H, $J_{AB}$ = 18.01 Hz, C21-$CH_2$) and 5.555 (br s, 1 H, C11-CH=). MS (EI) m/z (relative intensity): 402 ($M^+$, 27.7), 370 (7.2), 297 (100.0), 211 (62.1), 169 (41.6) and 99 (62.7). Anal. Calcd. for $C_{24}H_{34}O_5 . 3/5H_2O$: C, 69.74; H, 8.58. Found: C, 69.82; H, 8.43.

***Step 3. 3,3-Ethylenedioxy-17α,21-dimethoxy-19-norpregna-5(10),9(11)-dien-20-ol(109):***

The 3-ketal 17α,21-dimethoxy-20-one (**108**, 5.0 g, 12.42 mmol) was dissolved in dry THF (100 mL) and 2 equivalents of $LiAlH_4$ (25 mL), 25 mmol, 1.0 M in ether) were added *via* syringe. The solution was stirred magnetically at room temperature under nitrogen. After 15 minutes, examination by TLC (5% acetone in $CH_2Cl_2$) indicated the starting material had been cleanly converted to a single, more polar product (**109**). The reaction mixture was cooled in an ice bath, and saturated $Na_2SO_4$ (~2 - 3 mL) was added dropwise *via* pipette. When the reaction was quenched, several scoops of $Na_2SO_4$ were added and the mixture allowed to stir 1 hr. Filtration through a sintered glass funnel, followed by evaporation *in vacuo* produced a concentrated syrup. The syrup was taken up in $H_2O$ and $CH_2Cl_2$, transferred to a separatory funnel, and the layers allowed to separate. The organic fraction was washed again with brine. Combined $CH_2Cl_2$ extracts (3x) were dried by filtration through $Na_2SO_4$ and evaporated *in vacuo.* The resulting white foam was dried further under high vacuum to recover 4.69 g of the crude **109**. Purification of this crude product by flash chromatography (5% isopropanol in $CH_2Cl_2$) gave 4.24 g of **109** as a white foam in 84.4% yield.

The two purest fractions were combined and taken up in a minimum amount of acetone/hexane. After standing six days at room temperature, large, colorless crystals had formed. The crystals were collected by centrifugation, washed with several portions of hexane, and dried under high vacuum to recover 177 mg. Analysis by TLC (10% acetone in $CH_2Cl_2$) indicated the crystals were of the highest purity. Analysis of this material by NMR indicated a single isomer. No further work was done for identification of this single isomer. A second crop of 78 mg with only a trace of impurity was obtained from the mother liquors; m.p. =111-115°C. FTIR (KBr, diffuse reflectance): $\nu_{max}$ 3576, 3456, 2930, 2891, 2827, 1460 and 1372 $cm^{-1}$. NMR (300 MHz, $CDCl_3$): δ 0.824 (s, 3 H, C18-$CH_3$), 3.298 (s, 3 H, C17α-$OCH_3$), 3.392 (s, 3 H, C21-$OCH_3$), 3.416 (dd, 1 H, $J_1$ = 9.30 Hz, $J_2$ = 8.10 Hz, C21-$CH_2$), 3,490 (dd, 1 H, $J_1$= 9.30 Hz, $J_2$ = 3.30 Hz, C21-$CH_2$), 3.923 (dd, 1 H, $J_1$ = 8.10 Hz, $J_2$ = 3.30 Hz, C20-CH), 3.980 (s, 4 H, C3-$O\overline{CH_2CH_2}O$) and 5.595 (br s, 1 H, C11-CH=). MS (EI) m/z (relative intensity): 404 ($M^+$, 2.1), 372 (5.7), 329 (1.7), 297 (100.0) and 211 (35.7). Anal. Calcd. for $C_{24}H_{36}O_5 . 1/5C_6H_{14}$: C, 71.76; H, 9.27. Found: C, 71.83; H, 9.04.

***Step 4. 3,3-Ethylenedioxy-5α,10α-epoxy-17α,21-dimethoxy-19-norpregn-9(11)-en-20-ol (<u>110</u>):***

To a solution of hexafluoroacetone (2.01 mL, 14.39 mmol) in $CH_2Cl_2$ (50 mL), was added solid $Na_2HPO_4$ (1.36 g, 9.59 mmol) and 30% $H_2O_2$ (2.16 mL, 21.1 mmol). The mixture was transferred to the cold room and stirred vigorously for ½ hr at 4°C. A chilled solution of the 20-alcohol (**<u>109</u>**, 3.88 g. 9.59 mmol) in $CH_2Cl_2$ (25 mL) was added via pipette and rinsed in with additional CH $2Cl_2$ (25 mL). After stirring overnight at 4°C, TLC (7.5% acetone in $CH_2Cl_2$) indicated virtually all of the starting material had been converted to one major, more polar product with only a trace of by-products. The reaction mixture was transferred to a separatory funnel and washed with 10% $Na_2SO_3$ (1x), $H_2O$ (1x), and brine (1x). Combined $CH_2Cl_2$ extracts (3x) were dried by filtration through $Na_2SO_4$ and evaporated *in vacuo* to recover a foam. NMR analysis of the crude material indicated the a and β epoxides were present in approximately a 9:1 ratio. Trituration with ether produced 2.27 g of the pure 5α,10 α epoxide (**<u>110</u>**) as a white powder in 56.3% yield; m.p. = 146 - 1 53 °C. FTIR (KBr, diffuse reflectance): $v_m$ax 3558,2939, 1638,1446,1373 and 1247 $cm^{-1}$. NMR (300MHz, $CDCl_3$): δ 0.824 (s, 3H, C18-$CH_3$), 3.273 (s, 3 H, -C17α-$OCH_3$), 3.389 (s, 3H, C21-$OCH_3$), 3.402 (dd, 1H, $J_1$ = 9.61 Hz, $J_2$ = 8.10 Hz, C21-$CH_2$), 3,476 (dd, 1H, $J_1$ = 9.1 Hz, $J_2$ = 3.30 Hz, C21-$CH_2$), 3.908 (m, 5H, C3-O<u>$CH_2CH_2$</u>O and C20-CH) and 6.053 (br s, 1H, C11-CH=). MS (EI) m/z (relative intensity): 420 ($M^+$, 1.7), 402 (6.0), 370 (6.2), 345 (20.0), 313 (77.8), 295 (100.0) and 99 (95.4). Anal. Calcd. for $C_{24}H_{36}O_5 \cdot 1/10H_2O$: C, 68.25; H, 8.64. Found: C, 68.31; H, 8.71.

***Step 5. 3,3-Ethylenedioxy-5α-hydroxy-11β-[4-(N-piperidino)phenyl]-17α,21-dimethoxy-19-norpregn-9-en-20-ol (<u>111c</u>):***

A dry 50 mL 2-neck flask was equipped with a stirring bar, a reflux condenser and a rubber septum. Magnesium (137 mg, 5.64 mmol) was added and the entire apparatus was dried futher under a stream of nitrogen with a heat gun. After cooling slightly, one crystal of iodine was added. The apparatus was allowed to cool completely and dry THF (4 mL) was added followed by 1 drop of 1,2-dibromoethane. A solution of N-(4-bromophenyl)piperidine (*see,* EXAMPLE 2, Step 5) (1.23 g, 5.13 mmol) in THF (2.0 mL) was added *via* a transfer needle and rinsed in with additional THF (2.0 mL). The reaction mixture was brought to reflux for 1 hr. At that time, the Grignard reagent had formed as evidenced by consumption of almost all of the magnesium and bleaching of the iodine color. The cloudy, dark gray mixture was allowed to cool to room temperature and copper (I) chloride (55.4 mg, 0.56 mmol) was added as a solid. After stirring ½ hr, a solution of the 5α,10α-epoxide (**<u>110</u>**, 1.0 g, 2.38 mmol) in THF (4.0 mL; heated gently to achieve a solution) was added via transfer needle and rinsed in with additional THF (4.0 mL). After stirring 2 hr at room temperature, the reaction was quenched by the addition of saturated $NH_4Cl$ (16 mL). Air was drawn through the mixture for ½ hr with vigorous stirring. The mixture was transferred to a separatory funnel, $H_2O$ and ether were added, and the layers allowed to separate. The organic fraction was washed with $H_2O$ (1x), and brine (1x). Combined ether extracts (3x) were dried by filtration through anhydrous $Na_2SO_4$, evaporated *in vacuo*, and dried further under high vacuum to recover 1.73 g of an amber gum. Examination of the gum by TLC (15% acetone in $CH_2Cl_2$) revealed a single, slightly more polar product and trace of the epoxide. Trituration with ether failed to produce a solid. The crude product was purified by flash chromatography, (15% acetone in $CH_2Cl_2$). Fractions containing the pure product **<u>111c</u>** were combined and evaporated to give 0.36 g of a white foam. Fractions containing the product plus the epoxide were rechromatographed to give 0.43 g of additional pure product **<u>111c</u>**. The total yield of the purified product obtained was 0.79 g of **<u>111c</u>** as a white foam in 56.7% yield. A small amount was triturated with heptane and dried overnight in a drying pistol with acetone to give 73.8 mg of a white powder (**<u>111c</u>**) which was reserved for analysis; m.p. = 162 - 171°C. FTIR (KBr, diffuse reflectance): $v_{max}$. 3470, 2934, 2868, 2816, 1610,1511, 1440 and 1380 $cm^{-1}$. NMR (300 MHz, $CDCl_3$): δ 0.475 (s, 3 H, C18-$CH_3$), 3.091 (m, 4H, piperidyl α-CH2), 3.285 (s, 3 H, C17α-$OCH_3$), 3.361 (s, 3 H, C21-$OCH_3$), 3.34- 3.45 (m, 2 H, C21-$CH_2$), 3.794 (m, 1 H, C20-CH), 3.998 (m, 5 H, C3-O<u>$CH_2CH_2$</u>O and C20-OH), 4.178 (br s, 1 H, C11α-CH), 4.389 (s, 1 H, C5α-OH), 6.810 (d, 2 H, J = 8.85 Hz, 3', 5' aromatic-CH's) and 7.073 (d, 2 H, J = 8.85 Hz, 2', 6' aromatic-CH's). MS (EI) m/z (relative intensity): 581 ($M^+$, 39.0), 563 (24.4), 456 (5.9), 174 (24.9), 161 (100.0) and 99 (12.1). Anal. Calcd. for $C_{35}H_{51}NO_6$: C, 72.26; H, 8.84; N, 2.41. Found: C, 72.31; H, 8.78; N, 2.36.

***Step 6. 3,3-Ethylenedioxy-5α-hydroxy-11β-[4-(N-piperidino)phenyl]-17α,21-dimethoxy-19-norpregn-9-en-20-one (<u>112c</u>):***

To a suspension of IBX (0.49 g, 1.76 mmol) in DMSO (7.0 mL) was added a solution of the Grignard adduct (**<u>111c</u>**, 0.68 g, 1.17 mmol) in DMSO (6.0 mL). Additional DMSO (2 x 2.0 mL) was used to rinse in residual **<u>111c</u>**. Almost immediately upon addition of **<u>111c</u>**, a purple solution formed. The reaction was allowed to stir 2 hr at ambient temperature without any precautions against oxygen or moisture. At that time, the color had turned from purple to deep red. Examination of this solution by TLC (15% acetone in $CH_2Cl_2$; aliquot was diluted with $H_2O$ and extracted into EtOAc) revealed all of the starting material had been cleanly converted to a single, less polar product. The reaction mixture was transferred to a separatory funnel, $H_2O$ and $CH_2Cl_2$ were added, and the layers allowed to separate. The organic fraction was washed

again with $H_2O$ (1x) and brine (1x). Combined $CH_2Cl_2$ extracts (3x) were dried by filtration through anhydrous sodium sulfate and evaporated *in vacuo*. The resulting residue was dried overnight under high vacuum to recover 0.72 g of a purple gum. Purification by flash chromatography (15% acetone in $CH_2Cl_2$) gave 572 mg of a colorless gum. Trituration with heptane afforde 529 mg of **112c** as a white solid in 77.8% yield. A small amount was reserved and dried further in a drying pistol with acetone for analysis; m.p. = 107 -111 °C. FTIR (KBr, diffuse reflectance): $\nu_{max}$ 3534, 2931, 2823, 1721, 1609, 1511 and 1450 $cm^{-1}$. NMR (300 MHz, $CDCl_3$): δ 0.234 (s, 3 H, C18-$CH_3$), 3.089 (m, 4 H, piperidyl α-$CH_2$), 3.134 (s, 3 H, C17α-$OCH_3$), 3.429 (s, 3 H, C21-$OCH_3$), 3.995 (m, 4 H, C3-$OCH_2CH_2O$), 4.213 and 4.355 (AB, 2 H, $J_{AB}$ = 18.01 Hz, C21-$CH_2$), 4.212 - 4.306 (m, 2 H, C11α-CH and C5α-OH), 6.803 (d, 2 H, J = 8.70 Hz, 3'; 5' aromatic-CH's) and 7.021 (d, 2 H, J = 8.70 Hz, 2', 6' aromatic-CH's). MS (EI) m/z (relative intensity): 579 ($M^+$, 38.7), 561 (16.1), 174 (23.7), 161 (100.0) and 99 (12.1) Anal. Calcd. for $C_{35}H_{49}NO_6$: C, 72.51; H, 8.52; N, 2.42. Found: C, 72.47; H, 8.58; N, 2.35.

***Step 7. Preparation of the target compound 113c:***

To a solution of the 3-ketal-20-ketone (**112c,** 471 mg, 0.81 mmol) in THF (5.0 mL) was added glacial acetic acid (15 mL, 261 mmol) followed by $H_2O$ (5.0 mL). The mixture was brought to reflux under nitrogen. After 3 br, TLC (10% acetone in $CH_2Cl_2$; neutralized with $NH_4OH$ before developing) indicated the 3-ketal had been hydrolyzed to give the slightly less polar ketone. The reaction mixture was allowed to cool to room temperature and neutralized by the addition of concentrated $NH_4OH$ (17.6 mL, 261 mmol, pH 7 by a pH paper). The mixture was transferred to a separator funnel and extracted into $CH_2Cl_2$ (3x). The organic fractions were washed again with $H_2O$ (1x), and brine (1x). Combined $CH_2Cl_2$ extracts were dried by filtration through anhydrous $Na_2SO_4$ and evaporated *in vacuo* to recover 426 mg of a yellow glass. This crude product was purified by flash chromatography (5% acetone in $CH_2Cl_2$). Fractions containing highly pure product were combined and evaporated to give a pale yellow glass **113c**. Trituration of **113c** with heptane produced a pale yellow solid. The product was dried overnight in a drying pistol with benzene to give 189.6 mg of **113c** as a pale yellow solid in 45.7% yield; m.p. = 108 - 112°C. Analysis by HPLC on a Waters Assoc. NovaPak $C_{18}$ column eluted with 30% 50 m<u>M</u> $KH_2PO_4$, pH 3.0 in MeOH at a flow rate of 1 mL per min and at λ = 302 nm, indicated a purity of 97.22% with a retention time ($t_R$) of 3.73 min. FTIR (KBr, diffuse refleatance): $\nu_{max}$ 2935, 2822, 1723, 1664, 1609, 1511, 1488, 1451 and 1386 $cm^{-1}$. NMR (300 MHz, $CDCl_3$): δ 0.304 (s, 3 H, C18-$CH_3$), 3.100 (m, 4 H, piperidyl α-$CH_2$), 3.172 (s, 3 H, C17α-$OCH_3$), 3.450 (s, 3 H, C21-$OCH_3$), 4.227 and 4.370 (AB, 2 H, $J_{AB}$ = 18.01 Hz, C21-$CH_2$), 4.366 (br s, 1 H, C11α-CH), 5.753 (s, 1 H, C4-CH=), 6.821 (d, 2 H, J = 8.70 Hz, 3',5'aromatic-CH's) and 6.985 (d, 2 H, J = 8.70 Hz, 2', 6' aromatic-CH's). MS (EI) m/z (relative intensity): 517 ($M^+$, 57.8), 412, (4.6), 318 (6.6),174 (1.5.8), and 161 (100.0). Anal. Calcd. for $G_{33}H_{43}NO_4$: C, 76.56; H, 8.37; N, 2.71. Found: C, 76.45; H, 8.37; N, 2.70..

**EXAMPLE 5**

This example illustrates the preparation and properties of 17α-Acetoxy-11β-[4-(N-piperidino)phenyl]-21-methoxy-19-norpregna-4,9-diene-3,20-dione (**123a**):

***Step 1. 17α-Hydroxy-21-chloro-19-norpregna-4,9-diene-3,20-dione (115):***

The 3-ketal cyanohydrin (**98**, 50g, 73.22 mmol) was magnetically stirred with freshly distilled THF (550 mL) under nitrogen at room temperature. 4-Dimethylaminopyridine (DMAP) (4.47 g, 36.59 mmol) was added as a solid. Freshly distilled $Et_3N$ (27.60 mL, 197.68 mmol) followed by freshly distilled chloro-(chloromethyl)dimethylsilane (25.1 mL, 190.36 mmol) was added *via* syringe. The reaction was allowed to stir overnight at room temperature. The next day TLC on silica (2% acetone in $CH_2Cl_2$) showed all starting material had been converted to the silyl ether. The reaction mixture was cooled to -78°C in a dry ice bath with isopropanol, and then diluted with THF (800 mL). Lithium diisopropylamide (LDA) (2.0 <u>M,</u> 300 mL, 600 mmol) was added dropwise to the reaction *via* an additional funnel over a period of 45 min. Once addition was complete, the reaction was stirred for 1.5 hr at -78°C. HCl (4 <u>N</u>, 1250 mL, 5 mol) was added *via* the addition funnel. The dry ice bath was removed, and the reaction was allowed to stir overnight at room temperature. The reaction mixture was then cooled to 0°C and neutralized by the addition of concentrated $NH_4OH$ (305 mL). The mixture was transferred to a separatory funnel and extracted with EtOAc (3x), washed with $H_2O$ (2x) and brine (1x). The organic fractions were combined, filtered through $Na_2SO_4$ and evaporated *in vacuo.* The resulting solid was triturated with ether (1000 mL), collected on a Buchner funnel, and washed with additional ether. After drying overnight *in vacuo,* 38.94 g of **115** as a dark yellow solid was recovered in 76.61% yield. Analysis by TLC on silica (5% acetone in $CH_2Cl_2$) showed the material was suitable to carry directly on to the next reaction; m.p. = 204 - 207°C. FTIR (KBr, diffuse reflectance): $\nu_{max}$ 3465, 2946, 1729, 1664, 1599 and 1573 $cm^{-1}$. NMR (300 MHz, $CDCl_3$): δ 0.833 (s, 3 H, C18-$CH_3$), 4.352 and 4.655 (AB, 2 H, $J_{AB}$ = 16.8 Hz, C21-$CH_2$) and 5.687 (s, 1 H, C4-CH=) MS (EI) m/z (relative intensity): 350 ($M^+$, 33.1), 348 (100.0), 253 (63.7), 213 (71.5) and 91 (62.6).

***Step 2. 17αHydroxy-21-acetoxy-19-norpregna-4,9-diene-3,20-dione (116):***

The 21-chloro compound (**115**, 37.90 g, 108.64 mmol), KOAc (111.83 g, 1139.63 mmol) and acetonitrile (927 mL) were mechanically stirred. The suspension was brought to reflux under nitrogen. After 2.5 hr, TLC on silica (5% acetone in methylene chloride) indicated the reaction had gone to completion. The reaction mixture was allowed to cool to room temperature, and precipitated KCl was removed by filtration through a sintered glass funnel. Acetonitrile was evaporated *in vacuo,* and the resulting residue was taken up in $CH_2Cl_2$ and $H_2O$. The mixture was transferred to a separatory funnel, extracted with $CH_2Cl_2$ (3x), and washed with $H_2O$ (2x) and brine (1x). The organic fractions were combined, filtered through $Na_2SO_4$ and evaporated *in vacuo* to give 36.26 g of 116 in 89.61% crude yield. The solid material was taken up in hot acetone (150 mL) and $CH_2Cl_2$ (150 mL). The solution was scratched, seeded and stored in the freezer for 4 br. The crystals were then filtered through a Buchner funnel and dried *in vacuo* to recover 10.71 g of the 17α-ol-21-acetate (**116**) in 52.14% yield. The mother liquor was evaporated *in vacuo* and purified by flash column chromatography eluted with 10% acetone in $CH_2Cl_2$. Fractions containing the 17α-ol-21-acetate (**116**) were combined and evaporated *in vacuo* to recover 2.58 g of a golden yellow solid in 12.61%. The total yield of the purified 17α-ol-21-acetate (**116**) was 13.29 g of a golden yellow solid in 64.7% yield; m.p. = 213 - 218°C. FTIR (KBr, diffuse reflectance): $v_{max}$ 3475, 2947, 2951, 1744, 1720, 1646, 1606, 1578, 1367 and 1235 $cm^{-1}$. NMR (300 MHz, $CDCl_3$): δ 0.841 (s, 3 H, C18-$CH_3$), 2.182 (s, 3 H, C21-$OA_C$), 4.868 and 5.081 (AB, 2 H, $J_{AB}$ = 17.4 Hz, C21-$CH_2$) and 5.683 (s, 1 H, C4-CH=) MS (EI) m/z (relative intensity): 372 ($M^+$, 78.3), 354 (9.7), 312 (75.6), 253 (100.0) and 91 (69.3).

***Step 3. 17α,21-Dihydroxy-19-norpregna-4,9-diene-3,20-dione (117):***

The 17α-ol-21-acetate (**116**) (35.15 g, 94.37 mmol) was suspended in freshly opened MeOH (2870 mL) and deoxygenated by bubbling nitrogen through the mixture for 45 min. $KHCO_3$ (deoxygenated, 0.5 M, 283 mL, 141.74 mmol) was added, and the suspension was mechanically stirred and brought to reflux under nitrogen. After 10 minutes at reflux, TLC on silica (5% isopropanol in $CH_2Cl_2$) showed the reaction to be complete. The reaction mixture was cooled to room temperature, neutralized by the addition of HOAc (8.15 mL), and MeOH was evaporated *in vacuo*. The reaction mixture was extracted with $CH_2Cl_2$ (3x), and washed with $H_2O$ (2x) and brine (1x). The combined organic fractions were filtered through $Na_2SO_4$ and evaporated *in vacuo* to recover 29.83 g of the solid in 95.7% yield. The solid was taken up in acetone with a small amount of $CH_2Cl_2$. The solution was scratched, seeded and stored in the freezer for 1 hr. The resulting crystals were collected on a Buchner funnel, rinsed with acetone and dried *in vacuo* to recover the first crop. The mother liquor was concentrated and stored in the freezer overnight to afford a second crop of crystals. The combined solid recovered was 16.15 g in 51.8% crude yield. The mother liquors were evaporated *in vacuo* and purified by flash column chromatography eluted with 5% isopropanol in $CH_2Cl_2$. Fractions containing the diol (**117**) were combined and evaporated *in vacuo* to recover 4.86 g. The total yield of **117** was 19.75 g of a light yellow solid in 76.7%; m.p. = 197 - 204°C. FTIR (KBr, diffuse reflectance): $v_{max}$ 3917,2954,2869,1715,1635, and 1590 $cm^{-1}$. NMR (300 MHZ, $CDCl_3$): δ 0.827 (s, 3 H, C18-$CH_3$), 4.323 and 4.690 (AB, 2 H, $J_{AB}$ = 19.81 Hz, C21-$CH_2$) and 5.686 (s, 1 H, C4-CH=). MS (EI) m/z (relative intensity): 330 ($M^+$, 100.0), 312 (10.1), 253 (61.7), 213 (64.5), 174 (26.1) and 91. (38.5).

***Step 4 3,20-bis-Ethylenedioxy-17α,21-Dihydroxy-19-norpregna-5(10),9(11)-diene (118):***

The diol (**117**, 9.88 g, 29.89 mmol) and freshly opened ethylene glycol (750 mL) were magnetically stirred. *p*-Toluenesulfonic acid monohydrate (0.49 g, 2.60 mmol) was added to the suspension as a solid. The ethylene glycol was distilled *in vacuo* at 81°C under 2 mm Hg. After distilling for 3 hr, the mixture was cooled to room temperature and poured into saturated $NaHCO_3$ (250 mL) and $H_2O$ (250 mL). The mixture was extracted with $CH_2Cl_2$ (3x), washed with $H_2O$ (2x) and brine (1x). The organic fractions were combined, filtered through sodium sulfate and evaporated *in vacuo* to recover a solid. Analysis by TLC on silica (5% isopropanol in $CH_2Cl_2$) showed all of the starting material to be converted to an 85:15 mixture of 3,20-diketal to 3-ketal with a small amount of byproduct. The resulting solid was triturated with ether, collected on a Buchner funnel, washed with additional ether and dried *in vacuo* to recover 6.46 g of **118** in 51.64% yield. The mother liquor was evaporated *in vacuo* and purified through flash chromatography eluting with 4% isopropanol in $CH_2Cl_2$. This recovered 0.6 g of the light beige, solid diketal in 4.8% yield. The total yield of the solid diketal (**118**) was 7.06 g of a light beige solid in 56.44% yield; m.p. = 173 - 176°C. FTTR (KBr, diffuse reflectance): $v_{max}$ 3452, 2892, 1652, 1436, 1370, 1223 and 1055 $cm^{-1}$. NMR (300 MHz, $CDCl_3$): δ 0.795 (s, 3 H, C18-$CH_3$), 3.686 and 3.894 (AB, 2 H, $J_{AB}$ = 12.61 Hz, C21-$CH_2$), 3.987 (s, 4 H, C3-$OCH_2CH_2O$-), 4.130 (m, 4 H, $C_{20}$-$OCH_2CH_2O$-) and 5.555 (br s, 1 H, C11-CH=). MS(EI) m/z (relative intensity): 418 ($M^+$, 5.6), 400 (0.7), 387 (3.9), 314 (3.5), 211 (4.6) and 103 (100.0).

***Step 5. 3,20-*bis*-Ethylenedioxy-17α-hydroxy-21-methoxy-19-norpregna-S(10),9(11)-diene (119):***

To a solution of the diketal (**118**, 0.5 g, 1.19 mmol) in $CH_2Cl_2$ (50 mL) was added 1,8-*bis*-(dimethylamino)naphthalene ("Proton Sponge", 1.28 g, 5.97 mmol) followed by trimethyloxonium tetrafluoroborate (0.88 g, 5.97 mmol), The mixture was mechanically stirred in an ice bath under nitrogen. The ice bath was allowed to melt to bring the reaction to room temperature. The reaction mixture was stirred for 3 hr, at which time TLC (5% isopropanol in $CH_2Cl_2$) indicated the reaction had gone to completion. The mixture was poured into a separatory funnel and washed with $H_2O$ (2x). The $CH_2Cl_2$ extracts (3x) were combined, filtered through $Na_2SO_4$ and evaporated in *vacuo.* The resulting residue was taken up in EtOAc, washed with ice-cold 1 N HCl (2x), $H_2O$ (1x), saturated $NaHCO_3$ (1x), $H_2O$ (1x), and brine (1x). Combined EtOAc fractions (3x) were filtered through $Na_2SO_4$ and evaporated *in vacuo* to give 0.5 g of **119** as a yellow foam in 97.14% yield. The material was of adequate purity to carry onto the subsequent epoxidation. The reaction was repeated to produce a total of 13.57 g of the 21-methoxy compound (**119**). NMR (300 MHz, $CDCl_3$): δ 0.798 (s, 3 H, C18-$CH_3$), 3.415 (s, 3 H, C21-$OCH_3$), 3.546 and 3.715 (AB, 2 H, $J_{AB}$ = 10.51 Hz, C21-$CH_2$), 3.985 (s, 4 H, C3-$OCH_2CH_2O$-), 4.05 (m, 4 H, C20-$OCH_2CH_2O$-) and 5.54 (br s, 1 H, C11-CH=). Decomposition of analytical sample precluded further analysis.

*Step 6. 3,20-***bis***-Ethyleneedioxy-5α,10α-epoxy-17α-hydroxy-21-methoxy-19-norpregn-9(11)-ene (120):*

Hexafluoroacetone trihydrate (6.49 mL, 46.64 mmol) and $CH_2Cl_2$ (100 mL) were mechanically stirred vigorously at 4°C. Solid $Na_2HPO_4$ (3.67 g, 25.91 mmol) and 30% $H_2O_2$ (7.01 mL, 68.39 mmol) were added and stirred for 15 minutes at 4°C. A cold solution of the 21-methoxy compound (**119**, 13.45 g, 31.09 mmol) in $CH_2Cl_2$ (100 mL) was added to the mixture *via* an additional funnel over a period of 1 br. The reaction mixture was allowed to stir overnight at 4°C. Examination by TLC (25% EtOAc in $CH_2Cl_2$) showed all of the starting material had been converted to a mixture of the a and β epoxides in about a 2:1 ratio. The mixture was transferred to a separatory funnel and washed with 10% $Na_2SO_3$ (1x), saturated $NaHCO_3$ (1x) and $H_2O$ (1x). Combined $CH_2Cl_2$ extracts (3x) were filtered through $Na_2SO_4$ and evaporated *in vacuo* to recover 14.06 g of the epoxide (**120**) as a white foam in quantitative yield. The 2:1 mixture of α- and β-epoxides was used directly for the subsequent Grignard reaction. NMR (300 MHz, $CDCl_3$): δ 0.700 (s, 3 H, C18-$CH_3$), 3.407 (s, 3 H, C21-$OCH_3$), 3.539 and 3.692 (AB, 2 H, $J_{AB}$ = 10.51 Hz, C21-$CH_2$), 4.051 (m, 8 H, C3- and C20-$OCH_2CH_2O$-), 5.819 (br s, 0.3 H, C11-CR= of β-epoxide), and 5.997 (br s, 0.6 H, C11-CH= of α-epoxide). Decomposition of analytical sample precluded further analysis.

*Step 7. 3,20-***bis***-Ethylenedioxy-5α,17α-dihydroxy-11β-[4-(N-piperidino)phenyl]-21-methoxy-19-norpregn-9-ene (121a):*

Magnesium (1.27 g, 52.25 mmol), a crystal of iodine, dry THF (55 mL), and one drop or 1,2-dibromoethane were stirred together in dry glassware over nitrogen. A solution of N-(4-bromophenyl)piperidine (*see,* EXAMPLE 2, Step 5) (13.80 g, 57.48 mmol) in dry THF (45 mL) was added to the reaction flask, then rinsed in with an additional 10 mL of THF. The mixture was heated until all of the Magnesium metal was gone. The reaction was allowed to reflux for 1.5 hr, and then cooled to room temperature. Copper (I) chloride (0.57 g, 5.75 mmol) was added, and stirring continued for 1 hr. A solution of the epoxide (**120**, 4.69 g, 10.45 mmol) in dry THF was added to the reaction and rinsed in with an additional 10 mL of THF. The reaction was stirred under nitrogen, at room temperature, for 1 hr. The reaction was quenched with saturated $NH_4Cl$ (138 mL). Air was drawn through the mixture with vigorous stirring for 20 min, The mixture was transferred to a separatory funnel, extracted with ether (3x), washed with $H_2O$ (2x) and brine (1x). The combined organic fractions were dried with $Na_2SO_4$ for ½ hr, and evaporated *in vacuo* to recover 12.97 g of the crude product. Analysis by TLC (20% acetone in $CH_2Cl_2$) showed many impurities. The crude material was triturated with pentane to recover 4.45 g of a pale green solid. Analysis by TLC (20% acetone in $CH_2Cl_2$) showed a small amount of by-product still present. The precipitate was further purified by flash column chromatography (10% acetone in $CH_2Cl_2$). Fractions containing the pure Grignard adduct (**121a**) were combined and evaporated *in vacuo* to recover 2.56 g of an aqua-green solid in 40.17% yield. The mother liquors from the trituration were combined and evaporated *in vacuo* to recover 8.15 g of material. Purification of this material by flash column chromatography (20% acetone in $CH_2Cl_2$) afforded 0.29 g of a green gum. All recovered products were combined and triturated with ether to recover a total of 2.16 g of Grignard adduct (**121a**) in 33.9% yield; m.p. = 218 220°C. FTIR (KBr, diffuse reflectance): $v_{max}$ 3508, 2940,1609 and 1509 $cm^{-1}$. NMR ($CDCl_3$): 8 0.449 (s, 3 H, C18-$CH_3$), 3.094 (t ,10 H, -$NC_5H_{10}$), 3.437 (s, 3 H, C21-$OCH_3$), 3.989 (m, 10 H, C3 and C20-$OCH_2CH_2O$- and C21-$CH_2$-), 6.822 (d, 2 H, J = 8.85, 3', 5' aromatic-CH's) and 7.067 (d, 2 H, J = 8.85 Hz, 2', 6' aromatic-CH's). MS (EI) m/z (relative intensity): 609 ($M^+$, 29.1), 591 (46.6), 364 (8.6), 174 (29.2),161 (100.0) and 117 (96.4). AnaL Calcd. for $C_{36}H_{51}N_7 \cdot 1/3H_2O$: C, 70.22; H, 8.46; N, 2.27. Found: C, 70.10; H, 8.33; N, 2.40.

*Step 8.17a-Hydroxy-11β-[4-(N-piperidino)phenyl]-21-methoxy-19-norpregna-4,9-diene-3,20-dione (122a):*

A solution of the Grignard adduct (**121a**, 2.10 g, 3.44 mmol) in THF (20 mL) was mechanically stirred under nitrogen at room temperature. Trifluoroacetic acid (60 mL, 764.26 mmol) and $H_2O$ (20 mL) were added, and the mixture was stirred under nitrogen for 3 hr. Examination by TLC (20% acetone in $CH_2Cl_2$) showed the reaction had gone to

completion. The reaction mixture was cooled in an ice bath, and $NH_4OH$ (51.46 mL) was slowly added to neutralize the reaction to a pH of 7 by pH paper. The mixture was transferred to a separatory fumiel, extracted with EtOAc (3x). The organic fractions were washed with $H_2O$ (2x) and brine (1x). The combined EtOAc fractions were dried with $Na_2SO_4$ and evaporated *in vacuo* to give 1.70 g of an amber foam. The crude product was purified by flash column chromatography (20% acetone in $CH_2Cl_2$) to recover 1.16 g of **122a** as a bright yellow foam in 66.95% yield; m.p. = 2111- 216°C. FTIR (KBr, diffuse reflectance): $\nu_{max}$ 3429, 2941, 1721,1648,1601 and 1511 $cm^{-1}$. NMR ($CDCl_3$): 8 0.391 (s, 3 H, C18-$CH_3$), 2.979 (t ,10H, -$NC_5H_{10}$), 3. 454 (s, 3 H, C21-$OCH_3$), 4.243 and 4.383 (AB, 2H, $J_{AB}$ = 17.71 Hz, C21-$CH_2$-), 5.762 (s, 1H, C4-CH=), 6.820 (d, 2 H, J = 8.55 Hz, 3', 5' aromatic-CH's) and 6.980 (d, 2 H, J = 8.55 Hz, 2', 6' aromatic-CH's). MS(EI) m/z (relative intensity): 503 ($M^+$, 57.9), 318 (5.8),174 (12.3) and 161 (100.0). Anal. Calcd. for $C_{32}H_{41}NO_4 \cdot 1/3H_2O$: C, 75.42; H, 8.24; N, 2.75. Found: C, 75.23; H, 8.04; N, 2.94.

***Step 9 Preparation of the target compound 123a:***

A mixture of $CH_2Cl_2$ (50 mL), trifluoroacetic anhydride (11.70 g, 55.65 mmol) and glacial acetic acid (3.35 g, 55.59 mmol) was stirred under nitrogen at room temperature for ½ hr. The mixture was cooled in an ice bath, and *p*-toluenesulfonic acid monohydrate (0.47 g, 2.45 mmol) was added. The 17α-OH (**122a**, 1.12 g, 2.22 mmol) dissolved in $CH_2Cl_2$ (7.5 mL) was transferred to the reaction flask' and then rinsed in with an additional 8 mL of $CH_2Cl_2$. The reaction mixture was stirred at 0°C for 2 hr. Examination by TLC (10% acetone in $CH_2Cl_2$) showed the reaction had gone to completion. The reaction was kept at 0°C and diluted with $H_2O$ (30 mL), then neutralized by the addition of $NH_4OH$ (11.45 mL). Additional $NH_4OH$ was added until the pH of 6 - 7 by pH paper was reached. The mixture was transferred to a separatory funnel, the layers allowed to separate and $CH_2Cl_2$ fractions then washed with $H_2O$ (1x) and brine (1x). The organic fractions were filtered through $Na_2SO_4$ and evaporated in vacuo to give 1.21 g of a dark yellow foam. The crude product was purified by flash column chromatography (10% acetone in $CH_2Cl_2$) to give 1.08 g of **123a** as a bright yellow foam. The purified product was then triutrated with pentane to give 0.92 g of **123a** as a pale yellow powder in 76% yield; m.p. =1.42 -144°C. FTIR (KBr, diffuse reflectance): $\nu_{max}$ 2941, 2360, 2338, 1.737, 1664,1608 and 1512 $cm^{-1}$. NMR ($CDCl_3$): δ 0.378 (s, 3 H, C18-$CH_3$), 2.105 (s, 3 H, C17α-OAc), 3.095 (t,10 H, -$NC_5H_{10}$), 3. 413 (s, 3 H, C21.-$OCH_3$), 4.099 and 4.307 (AB, 2 H, $J_{AB}$ = 17.11 Hz, C21-CH $_2$-),4.377 (d, 1H, J = 6.60 Hz, C11α-CH), 5.779 (s, 1H, C4-CH=), 6.810 (d, 2 H, J = 8.70 Hz, 3', 5' aromatic-CH's) and 6.973 (d, 2 H, J' = 8.70 Hz, 2', 6' aromatic-CH's). MS (EI) m/z (relative intensity); 545 ($M^+$, 34.5), 485 (8.6), 412 (2.2), 174 (10.1), 161 (100.0) and 105 (2.5). Anal. Calcd for $C_{34}H_{43}NO_5 \cdot /10H_2O$: C, 74.59; H, 7.95; N, 2.56. Found: C, 74.58; H, 7.89; N, 2.65.

**EXAMPLE 6**

This example illustrates the preparation and properties of 17α-Acetoxy-11β-[(4-N-piperidino)phenyl]-19-norpregna-4,9-diene-3,20-dione 3-oxime (**141**) (**Figure 4**):

Under nitrogen, a solution of the dienedione (**71**, 200 mg, 0.38 mmol) in absolute EtOH (25 mL) was treated with a 10-fold excess of solid hydroxylamine hydrochloride (269 mg, 3.87 mmol). The reaction mixture was stirred at room temperature, for 11/4 hr. At that time, TLC (10% acetone in $CH_2Cl_2$) showed no starting material and two major more polar spots. The reaction was diluted with saturated sodium bicarbonate solution (100 mL) and extracted with methylene chloride (3x). The orange fractions were washed with water and brine, dried over anhydrous sodium sulfate, filtered and concentrated *in vacuo* to yield 290 mg of off-white powder. Flash chromatography (10% acetone in methylene chloride) gave 177 mg of the material. Trituration with pentane with sonication gave 163 mg of **141** as an off-white solid in 80.8% yield after drying. HPLC analysis indicated a syn:anti ratio of 1:3.2; m.p. = 167 - 172°C. FTIR (KBr, diffuse reflectance): $\nu_{max}$ 3237, 2932, 2855,1735,1714,1610, 1512,1452,1369 and 1236 $cm^{-1}$. NMR (300 MHZ, $CDCl_3$): δ 0306 (s, 3 H, C18-$CH_3$), 2.086 (s, 3 H, C17α-OAc), 2.125 (s, 3H, C21-$CH_3$), 3.10 (m, 4 H, -$CH_2CH_2$-N- of piperidine ring) 4.33 (m, 1H, C11α-CH), 5.869 (s, 1H, C4-CH= of *anti*-oxime), 6.525 (s, 1H, C4-CH= of syn-oxime) and 6.805 - 6.975 (dd, 4H, aromatic-CH's), MS (EI) m/z (relative intensity): 530 ($M^+$). Anal. Calcd. for $C_{33}H_{42}O_4N_2$: C, 74.72; H, 7.92; N, 5.28. Found: C, 73.73; H, 8.16; N, 5.16.

**EXAMPLE 7**

This example illustrates the preparation and properties of 17α-Methoxy-11β-[4-(N-piperidino)phenyl]-19-norpregna-4,9-diene-3,20-dione 3-oxide (**142b**) (**Figure 6**):

Under nitrogen, a solution of the dienedione (**97b**, 250 mg, 0.513 mmol) in absolute EtOH (25 mL) was treated with a 10-fold excess of solid hydroxylamine hydrochloride (38 mg, 5.13 mmol). The reaction mixture was stirred at room temperature for 1.1/4 hr. At that time, TLC (10% acetone in methylene chloride) showed no starting material and two major more polar products. The reaction was diluted with saturated sodium bicarbonate solution (100 mL) and extracted with methylene chloride (3x). The organic fractions were washed with water and brine, dried over anhydrous sodium

sulfate, filtered and concentrated *in vacuo* to yield 260 mg of yellow foam.. Flash chromatography (10% acetone in methylene chloride) gave 186 mg of the material. Trituration with pentane with scratching and sonication gave 172 mg of the product **142b** after drying. HPLC analysis indicated this material to be 94% pure. Two additional flash column chromatography, trituration with pentane and drying again in vacuo yielded 143 g of **142b** as an off- white solid in 55.5% yield; m.p. =157-162°C (amber gel) and 195 - 200°C (gel melts). HPLC analysis on a Waters NovaPak $C_{18}$ ODS column eluted with MeOH: water (80:20) with 0.05% $Bt_3N$ at a flow rate of 1 mL per min and at $\lambda$= 260 nm indicated a purity of 97.9%. FTIR (KBr, diffuse reflectance): $v_{max}$ 3183, 2934, 1707, 1610, 1511, 1450, 1385, 1349 and 1234 $cm^{-1}$. NMR (300 MHZ, $CDCl_3$): $\delta$ 0.239 (s, 3 H, C18-$CH_3$), 2.175 (s, 3H, C21-$CH_3$), 3.07-3.150 (m, 4 H, -N-$CH_2CH_2$- of piperidine ring), 3.13 (s, 3 H, C17$\alpha$-$OCH_3$), 4.28 - 4.30 (d., 1 H, C11$\alpha$-CH), 5.840 (s, 0.69 H, C4-CH=of *anti*-oxime), 6.493 (s, 0.31H, C4-CH=of *syn*-oxime), 6.8 - 7 .0 (dd, 4H, aromatic-CH's). MS (EI) m/z (relative intensity): 502 ($M^+$). Anal. Calcd. for $C_{32}H_{42}O_3N_2$: 76.46; H, 8.42; N, 5.57. Found: C, 75.38; H, 8.60; N, 5.39

**Biological Properties of the Compounds of Formula I**

**MATERIALS AND METHODS**

***Statistical Analysis***

Statistical analysis was performed using standard methods and a PROPHET data management system operating on SUN Microsystems OS 4.4.1 (Bliss, Cl., The Statistics of Bioassay, New York, Academic Press (1952); Hollister, C., Nucleic Acids Research, 16:1873-1875 (1988)). Raw data, statistical andregression analysis are available.

***AntiMcGinty Test (McGinty, et al., Endocrinology, 24:829-832 (1939))***

Immature female rabbits of the New Zealand White breed (approx. 1 kg body weight) were maintained under standard laboratory conditions and received a subcutaneous injection of 5 $\mu$g estradiol in 10% ethanol/sesame oil daily for 6 consecutive days. Twenty-four hours after the last injection of estradiol (day 7) animals underwent sterile abdominal surgery to ligate a 3-4 cm segment of both uterine homs. The experimental compound in appropriate solvent (usually 10% ethanol/sesame oil) was inj ected intraluminally into the ligated segment of one uterine horn and the vehicle alone into the ligated segment of the contralateral horn. Injection volume was limited to 0.1 ml, and care was taken to prevent leakage. A stimulating dose of progesterone (0.8 mg/day) was administered subcutaneously to each rabbit daily for the next three days (days 7, 8 and 9) for the purpose of inducing endometrial proliferation. All animals were sacrificed on day 10 when a segment central to the ligatures was removed and fixed in 10% neutral buffered formalin and submitted for histological processing. Five micron sections stained with hematoxylin and eosin (H&E) were evaluated microscopically for the degree of endometrial glandular proliferation according to the method of McPhail. (McPhail, J. Physiol., 83:145 (1934). The percent inhibition of endometrial proliferation for each rabbit was calculated and the mean of the group of five animals recorded.

***AntiClauberg Test (Clauberg, C., Zentr. Gynakol., 54:2757-2770 (1930))***

Immature female rabbits of the New Zealand White breed (approx. 1 kg body weight) were maintained under standard laboratory conditions and received a subcutaneous injection of 5 $\mu$g estradiol in 10% ethanol/sesame oil daily for 6 consecutive days. Twenty-four hours after the last dose of estradiol (day 7) animals received progesterone by subcutaneous injection (0.8 mg/day) and the experimental compound in appropriate vehicle (usually 10% ethanol/sesame oil) orally or subcutaneously for five consecutive days. One group of rabbits received progesterone only. Twenty-four hours after the last dose all animals were sacrificed for removal of the uterus which was cleaned of all fat and connective tissue, weighed to the nearest 0.2 mg and placed in 10% neutral buffered formalin for subsequent histological processing. Five micron sections stained with hematoxylin and eosin (H&E) were evaluated microscopically for the degree of endometrial glandular proliferation according to the method of McPhail (McPhail, *supra*). The percent inhibition of endometrial proliferation at each dose level of the experimental compound was derived by comparison with the progesterone-stimulated animals alone.

***Postcoital Test***

Adult female rats of the Sprague-Dawley strain were maintained under standard laboratory conditions, 14 hours of light and 10 hours of darkness each day and cohabited with proven fertile males when in proestrus. Sperm-positive animals were randomly assigned to control and experimental groups. The day vaginal sperm were found in vaginal washings constituted day 0 of gestation. Rats received experimental compounds or vehicle (control) daily by the oral

route on days 0-3 or 4-6. and were sacrificed between days 10 and 17 to record the number and condition of conceptuses.

### *Antiovulatory Test*

Immature female rats of the Sprague-Dawley strain weighing 200 to 250 g were maintained under standard laboratory conditions, 14 hours of light and 10 hours of darkness each day. Vaginal washings were obtained daily and evaluated microscopically to establish the estrous cycle of each animal. Animals exhibiting two consecutive four-day cycles were used in the test. Each dose group consisted of eight rats and one group served as the vehicle controL Animals were dosed at noon on the day of proestrus and sacrificed 24 hours later when ova can usually be visualized in the distended ampulla of the oviduct using a dissecting microscope. The oviducts were excised, an incision made in the distended ampulla and the ova teased out in a drop of water on a microscope slide so that the number shed could be counted. Historically, control animals shed between 12 and 14 ova during each estrous cycle. Agents which inhibit ovulation usually exhibit an "all or none" effect; it is rare that ovulation is "partially" inhibited. Treatment groups were compared with the control group using a 95% contingency table or the $ED_{100}$ was established with additional dose levels.

### *Relative Binding Affinities for the Progesterone and Glucocorticoid Receptors*

Uteri and thymus glands were obtained from estradiol-primed immature female rabbits of the New Zealand White strain for preparation of cytosols for the progesterone and glucocorticoid receptor assays, respectively. Tissues were excised and immediately placed in ice cold TEGDM buffer (10 mM Tris, pH 7.4; 1.5 mM EDTA; 10% glycerol vol/vol/; 1 mM dithiothreitol [DTT]; and 20 mM sodium molybdate). The tissues were dissected free of connective tissue and fat, weighed and minced finely. Minced tissues were homogenized in 3 volumes TEGDM/gm with four 10 second bursts of a VirTis Cyclone set at half maximum speed with a 30 second cooling period (in ice) between bursts. Homogenates were centrifuged at 109,663 g at 4°C for 1 hour to yield the soluble cytosol fraction. Aliquots of cytosol were snap frozen and stored at -75°C.

All binding assays were carried out at 2-6°C for 16-18 hours. The following radioactive ligands were used: [1,2-$^{3}$(H)]-progesterone (50.0 Ci/mmole) for the progesterone receptor (PR). [6,7-$^{3}$H(N)-dexamethasone (39.2 Ci/mmole) for the glucocorticoid receptor (GR) and [2,4,6,7-$^{3}$H(N)]-estradiol for the estrogen receptor. For the progesterone receptor RBA assays 0.02 ml uterine cytosol or TEDGM buffer, 0.05 ml of various concentrations of test compounds or progesterone, 0.13 ml TEGDM buffer and 0.05 ml [$^{3}$H] progesterone were added to duplicate tubes. For the glucocorticoid receptor RBA assays 0.1 ml thymus cytosol or TEDGM buffer, 0.05 ml of various concentrations of test compounds or dexamethasone, 0.05 ml TEGDM buffer and 0.05 ml [$^{3}$H]-dexamethasone were added to duplicate tubes. For the estrogen receptor RBA assays 0.05 ml uterine cytosol, 0.1 ml TEGDM buffer, 0.05 ml of various concentrations of test compounds or estradiol and 0.05 ml [$^{3}$H]-estradiol were added to duplicate tubes. The concentrations of the test compounds, progesterone, dexamethasone and estradiol ranged from 0.05 to 100 nM and the concentrations of the competitors ranged from 0.5 to 500 nM. Total binding was measured at radioligand concentrations of 3.5 nM and nonspecific binding was measured in the presence of a 200-fold excess of unlabeled progesterone (PR), dexamethasone (GR) or diethylstilbestrol (ER), respectively.

In all incubations bound and free ligand were separated using dextra-coated charcoal (DCC). A 0.1 ml aliquot of DCC (0.5% charcoal/0.05% Dextran T-70) was added to each tube. The tubes were vortexed and incubated on ice for 10 minutes. Five-tenths ml TEG buffer (without DTT or molybdate) was then added to all tubes to improve supernatant recovery following centrifugation. The charcoal was pelleted by centrifugation at 2,100 g for 15 minutes at 4°C. The supernatants containing the [$^{3}$H]-steroid receptor complexes were decanted into vials containing 4 ml Optifluor (Packard Instrument Co.), vortexed, equilibrated in a liquid scintillation counter for 30 minutes and then counted for 2 minutes. This provided the quantity of receptor bound [$^{3}$H]-steroid at each competitor concentration.

The standard curves and the $EC_{50}$ (Effective Concentration) for each standard curve and curve for each test compound was determined by entering the counting data (receptor bound [$^{3}$H]-progesterone, [$^{3}$H]-dexamethasone or [$^{3}$H]-estradiol) into a four parameter sigmoidal computer program (RiaSmart® Immunoassay Data Reduction Program, Packard Instrument Co., Meriden, Connecticut. The RBA for each test compound was calculated using the following equation:

$$RBA = \frac{EC_{50}\ \text{Standard}}{EC_{50}\ \text{Test Compound}} X\ 100$$

Where $EC_{50}$ Standard = molar concentration of unlabeled progesterone, dexamethasone or estradiol required to decrease bound [$^{3}$H]-progesterone (PR), [$^{3}$H]-dexamethasone (GR) or [$^{3}$H]-estradiol to 50% of the respective buffer control (100% bound radioligand) and $EC_{50}$ Test Compound = molar concentration of test compound required to decrease bound

[$^3$H]-progesterone (PR), [$^3$H]-dexamethasone (GR) or [$^3$H]-estradiol to 50% of the respective buffer control (100% bound radioligand).

**RESULTS**

**EXAMPLE 1**

Since mifepristone (CDB-2477) is frequently used as a reference standard, Table 2, *infra,* contains data comparing the antiprogestational activity and relative binding affinity for the progesterone and glucocorticoid receptors of CDB-2914 with this standard. Recent studies have shown a good correlation between relative binding affinity for the glucocorticoid receptor and a biological test based upon the antagonism of dexamethasone-induced thymus involution in adrenalectomized male rats.

**Table 2**

| CDB NO. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoital[3] | Antiovulator[4] |
| 2914 | 69B | 122 (234) | 114 | 100 | 2 | 1 |
| 3875 | 69A | 164 | 30 | 97 | | |
| 3247 | 69C | 91 | 49 | -10 | 2* | |
| 3248 | 69D | 40 | 89 | weak (subcu) | inactive @2* | |
| 4243 | 91 | 171 | 59 | inactive | | |
| 4418 | 70 | 79 | /2 | ~25 | | |
| 4363 | 71 | 123 (203) | 20 | 253 | 0.5 | >16 |
| 4399 | 72 | 109 | 110 | 35 | | |
| 4176 | 74 | 131 | 32 | <10 | | |
| 4324 | 97a | 120 | 52 | 110 | | |
| 4398 | 97b | 47 | 38 | 99 | | |
| 4455 | 106a | | | | | |
| 4241 | 106b | 136 (172) | 14 | 34 | | |
| 4400 | 113A | 117(237) | 62 | 229 | | |
| 4454 | 113B | 59 | 34 | | | |
| 4417 | 113c | 63 | 45 | 70 | | |
| 4239 | 123a | 174 (140) | 11 | 45-83 | | |
| 4416 | 123b | 64 | 45 | 77 | | |
| 4393 | 139 | 30 | 79 | inactive | | |
| 4247 | 126a | 95 | 43 | 170 | | |
| 4362 | 126b | 76 | 15 | 125 | | |
| 4374 | 126c | 68 | 67 | 224 | | |
| 4361 | 129 | 155 | 20 | 303 | | |
| 4306 | 133 | 82 | 13 | 95 | | |

(continued)

| CDB NO. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoital[3] | Antiovulator[4] |
| 4352 | 138 | 63 | 14 | 57 | | |

[1]Progesterone receptor (estrogen-primed rabbit uterus); progesterone = 100%
Figure in 0 is relative binding affinity of the human isoform A progesterone receptor
Glucocorticoid receptor (estrogen-primed rabbit thymus) dexamethasone =100%.
[2]antiClauberg - oral except where indicated; CDB-2914 =100 (assigned).
[3]Postcoital- oral, rat $MED_{100}$ (mg/day) days 0-3 or *days 4-6 subcu; day sperm in vaginal washings = day 0.
[4]Antiovulatory - oral, rat $MED_{100}$(mg) single dose at noon on day of proestrus.

**EXAMPLE 2**

***AntiClauberg***

Data from antiClauberg tests following oral administration are shown in Tables 1 and 2. Compounds **15, 38, 40, 41, 46, 71, 97a, 113a, 126a, 126b, 126c** and **129** exhibited greater activity than the standard, **69B.** Previous studies have shown that 69B is significantly more potent than mifepristone (3.27 X; 95% C.I. = 1.41-7.58) in this test. Compounds **15, 38, 71** and **129** represent four of the most potent antiprogestational compounds known, and their low binding affinity for the glucocorticoid receptor would predict minimal antiglucocorticoid activity.

***Posteoital***

Compound **71** exhibited about four times the postcoital contraceptive activity of the standard, compound **69B,** following oral administration on days 0-3 of gestation.

***Antiovulatory***

Compound **71** was not fully active at a dose level 16 times the $MED_{100}$ for the standard, compound **69B,** and compound **113a** exhibited only about 6% of the antiovulatory activity of the standard.

***Relative Binding Affinity for the Progesterone and Glucocorticoid Receptors***

Relative binding affinities for the progesterone receptor (estrogen-primed rabbit uterine cytosol) and glucocorticoid receptor (estrogen~primed rabbit thymic cytosol) are shown in **Table 1.** Several compounds were also tested for binding affinity for the human isoform A progesterone receptor. Compounds 12, 13, 14A, 14B, 15, 28, 38, 69A, 91, 71, 72, 73, 97a, 106b, 113a, 113d, 112b and 129 showed binding affinities greater than that observed for the standard, compound 69B. On the other hand, most of the compounds tested exhibited reduced binding affinity for both the progesterone and the glucocorticoid receptor.

**DISCUSSION**

Many members of a series of derivatives of 19-norprogesterone possess potent antiprogestational activity following oral administration in experimental animals. They exhibit high binding affinity for the progesterone receptor (rabbit uterine) and only modest relative binding affinity for the glucocorticoid receptor (rabbit thymus). This is reflected in standard antiprogestational assays showing strong inhibition of progesterone-induced alterations of rabbit uterine endometrium. It is anticipated that the reduced binding affinity for the glucocorticoid receptor will reflect diminished biological antiglucocorticoid activity.

Antiprogestational agents including mifepristone are known to prevent implantation in the rat (Dao, B., et al., Contraception, 54:243-258 (1996); Reel, J., et al., Contraception, 58:129-136 (1998)), guinea pig (Batista, M., et al., Am, J. Obstet. Gynecol., 165:82-86 (1991), and man (Baulieu, E., Clinical Applications of Mifepristone (RU 486) and Other Antiprogestins (Donaldson, M., Dorflinger, L., Brown, S. and Benet, L. (eds.), National Academy Press, pp. 72-119 (1993)). Compound 71 was four times as potent as the standard, compound 69B, in preventing pregnancy when orally administered on days 0-3 of presumptive gestation. Curiously, compound **71** was only about 5% as potent as the standard in inhibiting ovulation Both compound **69B** and mifepristone have been shown to inhibit ovulation in the rat (Dao, *et al.,*

*supra*), and mifepristone has been shown to affect ovulation in human subjects (Baulieu, *et al., supra*). Compound **69B** has been shown to affect both follicular development and ovulation as well as endometrial maturation in human subjects following a single oral dose (unpublished data).

Compound **113a** exhibited high binding affinity for both the rabbit progesterone receptor (isoform B) and the human progesterone receptor (isoform A). This was reflected in potent antiprogestational activity *in vivo* where it was more than twice as active at the standard, compound **69B**. It also showed reduced binding affinity for the glucocorticoid receptor and was about half as effective as compound 69B in preventing pregnancy in the postcoital test. Strangly, this compound was only 6% as active as the standard in inhibiting ovulation. Thus, compound **113a** may represent an antiprogestational steroid with high tissue specificity.

## Claims

1. A compound having the general formula:

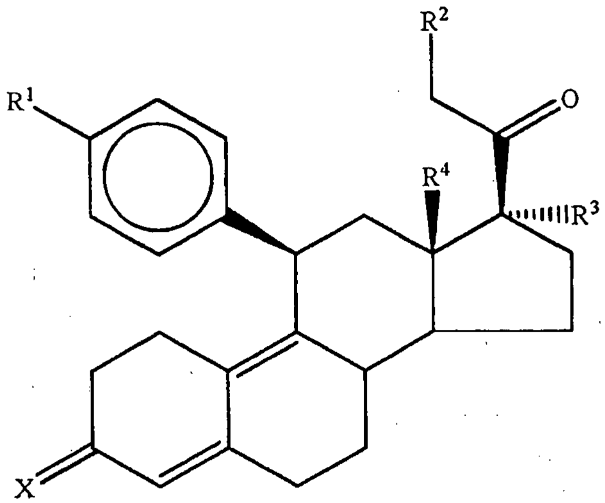

wherein
$R^1$ is $-NC_5H_{10}$;
$R^2$ is hydrogen;
$R^3$ is methoxy;
$R^4$ is methyl; and
X is =O;
$R^1$ is $-NC_5H_{10}$;
$R^2$ is acetoxy;
$R^3$ is acetoxy;
$R^4$ is methyl; and
X is =O;
$R^1$ is $-NC_5H_{10}$;
$R^2$ is methoxy;
$R^3$ is methoxy;
$R^4$ is methyl; and
X is =O;
$R^1$ is $-NC_5H_{10}$;
$R^2$ is methoxy;
$R^3$ is acetoxy;
$R^4$ is methyl; and
X is =O;
$R^1$ is $-NC_5H_{10}$;
$R^2$ is hydrogen;
$R^3$ is acetoxy;
$R^4$ is methyl; and

X is =N-OR$^5$, wherein R$^5$ is hydrogen;
$R^1$ is $-NC_5H_{10}$;
$R^2$ is hydrogen;
$R^3$ is methoxy;
$R^4$ is methyl; and
X is N-OR$^5$, wherein R$^5$ is hydrogen;
$R^1$ is $-NC_5H_{10}$;
$R^2$ is H;
$R^3$ is OH;
$R^4$ is methyl; and
X is =O;
$R^1$ is $-NC_5H_{10}$;
$R^2$ is Cl;
$R^3$ is OH;

$R^4$ is methyl; and X is =O;
$R^1$ is $NC_5H_{10}$;
$R^2$ is acetoxy;
$R^3$ is OH;
$R^4$ is methyl; and
X is =O; or
$R^1$ is $-NC_5H_{10}$;
$R^2$ is methoxy;
$R^3$ is OH;
$R^4$ is methyl; and
X is =O and wherein, in all instances, $-NC_5H_{10}$ signifies an N-piperidino group.

**2.** The compound of claim 1, wherein:

$R^1$ is $-NC_5H_{10}$;
$R^2$ is hydrogen;
$R^3$ is methoxy;
$R^4$ is methyl; and
X is =O.

**3.** The compound of claim 1, wherein:

$R^1$ is $-NC_5H_{10}$;
$R^2$ is acetoxy;
$R^3$ is acetoxy;
$R^4$ is methyl; and
X is =O.

**4.** The compound of claim 1, wherein:

$R^1$ is $-NC_5H_{10}$;
$R^2$ is methoxy;
$R^3$ is methoxy;
$R^4$ is methyl; and
X is =O.

**5.** The compound of claim 1, wherein:

$R^1$ is $-NC_5H_{10}$;
$R^2$ is methoxy;
$R^3$ is acetoxy;
$R^4$ is methyl; and
X is =O.

**6.** The compound of claim 1, wherein:

$R^1$ is $-NC_5H_{10}$;
$R^2$ is hydrogen;
$R^3$ is acetoxy;
$R^4$ is methyl; and
X is $=N-OR^5$, wherein $R^5$ is hydrogen.

**7.** The compound of claim 1, wherein:

$R^1$ is $-NC_5H_{10}$;
$R^2$ is hydrogen;
$R^3$ is methoxy;
$R^4$ is methyl; and
X is $=N-OR^5$, wherein $R^5$ is hydrogen.

**8.** A pharmaceutical composition comprising an effective amount of a compound in accordance with any one of claims 1 to 7 and a pharmaceutically acceptable excipient.

**9.** A compound in accordance with any one of claims 1 to 7 or a pharmaceutical composition in accordance with claim 8, for use in producing an antiprogestational effect in a patient.

**10.** A compound in accordance with any one of claims 1 to 7 or a pharmaceutical composition in accordance with claim 8, for use in

(a) inducing menses;
(b) treating endometriosis;
(c) treating dysmenorrhea;
(d) treating an endocrine hormone-dependent tumor;
(e) treating meningiomas;
(f) treating uterine fibroids;
(g) inhibiting uterine endometrial proliferation; or
(h) inducing labor in a patient.

**11.** Use of a compound in accordance with any one of claims 1 to 7 or a pharmaceutical composition in accordance with claim 8 in the preparation of a contraceptive.

**12.** Use of a compound in accordance with any one of claims 1 to 7 or a pharmaceutical composition in accordance with claim 8 in the preparation of a postcoital contraceptive.

**Patentansprüche**

**1.** Eine Verbindung mit der allgemeinen Formel:

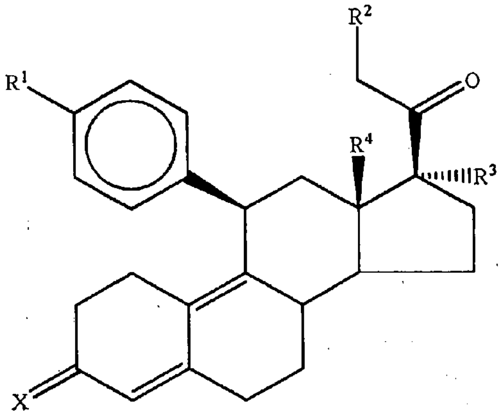

worin:

$R^1$ ist $-NC_5H_{10}$;
$R^2$ ist Wasserstoff;
$R^3$ ist Methoxy;
$R^4$ ist Methyl; und
X ist =O;
$R^1$ ist $-NC_5H_{10}$;
$R^2$ ist Acetoxy;
$R^3$ ist Acetoxy;
$R^4$ ist Methyl; und
X ist =O;
$R^1$ ist $-NC_5H_{10}$;
$R^2$ ist Methoxy;
$R^3$ ist Methoxy;
$R^4$ ist Methyl; und
X ist =O;
$R^1$ ist $-NC_5H_{10}$;
$R^2$ ist Methoxy;
$R^3$ ist Acetoxy;
$R^4$ ist Methyl; und
X ist =O;
$R^1$ ist $-NC_5H_{10}$;
$R^2$ ist Wasserstoff;
$R^3$ ist Acetoxy;
$R^4$ ist Methyl; und
X ist =N-OR$^5$, wobei $R^5$ Wasserstoff ist;
$R^1$ ist $-NC_5H_{10}$;
$R^2$ ist Wasserstoff;
$R^3$ ist Methoxy;
$R^4$ ist Methyl; und
X ist =N-OR$^5$, wobei $R^5$ Wasserstoff ist;
$R^1$ ist $-NC_5H_{10}$;
$R^2$ ist H;
$R^3$ ist OH;
$R^4$ ist Methyl; und
X ist =O;
$R^1$ ist $-NC_5H_{10}$;
$R^2$ ist Cl;
$R^3$ ist OH;
$R^4$ ist Methyl; und
X ist =O;

$R^1$ ist $-NC_5H_{10}$;
$R^2$ ist Acetoxy;
$R^3$ ist OH;
$R^4$ ist Methyl; und
X ist =O; oder
$R^1$ ist $-NC_5H_{10}$;
$R^2$ ist Methoxy;
$R^3$ ist OH;
$R^4$ ist Methyl; und
X ist =O, und wobei, in allen Fällen, $-NC_5H_{10}$ eine N-Piperidino-Gruppe bedeutet.

**2.** Die Verbindung nach Anspruch 1, worin:

$R^1$ ist $-NC_5H_{10}$;
$R^2$ ist Wasserstoff;
$R^3$ ist Methoxy;
$R^4$ ist Methyl; und
X ist =O.

**3.** Die Verbindung nach Anspruch 1, worin:

$R^1$ ist $-NC_5H_{10}$;
$R^2$ ist Acetoxy;
$R^3$ ist Acetoxy;
$R^4$ ist Methyl; und
X ist =O.

**4.** Die Verbindung nach Anspruch 1, worin:

$R^1$ ist $-NC_5H_{10}$;
$R^2$ ist Methoxy;
$R^3$ ist Methoxy;
$R^4$ ist Methyl; und
X ist =O.

**5.** Die Verbindung nach Anspruch 1, worin:

$R^1$ ist $-NC_5H_{10}$;
$R^2$ ist Methoxy;
$R^3$ ist Acetoxy;
$R^4$ ist Methyl; und
X ist =O.

**6.** Die Verbindung nach Anspruch 1, worin:

$R^1$ ist $-NC_5H_{10}$;
$R^2$ ist Wasserstoff;
$R^3$ ist Acetoxy;
$R^4$ ist Methyl; und
X ist $=N-OR^5$, wobei $R^5$ Wasserstoff ist.

**7.** Die Verbindung nach Anspruch 1, worin:

$R^1$ ist $-NC_5H_{10}$;
$R^2$ ist Wasserstoff;
$R^3$ ist Methoxy;
$R^4$ ist Methyl; und
X ist $=N-OR^5$, wobei $R^5$ Wasserstoff ist.

**8.** Eine pharmazeutische Zusammensetzung, umfassend eine wirksame Menge einer Verbindung gemäß einem beliebigen der Ansprüche 1 bis 7 und einen pharmazeutisch annehmbaren Hilfsstoff.

**9.** Eine Verbindung gemäß einem beliebigen der Ansprüche 1 bis 7 oder eine pharmazeutische Zusammensetzung gemäß Anspruch 8 zur Verwendung bei der Hervorrufung eines antiprogestationalen Effektes in einem Patienten.

**10.** Eine Verbindung gemäß einem beliebigen der Ansprüche 1 bis 7 oder eine pharmazeutische Zusammensetzung gemäß Anspruch 8 zur Verwendung für das:

(a) Induzieren von Menstruationen;
(b) Behandeln von Endometriose;
(c) Behandeln von Dysmenorrhö;
(d) Behandeln eines endokrinen hormonabhängigen Tumors;
(e) Behandeln von Meningiomen;
(f) Behandeln von Uterusfibroiden;
(g) Inhibieren der uterusendometrialen Proliferation; oder
(h) Induzieren von Wehen in einem Patienten.

**11.** Verwendung einer Verbindung gemäß einem beliebigen der Ansprüche 1 bis 7 oder einer pharmazeutischen Zusammensetzung gemäß Anspruch 8 zur Herstellung eines Kontrazeptivums.

**12.** Verwendung einer Verbindung gemäß einem beliebigen der Ansprüche 1 bis 7 oder einer pharmazeutischen Zusammensetzung gemäß Anspruch 8 zur Herstellung eines postkoitalen Kontrazeptivums.

## Revendications

**1.** Composé de formule générale

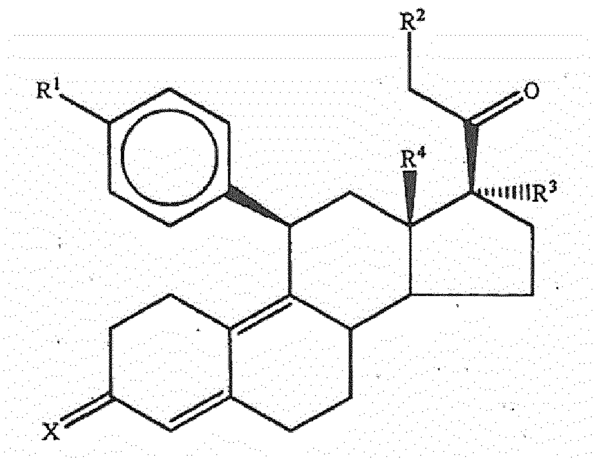

dans laquelle

- $R^1$ représente un groupe de formule $-NC_5H_{10}$,
- $R^2$ représente un atome d'hydrogène,
- $R^3$ représente un groupe méthoxy,
- $R^4$ représente un groupe méthyle,
- et X représente un substituant oxo (=O) ;

ou bien

- $R^1$ représente un groupe de formule $-NC_5H_{10}$,
- $R^2$ représente un groupe acétoxy,
- $R^3$ représente un groupe acétoxy,

- $R^4$ représente un groupe méthyle,
- et X représente un substituant oxo (=O) ;

ou bien

- $R^1$ représente un groupe de formule $-NC_5H_{10}$,
- $R^2$ représente un groupe méthoxy,
- $R^3$ représente un groupe méthoxy,
- $R^4$ représente un groupe méthyle,
- et X représente un substituant oxo (=O) ;

ou bien

- $R^1$ représente un groupe de formule $-NC_5H_{10}$,
- $R^2$ représente un groupe méthoxy,
- $R^3$ représente un groupe acétoxy,
- $R^4$ représente un groupe méthyle,
- et X représente un substituant oxo (=O) ;

ou bien

- $R^1$ représente un groupe de formule $-NC_5H_{10}$,
- $R^2$ représente un atome d'hydrogène,
- $R^3$ représente un groupe acétoxy,
- $R^4$ représente un groupe méthyle,
- et X représente un substituant de formule $=N-OR^5$ dans laquelle $R^5$ représente un atome d'hydrogène ;

ou bien

- $R^1$ représente un groupe de formule $-NC_5H_{10}$,
- $R^2$ représente un atome d'hydrogène,
- $R^3$ représente un groupe méthoxy,
- $R^4$ représente un groupe méthyle,
- et X représente un substituant de formule $=N-OR^5$ dans laquelle $R^5$ représente un atome d'hydrogène ;

ou bien

- $R^1$ représente un groupe de formule $-NC_5H_{10}$,
- $R^2$ représente un atome d'hydrogène,
- $R^3$ représente un groupe hydroxyle,
- $R^4$ représente un groupe méthyle,
- et X représente un substituant oxo (=O) ;

ou bien

- $R^1$ représente un groupe de formule $-NC_5H_{10}$,
- $R^2$ représente un atome de chlore,
- $R^3$ représente un groupe hydroxyle,
- $R^4$ représente un groupe méthyle,
- et X représente un substituant oxo (=O) ;

ou bien

- $R^1$ représente un groupe de formule $-NC_5H_{10}$,
- $R^2$ représente un groupe acétoxy,
- $R^3$ représente un groupe hydroxyle,
- $R^4$ représente un groupe méthyle,
- et X représente un substituant oxo (=O) ;

ou bien

- $R^1$ représente un groupe de formule $-NC_5H_{10}$,
- $R^2$ représente un groupe méthoxy,
- $R^3$ représente un groupe hydroxyle,
- $R^4$ représente un groupe méthyle,
- et X représente un substituant oxo (=O) ;

étant entendu que dans tous les cas, le groupe de formule $-NC_5H_{10}$ est un groupe N-pipéridino.

**2.** Composé conforme à la revendication 1, dans lequel

- $R^1$ représente un groupe de formule $-NC_5H_{10}$,
- $R^2$ représente un atome d'hydrogène,
- $R^3$ représente un groupe méthoxy,
- $R^4$ représente un groupe méthyle,
- et X représente un substituant oxo (=O).

**3.** Composé conforme à la revendication 1, dans lequel

- $R^1$ représente un groupe de formule $-NC_5H_{10}$,
- $R^2$ représente un groupe acétoxy,
- $R^3$ représente un groupe acétoxy,
- $R^4$ représente un groupe méthyle,
- et X représente un substituant oxo (=O).

**4.** Composé conforme à la revendication 1, dans lequel

- $R^1$ représente un groupe de formule $-NC_5H_{10}$,
- $R^2$ représente un groupe méthoxy,
- $R^3$ représente un groupe méthoxy,
- $R^4$ représente un groupe méthyle,
- et X représente un substituant oxo (=O).

**5.** Composé conforme à la revendication 1, dans lequel

- $R^1$ représente un groupe de formule $-NC_5H_{10}$,
- $R^2$ représente un groupe méthoxy,
- $R^3$ représente un groupe acétoxy,
- $R^4$ représente un groupe méthyle,
- et X représente un substituant oxo (=O).

**6.** Composé conforme à la revendication 1, dans lequel

- $R^1$ représente un groupe de formule $-NC_5H_{10}$,
- $R^2$ représente un atome d'hydrogène,
- $R^3$ représente un groupe acétoxy,
- $R^4$ représente un groupe méthyle,
- et X représente un substituant de formule $=N-OR^5$ dans laquelle $R^5$ représente un atome d'hydrogène.

**7.** Composé conforme à la revendication 1, dans lequel

- $R^1$ représente un groupe de formule $-NC_5H_{10}$,
- $R^2$ représente un atome d'hydrogène,
- $R^3$ représente un groupe méthoxy,
- $R^4$ représente un groupe méthyle,
- et X représente un substituant de formule $=N-OR^5$ dans laquelle $R^5$ représente un atome d'hydrogène.

**8.** Composition pharmaceutique comprenant, en une quantité efficace, un composé conforme à l'une des revendications 1 à 7, ainsi qu'un excipient pharmacologiquement admissible.

**9.** Composé conforme à l'une des revendications 1 à 7, ou composition pharmaceutique conforme à la revendication 8, pour utilisation dans la production d'un effet anti-progestationnel chez une patiente.

**10.** Composé conforme à l'une des revendications 1 à 7, ou composition pharmaceutique conforme à la revendication 8, pour utilisation

a) dans l'induction des règles ;
b) dans le traitement d'une endométriose ;
c) dans le traitement d'une dysménorrhée ;
d) dans le traitement d'une tumeur dépendant d'une hormone endocrinienne ;
e) dans le traitement d'un méningiome ;
f) dans le traitement d'un fibrome utérin ;
g) dans l'inhibition de la prolifération de l'endomètre dans l'utérus ;
h) ou dans l'induction du travail chez une patiente.

**11.** Utilisation d'un composé conforme à l'une des revendications 1 à 7, ou d'une composition pharmaceutique conforme à la revendication 8, dans la préparation d'un contraceptif.

**12.** Utilisation d'un composé conforme à l'une des revendications 1 à 7, ou d'une composition pharmaceutique conforme à la revendication 8, dans la préparation d'un contraceptif post-coïtal.

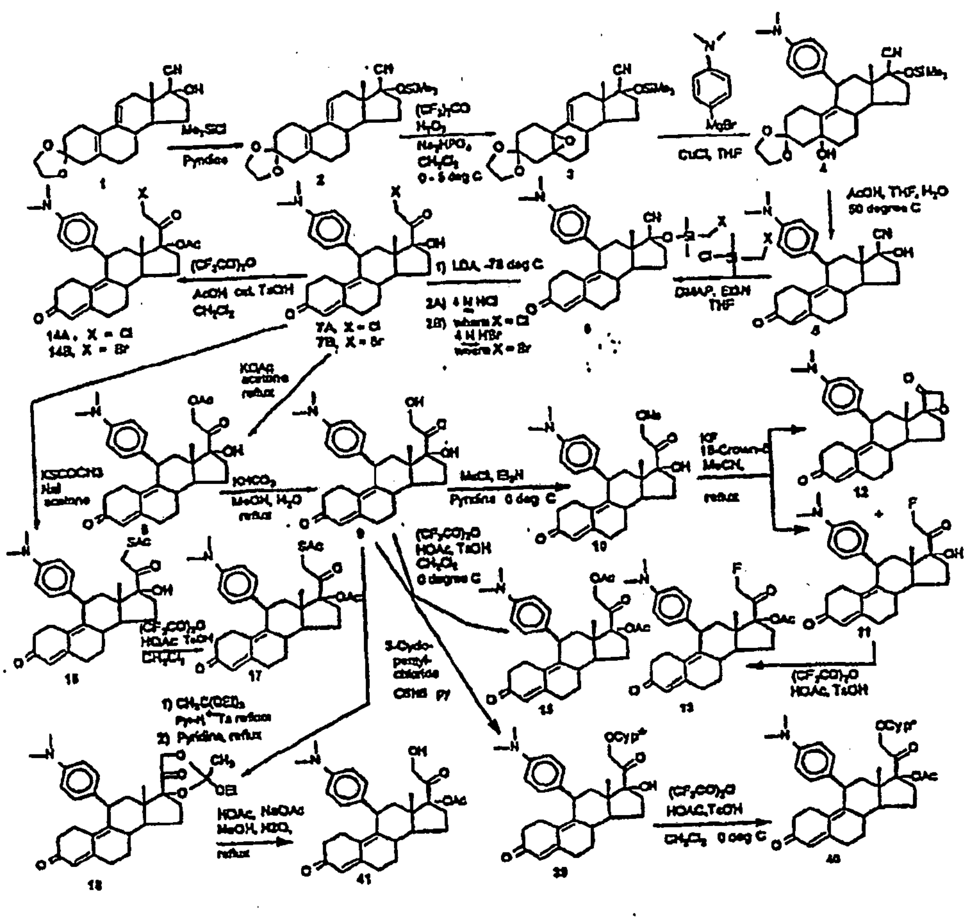
Me3SiCl
Pyridine
(CF3)2CO
H2O2
Na2HPO4
CH2Cl2
0 - 5 deg C
CuCl, THF
AcOH, THF, H2O
50 degree C
DMAP, Et3N
THF
1) LDA, -78 deg C
2A) 4 N HCl where X = Cl
2B) 4 N HBr where X = Br
(CF3CO)2O
AcOH cat. TsOH
CH2Cl2
14A, X = Cl
14B, X = Br
7A, X = Cl
7B, X = Br
KOAc
acetone
reflux
KSCOCH3
NaI
acetone
KHCO3
MeOH, H2O
reflux
MsCl, Et3N
Pyridine 0 deg C
KF
18-Crown-6
MeCN,
reflux
(CF3CO)2O
HOAc, TsOH
CH2Cl2
0 degree C
(CF3CO)2O
HOAc TsOH
CH2Cl2
(CF3CO)2O
HOAc, TsOH
5-Cyclopentylchloride
C6H6 py
1) CH3C(OEt)3
Py-H+Ts reflux
2) Pyridine, reflux
HOAc, NaOAc
MeOH, H2O,
reflux
(CF3CO)2O
HOAC,TsOH
CH2Cl2 0 deg C

FIGURE 1

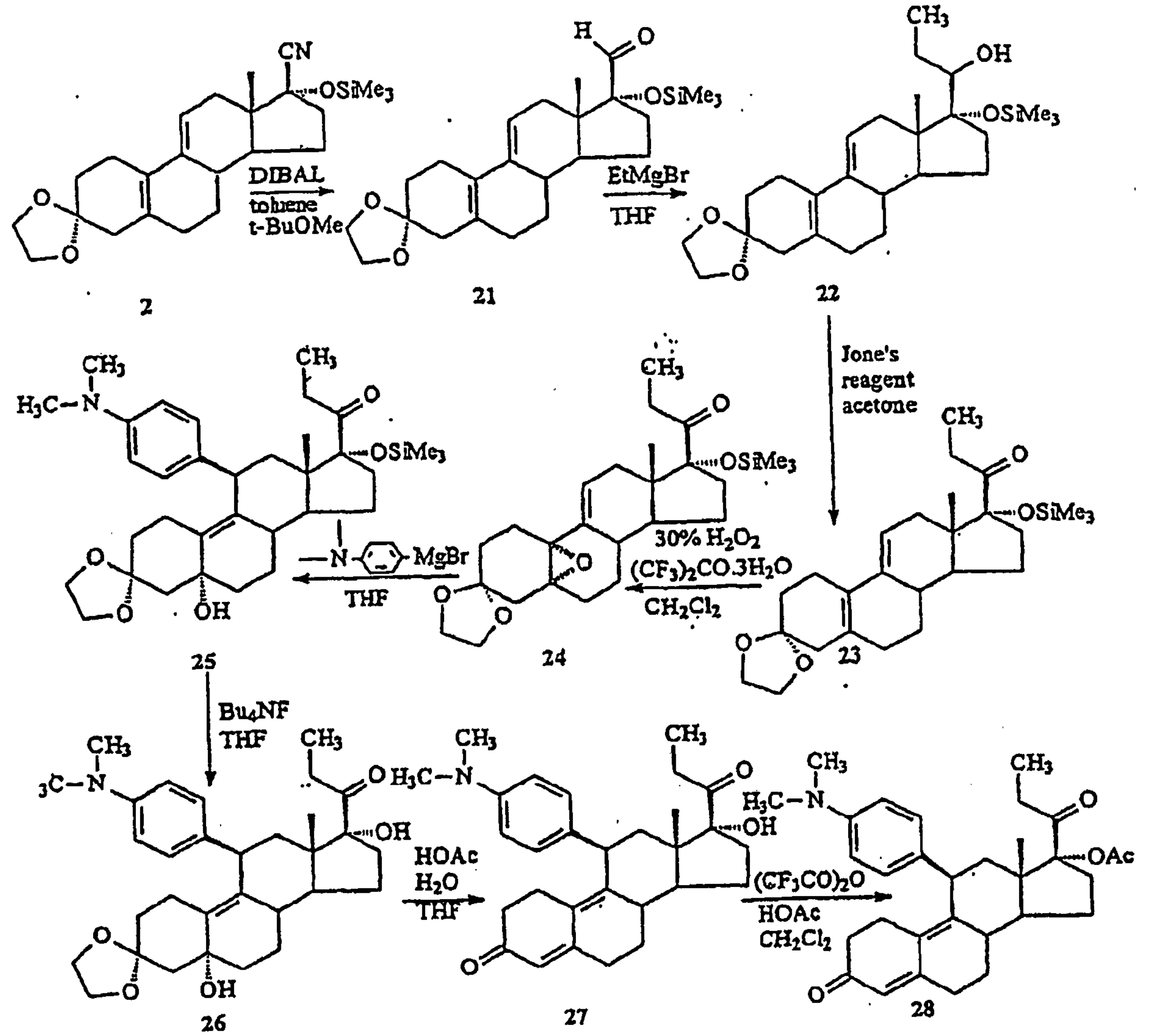
DIBAL
toluene
t-BuOMe
EtMgBr
THF
Jone's
reagent
acetone
30% $H_2O_2$
$(CF_3)_2CO.3H_2O$
$CH_2Cl_2$
THF
$Bu_4NF$
THF
HOAc
$H_2O$
THF
$(CF_3CO)_2O$
HOAc
$CH_2Cl_2$
2
21
22
23
24
25
26
27
28

FIGURE 2

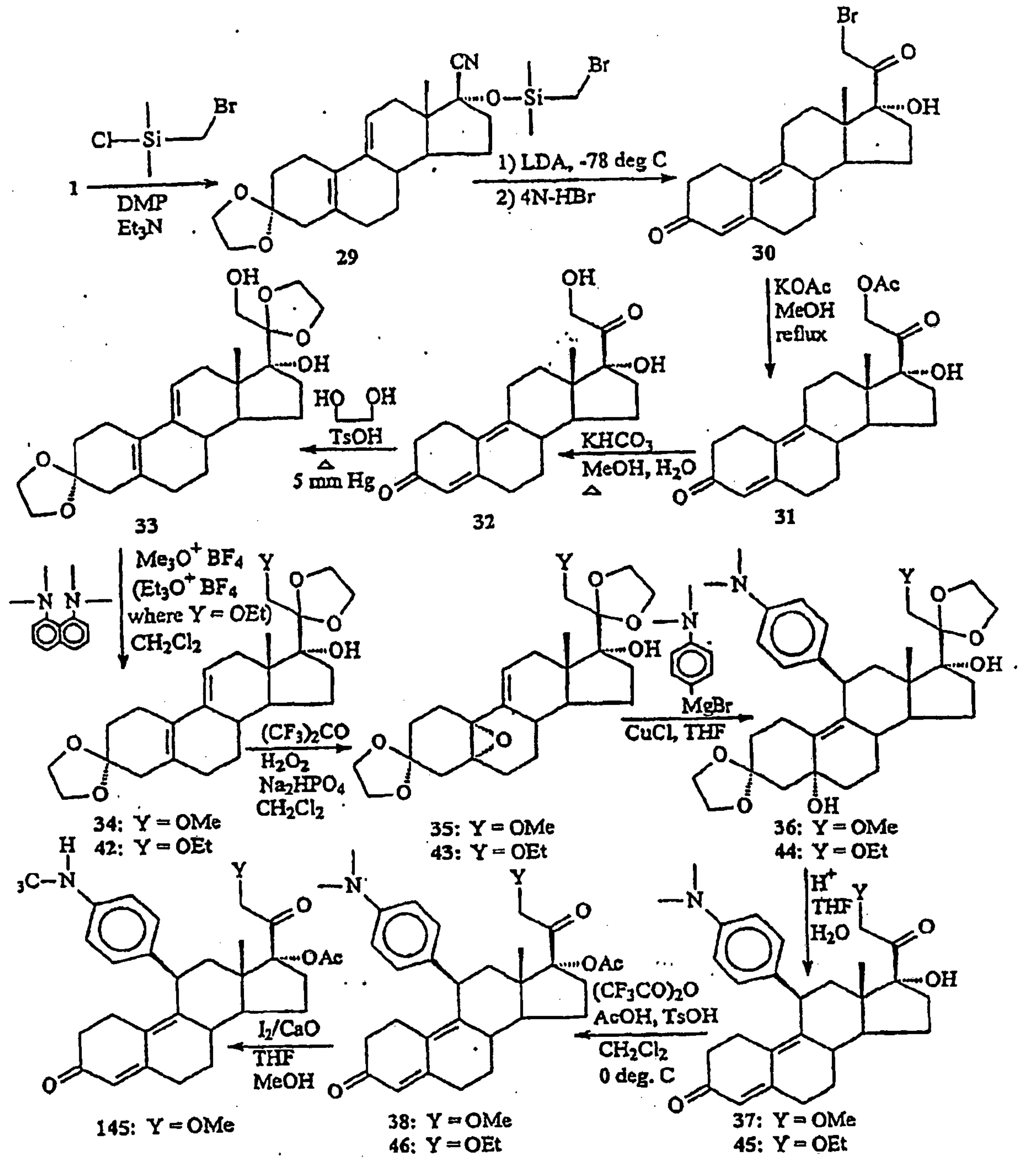


29
30
33
32
31
34: Y = OMe
42: Y = OEt
35: Y = OMe
43: Y = OEt
36: Y = OMe
44: Y = OEt
145: Y = OMe
38: Y = OMe
46: Y = OEt
37: Y = OMe
45: Y = OEt

FIGURE 3

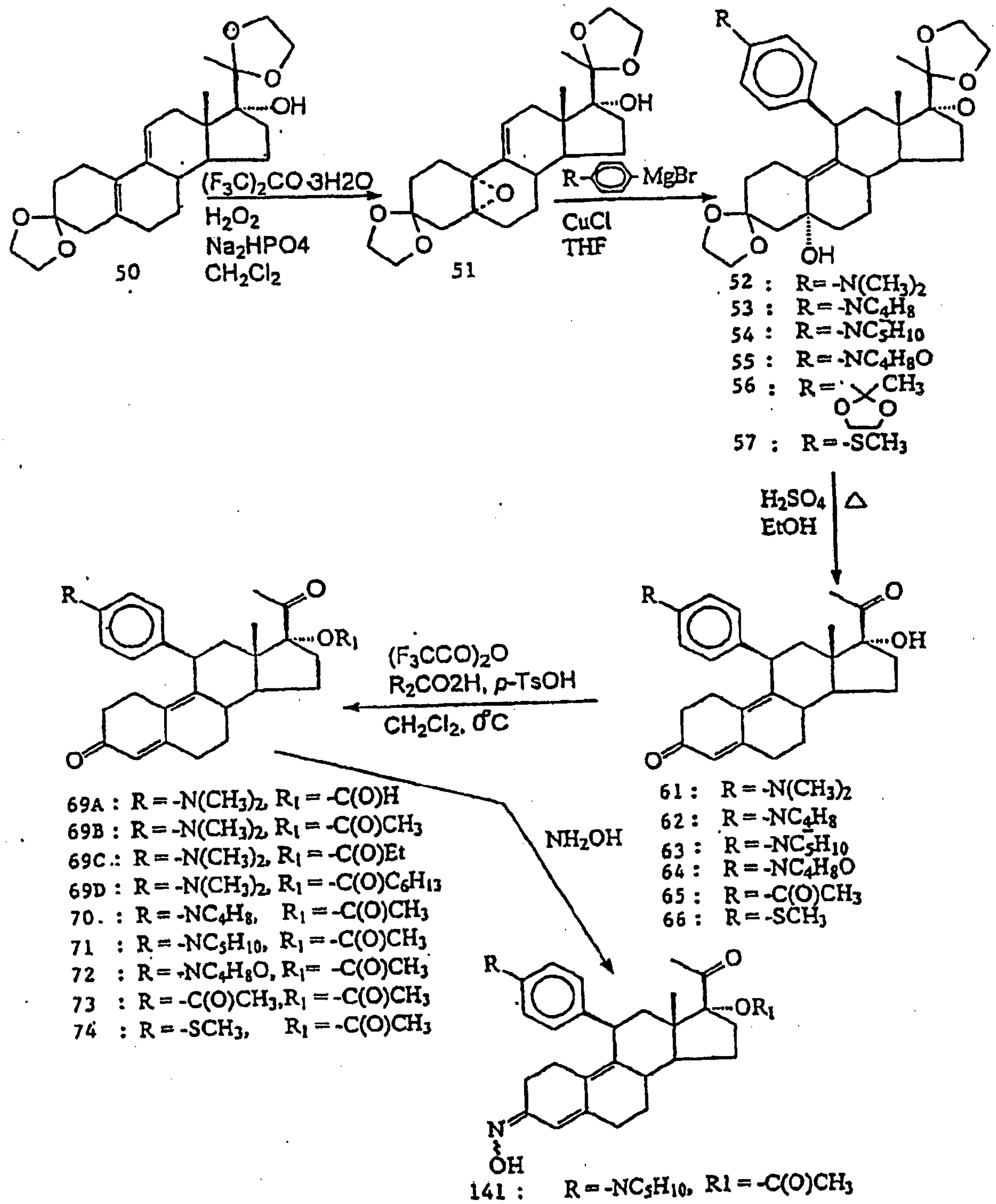


50

51

52 : $R = -N(CH_3)_2$
53 : $R = -NC_4H_8$
54 : $R = -NC_5H_{10}$
55 : $R = -NC_4H_8O$
56 : R =
57 : $R = -SCH_3$

61 : $R = -N(CH_3)_2$
62 : $R = -NC_4H_8$
63 : $R = -NC_5H_{10}$
64 : $R = -NC_4H_8O$
65 : $R = -C(O)CH_3$
66 : $R = -SCH_3$

69A : $R = -N(CH_3)_2$, $R_1 = -C(O)H$
69B : $R = -N(CH_3)_2$, $R_1 = -C(O)CH_3$
69C : $R = -N(CH_3)_2$, $R_1 = -C(O)Et$
69D : $R = -N(CH_3)_2$, $R_1 = -C(O)C_6H_{13}$
70. : $R = -NC_4H_8$, $R_1 = -C(O)CH_3$
71 : $R = -NC_5H_{10}$, $R_1 = -C(O)CH_3$
72 : $R = -NC_4H_8O$, $R_1 = -C(O)CH_3$
73 : $R = -C(O)CH_3$, $R_1 = -C(O)CH_3$
74 : $R = -SCH_3$, $R_1 = -C(O)CH_3$

141 : $R = -NC_5H_{10}$, $R1 = -C(O)CH_3$

Figure 4

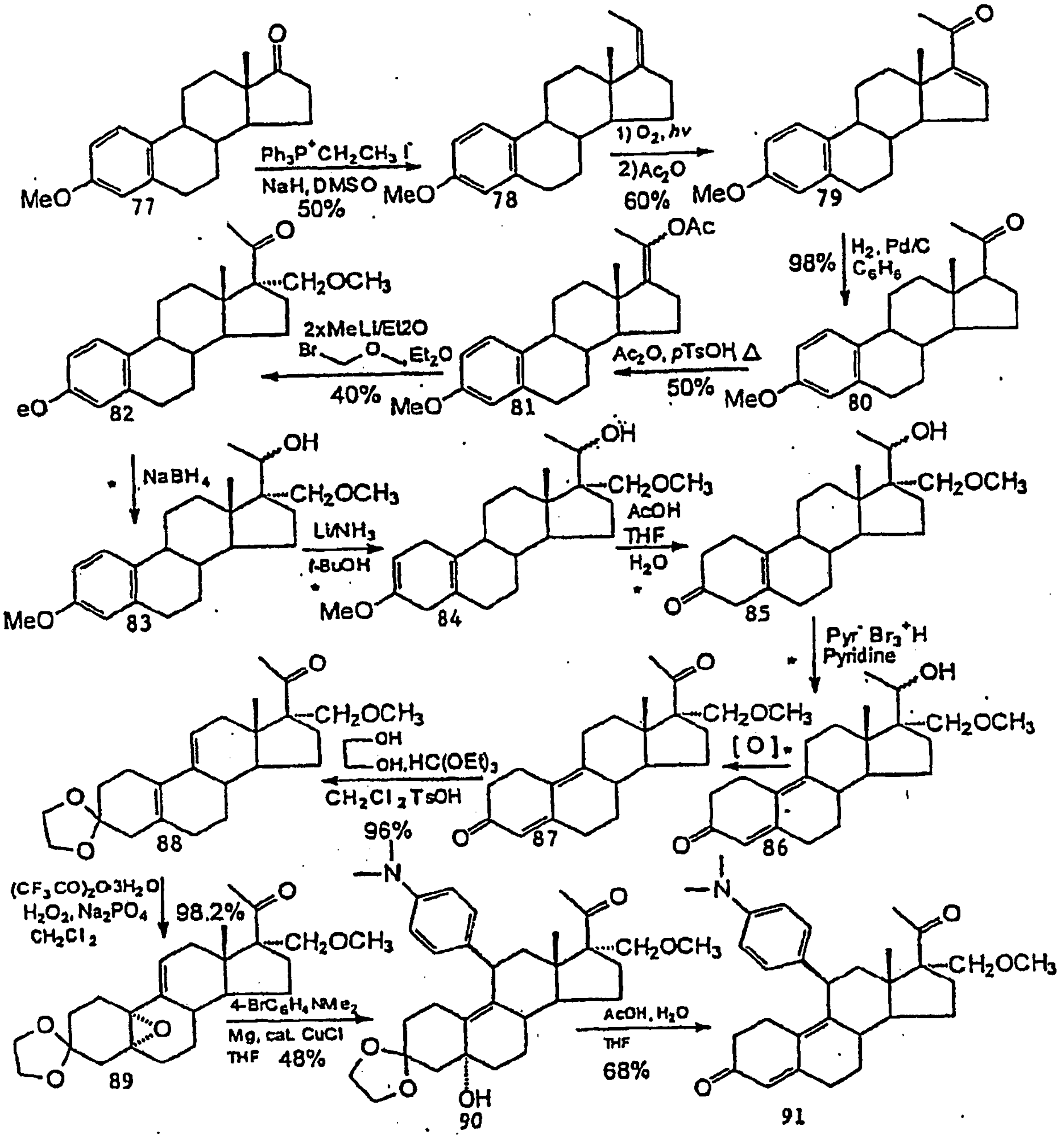


* Yield from 83 to 86is 37%

Figure 5

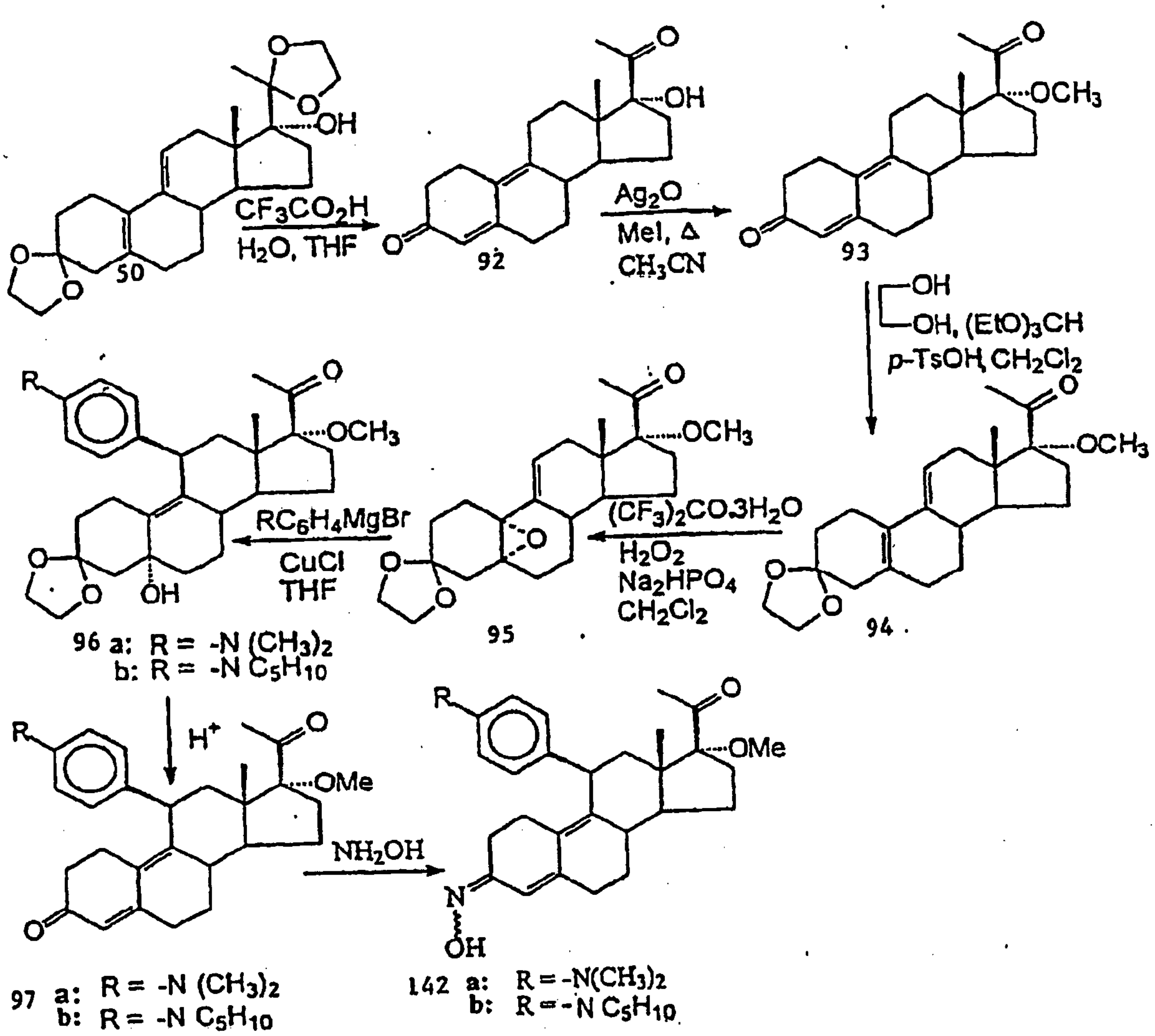

CF3CO2H
H2O, THF
50
92
Ag2O
MeI, Δ
CH3CN
93
OH, (EtO)3CH
p-TsOH, CH2Cl2
94
(CF3)2CO.3H2O
H2O2
Na2HPO4
CH2Cl2
95
RC6H4MgBr
CuCl
THF
96 a: R = -N (CH3)2
b: R = -N C5H10
H+
NH2OH
97 a: R = -N (CH3)2
b: R = -N C5H10
142 a: R = -N(CH3)2
b: R = -N C5H10


Figure 6

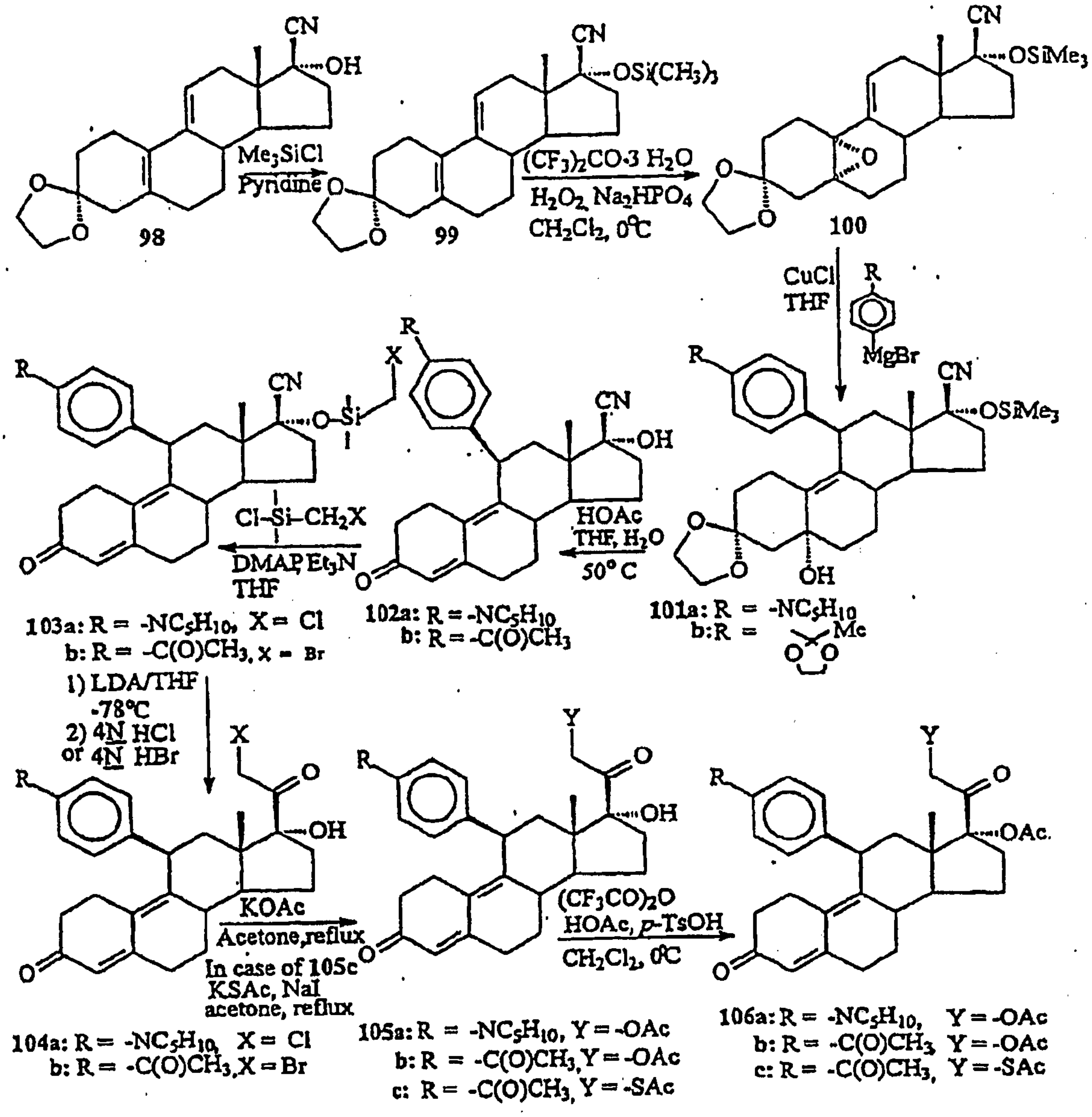
98
Me3SiCl
Pyridine
99
(CF3)2CO·3 H2O
H2O2, Na2HPO4
CH2Cl2, 0°C
100
CuCl
THF
MgBr
101a: R = -NC5H10
b: R =
Me
HOAc
THF, H2O
50° C
102a: R = -NC5H10
b: R = -C(O)CH3
Cl-Si-CH2X
DMAP, Et3N
THF
103a: R = -NC5H10, X = Cl
b: R = -C(O)CH3, X = Br
1) LDA/THF
-78°C
2) 4N HCl
or 4N HBr
KOAc
Acetone, reflux
In case of 105c
KSAc, NaI
acetone, reflux
104a: R = -NC5H10, X = Cl
b: R = -C(O)CH3, X = Br
(CF3CO)2O
HOAc, p-TsOH
CH2Cl2, 0°C
105a: R = -NC5H10, Y = -OAc
b: R = -C(O)CH3, Y = -OAc
c: R = -C(O)CH3, Y = -SAc
106a: R = -NC5H10, Y = -OAc
b: R = -C(O)CH3, Y = -OAc
c: R = -C(O)CH3, Y = -SAc

Figure 7

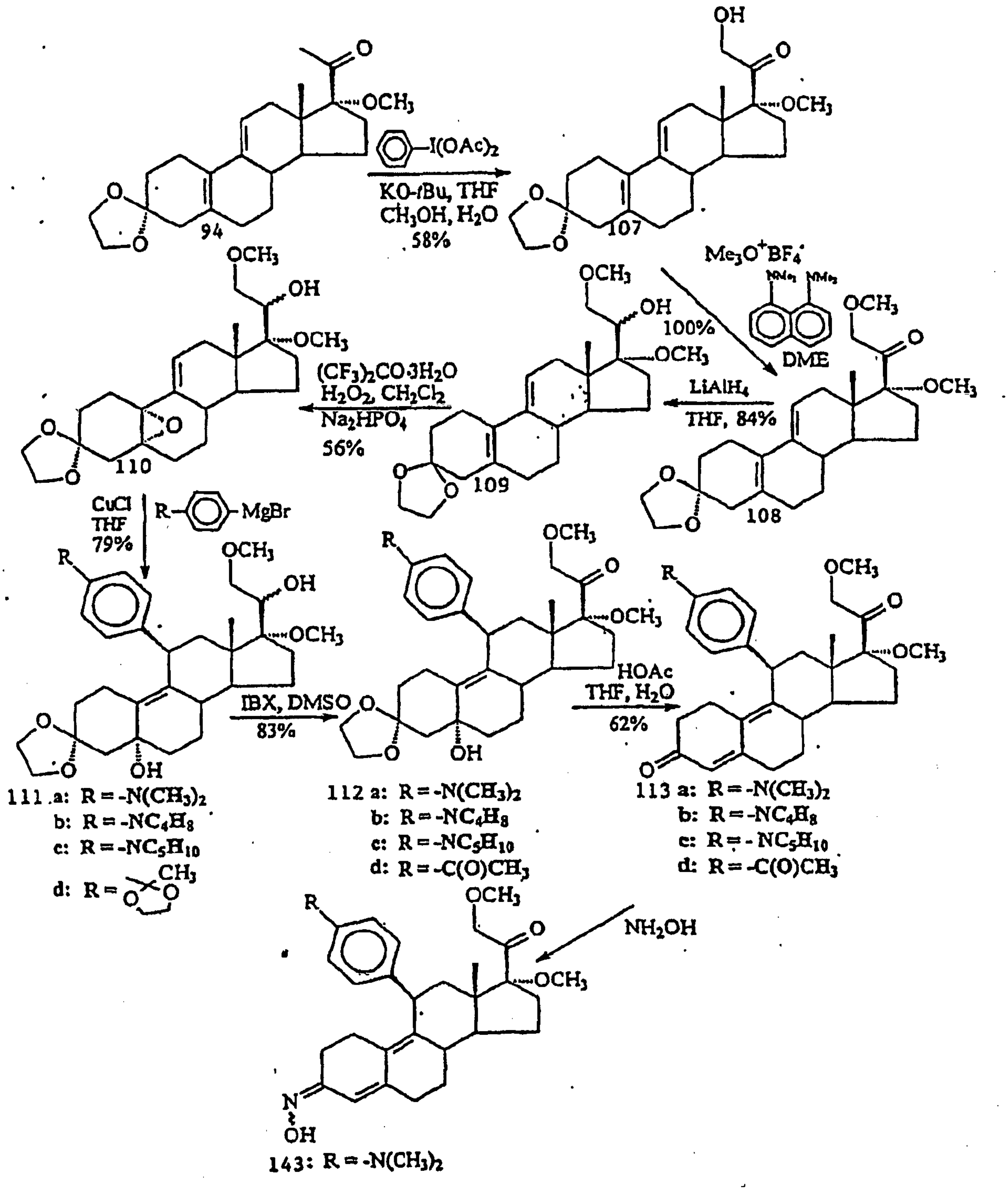


94

107

108

109

110

111 a: R = $-N(CH_3)_2$
b: R = $-NC_4H_8$
c: R = $-NC_5H_{10}$
d: R =

112 a: R = $-N(CH_3)_2$
b: R = $-NC_4H_8$
c: R = $-NC_5H_{10}$
d: R = $-C(O)CH_3$

113 a: R = $-N(CH_3)_2$
b: R = $-NC_4H_8$
c: R = $-NC_5H_{10}$
d: R = $-C(O)CH_3$

143: R = $-N(CH_3)_2$

Figure 8

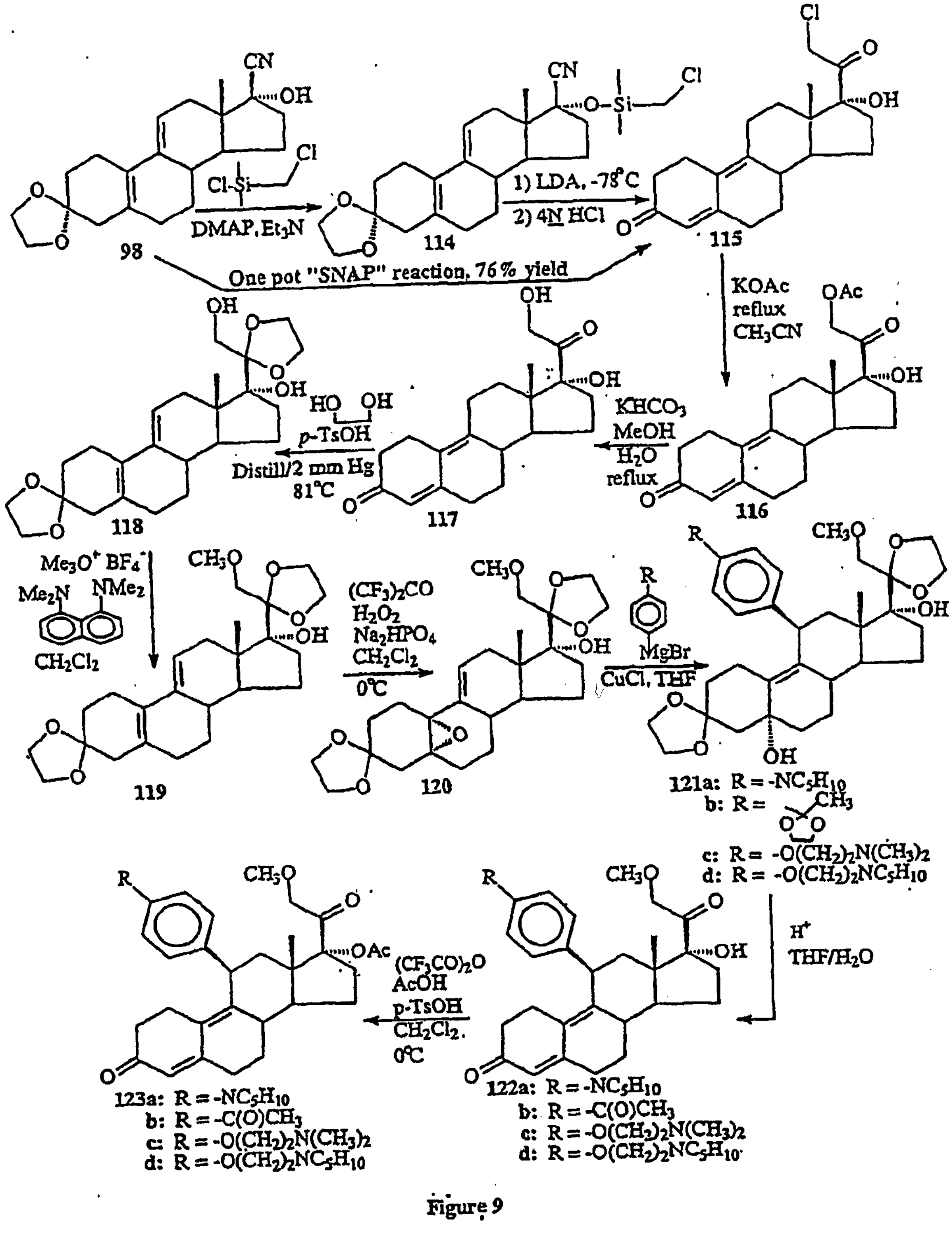
DMAP, Et3N
1) LDA, -78°C
2) 4N HCl
One pot "SNAP" reaction, 76% yield
KOAc
reflux
CH3CN
KHCO3
MeOH
H2O
reflux
HO OH
p-TsOH
Distill/2 mm Hg
81°C
Me3O+ BF4-
CH2Cl2
(CF3)2CO
H2O2
Na2HPO4
CH2Cl2
0°C
MgBr
CuCl, THF
H+
THF/H2O
(CF3CO)2O
AcOH
p-TsOH
CH2Cl2,
0°C
98
114
115
116
117
118
119
120
121a: R = -NC5H10
b: R =
c: R = -O(CH2)2N(CH3)2
d: R = -O(CH2)2NC5H10
122a: R = -NC5H10
b: R = -C(O)CH3
c: R = -O(CH2)2N(CH3)2
d: R = -O(CH2)2NC5H10
123a: R = -NC5H10
b: R = -C(O)CH3
c: R = -O(CH2)2N(CH3)2
d: R = -O(CH2)2NC5H10

Figure 9

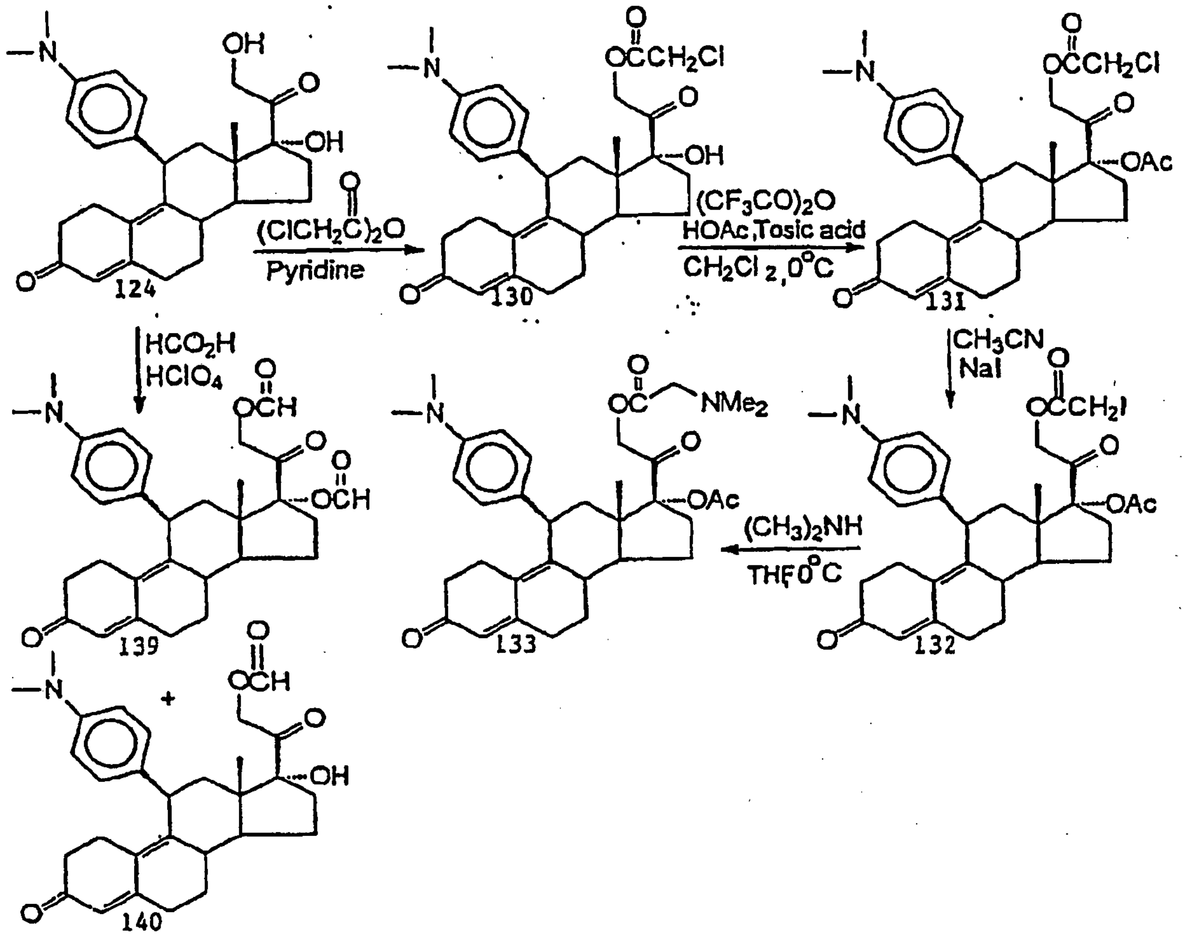
124
(ClCH2C)2O
Pyridine
130
(CF3CO)2O
HOAc,Tosic acid
CH2Cl 2, 0°C
131
HCO2H
HClO4
CH3CN
NaI
139
(CH3)2NH
THF,0°C
133
132
+
140

Figure 10

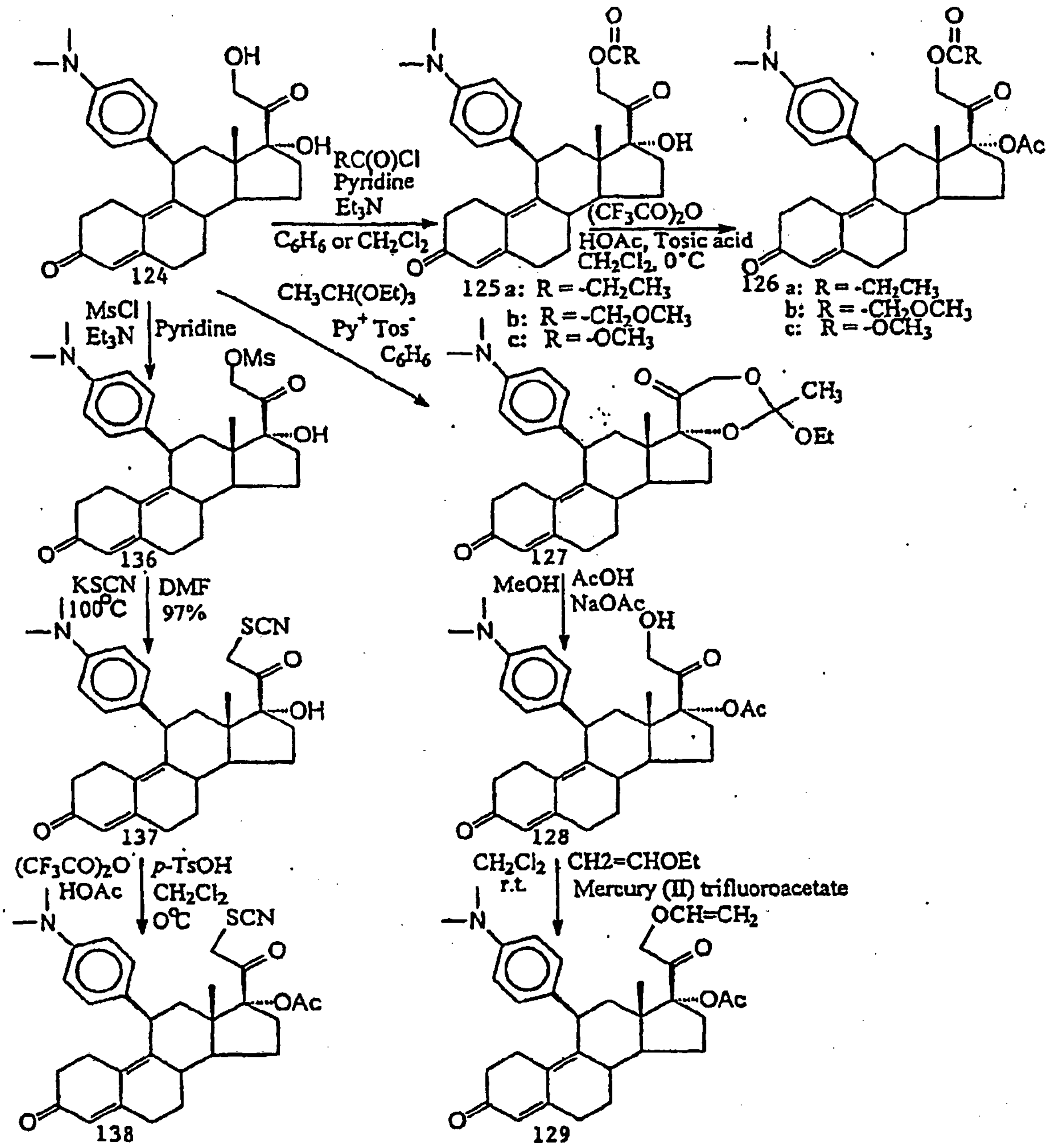
RC(O)Cl
Pyridine
Et3N
C6H6 or CH2Cl2
124
(CF3CO)2O
HOAc, Tosic acid
CH2Cl2, 0°C
125 a: R = -CH2CH3
b: R = -CH2OCH3
c: R = -OCH3
126 a: R = -CH2CH3
b: R = -CH2OCH3
c: R = -OCH3
MsCl
Et3N
Pyridine
CH3CH(OEt)3
Py+ Tos-
C6H6
136
127
KSCN
100°C
DMF
97%
MeOH
AcOH
NaOAc
137
128
(CF3CO)2O
HOAc
p-TsOH
CH2Cl2
0°C
CH2Cl2
r.t.
CH2=CHOEt
Mercury (II) trifluoroacetate
138
129

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9945022 A
- US 4092693 A, Livingston, D.A
- US 4977255 A, Livingston, D.A.


### Non-patent literature cited in the description


- **G. TEUTSCH.** Adrenal Steroid Antagonism. Water de Gruyter and Co, 1984, 43-75
- *IPPF Medical Bulletin,* 1986, vol. 20 (5
- **NIEMAN et al.** *J. Clin. Endocrinology Metab.,* 1985, vol. 61, 536
- **HORWITZ.** *Endocrinology,* 1985, vol. 116, 2236
- **HEIKINHEIMO et al.** *J. Steroid Biochem.,* 1987, vol. 26, 279
- **KETTEL, L.M. et al.** *Fertil Steril,* vol. 56, 402-407
- **MURPHY, A.A. et al.** *Fertil Steril,* vol. 6, 3761-766
- **GROW, D.R et al.** *J. Clin. Endocrinol. Metab.,* vol. 81, 1933-1939
- **MURPHY, A.A. et al.** *J. Clin. Endocrinol. Metab.,* vol. 76, 513-517
- **BROGDEN, R.N. et al.** *Drugs,* vol. 45, 384-409
- **POISSON, M. et al.** *J. Neurooncol.,* vol. 1, 179-189
- **CARROLL, R.S. et al.** *Cancer Res.,* vol. 53, 1312-1316
- **MAHAJAN, D.K. ; LONDON, S.N.** *Fertil Steril,* 1997, vol. 68, 967-976
- **ROCHEFORT, H.** *Trends in Pharmacol. Sci.,* vol. 8, 126-128
- **HORWITZ, K.B.** *Endocr. Rev.,* 1992, vol. 13, 146-163
- **MAHAJAN, D.K. ; LONDON. S.N.** *ENDOCR. REV.*
- **WOOD, A.J.J. ; N. ENGL.** *J. Med.,* 1993, vol. 329, 404-412
- **ULMANN, A. et al.** *Sci. Amer.,* 1990, vol. 262, 42-48
- **REEL, J.R et al.** *Contraception,* 1998, vol. 58, 129-136
- **ABRAMSON, H.N. et al.** *J. Pharm. Sci.,* 1976, vol. 65, 765-768
- **OLIVETO, E.P. et al.** *J. Am. Chem. Soc.,* 1955, vol. 77, 3564-3567
- **MORIARTY, R.M. et al.** *J. Chem. Soc. Chem. Commun.,* 1981, 641-642
- **VELERIO et al.** *Steroids,* 1995, vol. 60, 268-271
- **LIVINGSTON, D.A. et al.** *J. Am. Chem. Soc.,* 1990, vol. 112, 6449-6450
- **LIVINGSTON, D.A.** *Adv.Med. Chem.,* 1992, vol. 1, 137-174
- **CARRUTHERS, N.I. et al.** *J. Org. Chem.,* 1992, vol. 57, 961-965
- **FINCH et al.** *J. Org. Chem.,* 1975, vol. 40, 206-215
- **NUMAZAWA, M. ; NAGAOKA, M.** *J. Chem. Soc. Commun.,* 1983, 127-128
- **NUMAZAWA, M. ; NAGAOKA, M.** *J. Org. Chem.,* 1985, vol. 50, 81-84
- **GLAZIER, E.R.** *J. Org. Chem.,* 1962, vol. 27, 4397-4393
- **WIECHERT, R. ; NEEF, G.** *J. Steroid Biochem.,* 1987, vol. 27, 851-858
- **TEUTSCH, G. et al.** Adrenal Steroid Antagonism. Walter de Gruyter & Co, 1984, 43-75
- **YUR'EV, Y.K. et al.** *Izvest. Akad. Nauk. S.S.S.R., Otdel Khim Nauk,* 1951, vol. 166-171, 10236f
- **WOLFE, J.P. ; BUCHWALD, S.L.** *J. Org. Chem.,* 1997, vol. 62, 6066-6068
- **VERADRO, G. et al.** *Synthesis,* 1991, 447-450
- **JONES, D.H.** *J. Chem. Soc. (C),* 1971, 132-137
- **DETTY et al.** *J. Am. Chem. Soc.,* 1983, vol. 105, 875-882
- **RAO, P.N. et al.** *Steroids,* 1998, vol. 63, 523-550
- **TEUTSCH, G. ; BELANGER, A.** *Tetrahedron Lett.,* 1979, 2051-2054
- **DESS, D.B. ; MARTIN, J.C.** *J. Org. Chem.,* 1983, vol. 48, 4155-4156
- **FRIGERIO, M. ; SANTAGOSTINO, M.** *Tetrahedron Letters,* 1994, vol. 35, 8019-8022
- **FRIGERIO, M. et al.** *J. Org. Chem.,* vol. 60, 7272-7276
- **VERADRO et al.** *Synthesis,* 1991, 447-450
- **MORIARTY et al.** *J. Chem. Soc., Chem. Commun.,* 1981, 641-642

- **BLISS, CL.** The Statistics of Bioassay. Academic Press, 1952
- **HOLLISTER, C.** *Nucleic Acids Research,* 1988, vol. 16, 1873-1875
- **MCPHAIL.** *J. Physiol.,* 1934, vol. 83, 145
- **DAO, B. et al.** *Contraception,* 1996, vol. 54, 243-258
- **REEL, J. et al.** *Contraception,* 1998, vol. 58, 129-136
- **BATISTA, M. et al.** *Am, J. Obstet. Gynecol.,* 1991, vol. 165, 82-86
- **BAULIEU, E.** Clinical Applications of Mifepristone (RU 486) and Other Antiprogestins. National Academy Press, 1993, 72-119